# EUROPEAN PATENT SPECIFICATION

(11) **EP 3 743 163 B1**
(45) Date of publication and mention of the grant of the patent: **01.04.2026**
(21) Application number: 18752958.1
(22) Date of filing: 25.07.2018
(51) Int. Cl.: A61K 8/34, C11D 3/50, A61L 9/00, A61K 8/92, A61K 8/86, A61K 8/60, A61K 8/39, A61K 8/37, C11D 3/22, A61L 9/12, A61L 9/012, A61L 9/01

(54) **FRAGRANCE COMPOSITIONS AND USES THEREOF**
GEWÜRZZUSAMMENSETZUNGEN UND VERWENDUNGEN DAVON
COMPOSITIONS DE PARFUM ET LEURS UTILISATIONS

(30) Priority: 07.02.2018 US 201862627413 P
(43) Date of publication of application: 02.12.2020
(73) Proprietor: Coty, Inc., New York, NY 10018 (US)
(72) Inventor: HOLLAND, Lynette, Anne, Makins, Egham TW20 9NW (GB); DRING, Neil, Medmenham, Buckinghamshire SL7 2EZ (GB)
(74) Representative: Dilg, Haeusler, Schindelmann Patentanwaltsgesellschaft mbH
(86) International application number: PCT/US2018/043700
(87) International publication number: WO 2019/156707

(56) References cited:
- WO-A1-2011/154926
- WO-A2-2013/060691
- US-A- 4 324 703
- US-A1- 2014 287 982
- US-A1- 2016 362 630
- ANONYMOUS: "Perfume Fixative (Glucam P-20 humectant)", 1 January 2015 (2015-01-01), XP055514064, Retrieved from the Internet <URL:https://www.creatingperfume.com/GlucamP-20humectant.aspx> [retrieved on 20181010]

## Description

### FIELD OF THE INVENTION

Inventive subject matter herein relates to the field of perfumery. In particular, it provides compositions comprising fragrance materials in a top-heavy construction and at least one substantially non-odorous fragrance modulator for improving or enhancing the fidelity and/or longevity of the fragrance profile. A top-heavy construction generally refers to fragrances including high volatile fragrance materials, or a combination of high volatile and moderate volatile fragrance materials, accounting for at least 30 wt% of the fragrance. The invention also relates to methods of making and using said compositions.

### BACKGROUND OF THE DISCLOSURE

Fragrances can include fragrance components that can be classified, in part, by their volatility. Accordingly these fragrance components may be referred to as a high-, moderate-, or low-volatility fragrance components. Different fragrances may be dominated by any one or more of these component such that the fragrance may be associated with different perceptions by a user. While some high- or moderate-volatility fragrances may be associated with a favorable perception, the volatility of the fragrance may result in a short timeframe in which the fragrance is perceived by a a panel of experts or professional evaluators or individual experts or professional evaluators or in a rapid loss of the initial character of the fragrance (e.g., citrus, aquatic, aromatic, floral, spicy, fresh, or a combination thereof) and becoming dominated by a heavy fragrance character (e.g., woody or musky).

WO 2011/154926 discloses compositions comprising a fragrance component comprising low, moderate and high volatile fragrance materials and isocetyl alcohol as substantially non-odorous fragrance modulator. Similarly, WO 2013/060691 and US 2014/287982 A1 disclose compositions whrein the fraqgrance modulator is neopentyl glycol diethylhexanoate and PPG-3 myristyl ether respectively. A further document of the prior art disclosing compositions comprising a fragrance component and a modulator is US 2016/362630.

### SUMMARY OF THE DISCLOSURE

According to the invention, a top-heavy composition includes a fragrance component present in an amount of from 0.04 wt% to 30 wt%, relative to the total weight of the composition. The fragrance component includes at least one low volatile fragrance material having a vapor pressure less than 0.001 Torr (0.000133 kPa) at 25 °C present in an amount of from 1 wt% to 30 wt%, relative to the total weight of the fragrance component. The fragrance component further includes at least one moderate volatile fragrance material having a vapor pressure in the range of 0.1 Torr to 0.001 Torr (0.0133 kPa to 0.000133 kPa) at 25 °C present in an amount of from 25 wt% to 65 wt%, relative to the total weight of the fragrance component. The fragrance component further includes at least one high volatile fragrance material having a vapor pressure greater than 0.1 Torr (0.0133 kPa) at 25 °C present in an amount of greater than 30 wt % relative to the total weight of the fragrance component. The composition further includes at least one substantially non-odorous fragrance modulator present in the amount of from 0.1 wt% to 20 wt%, relative to the total weight of the composition, wherein the at least one substantially non-odorous fragrance modulator is chosen from methyl glucoside polyol, ethyl glucoside polyol, propyl glucoside polyol, or mixtures thereof. At least one of the moderate volatile fragrance material and the high volatile fragrance material is present in the fragrance component for a period of time that is longer than a corresponding fragrance component that is free of the substantially non-odorous fragrance modulator or the equivalent fragrance that has a traditional fragrance construction (e.g., greater than about 30 wt% low volatile material, greater than about 40 wt% low volatile material, greater than about 50 wt% low volatile material, or greater than about 60 wt% low volatile material) or that the high or moderate volatile fragrance material is at a greater level at a given point in time after product application than the same fragrance that is free of the substantially non-odorous fragrance modulator or the equivalent fragrance that has a traditional fragrance construction (e.g., greater than about 30 wt% low volatile material, greater than about 40 wt% low volatile material, greater than about 50 wt% low volatile material, or greater than about 60 wt% low volatile material). In some examples the high volatile fragrance materials may also be present for an extended period of time.

There, are many non-limiting reasons for using the compositions of the instant disclosure. For example, according to various embodiments, the characteristics of the composition can provide rules for objectively classifying fragrance materials according to their volatility using their vapor pressures defined at suitable temperature, instead of their characters. The objective rules operate irrespective of perfumers performing the classification. In particular, the rules classify the fragrance materials into low, moderate or high volatile fragrance materials for formulating into fragrance mixtures. Furthermore, according to some embodiments, the compositions can have improved fidelity to the perceived fragrance profile over time. According to some embodiments, pairing the fragrance components with a selective modulator of the present invention(e.g., PPG-20 Methyl Glucose Ether) can help to ensure that an initial fragrance impression is significantly consistent from its initial impression to the end. For example, an initial impression of the fragrance can maintain its quality, as perceived by a panel of experts or professional evaluators or individual experts or professional evaluators, for a longer period of time relative to a corresponding fragrance that is free of any one of the modulators, or combinations thereof described herein or relative to the equivalent fragrance that has a traditional fragrance construction (e.g., greater than about 30 wt% low volatile material, greater than about 40 wt% low volatile material, greater than about 50 wt% low volatile material, or greater than about 60 wt% low volatile material)

Beyond extending the initial impression of the fragrance, the strength and longevity of the high volatile fragrances can be improved. For example, according to some embodiments, compositions having improved longevity of the perceived fragrance profile can be present for long periods of time (e.g., greater than 30 mins, 1, 2, 4, 6, or even 8 hours). The improved longevity of the high volatile fragrances may result from the modulator slowing the evaporation of the high and moderate volatile fragrances from the composition.

Additionally, according to some embodiments, the perceived harshness of overdosing of the fragrance material is mitigated or absent, as compared to the same perception in a fragrance in the absence of the modulator.

### BRIF DESCRIPTIONOF THE FIGRUES

The drawings illustrate generally, by way of example, but not by way of limitation, various embodiments discussed in the present document.
FIG. 1 shows the total amount of high volatility fragrance materials according to the instant disclosure.
FIG. 2 shows the total amount of high volatility fragrance materials according to the instant disclosure.
FIG. 3 shows the total amount of high volatility fragrance materials according to the instant disclosure.
FIG. 4 is a graph showing perceived notes of fragrances according to the instant disclosure.
FIG. 5 is a graph showing perceived notes of fragrances according to the instant disclosure.
FIG. 6 is a graph showing perceived notes of fragrances according to the instant disclosure.
FIG. 7 is a graph showing perceived notes of fragrances according to the instant disclosure.
FIG. 8 is a graph showing perceived notes of fragrances according to the instant disclosure.
FIG. 9 is a graph showing perceived notes of fragrances according to the instant disclosure.
FIG. 10 is a graph showing perceived notes of fragrances according to the instant disclosure.
FIG. 11 is a graph showing perceived notes of fragrances according to the instant disclosure.
FIG. 13 is a graph showing perceived notes of fragrances according to the instant disclosure.
FIG. 14 is a graph showing perceived notes of fragrances according to the instant disclosure.
FIG. 15 is a graph showing perceived notes of fragrances according to the instant disclosure.
FIG. 16 is a graph showing perceived notes of fragrances according to the instant disclosure.
FIG. 17 is a graph showing perceived notes of fragrances according to the instant disclosure.
FIG. 18 is a graph showing perceived notes of fragrances according to the instant disclosure.

### DETAILED DESCRIPTION OF THE INVENTION

### Definitions

As used herein, articles such as "a" and "an" when used in a claim, are understood to mean one or more of what is claimed or described.

As used herein, the terms "include", "includes" and "including" are meant to be non-limiting.

As used herein, the term "body splash" means a body care formulation that is applied to the body. Typically, the body splash is applied to the body after bathing and provides a subtle hint of scent to the body. Body splashes are commonly used by consumers who prefer less strong fragrance compositions. A body splash may comprise an ethanol-free composition according to the present invention which comprises from 0.2-8 wt%, relative to the total weight of the composition, of a fragrance component. The body splash may further comprise alkyl polyglucosides as non-ionic surfactants.

As used herein, the term "body spray" means a formulation comprising fragrance materials intended to be applied to the body to prevent or mask body odor caused by the bacterial breakdown of perspiration on the body (e.g., armpits, feet, and other areas of the body). The body spray may also provide a fragrance expression to the a panel of experts or professional evaluators or individual experts or professional evaluators. Typically, body spray compositions are applied as an aerosol spray in an effective amount on the skin of a consumer.

As used herein, the term "composition" includes a fine fragrance composition intended for application to a body surface, such as for example, skin or hair, e.g., to impart a pleasant odor thereto, or cover a malodour thereof. They are generally in the form of perfume concentrates, perfumes, eau de parfums, eau de toilettes, aftershaves, or colognes. The fine fragrance compositions may be an ethanol-based composition. The term "composition" may also include a cosmetic composition, which comprises a fragrance material for the purposes of delivering a pleasant smell to drive consumer acceptance of the cosmetic composition. The term "composition" may also include body splashes or body sprays. The term "composition" may also include cleaning compositions, such as fabric care composition or home care compositions, including air care compositions (e.g., air fresheners), for use on clothing or other substrates such as hard surfaces (e.g., dishes, floors, countertops). Additional non-limiting examples of "composition" may also include facial or body powder, deodorant, foundation, body/facial oil, mousse, creams (e.g., cold creams), waxes, sunscreens and blocks, bath and shower gels, lip balms, self-tanning compositions, masks and patches.

As used herein, the term "consumer" means both the user of the composition and the observer nearby or around the user.

As used herein, the term "fragrance material" and "fragrance materials" relates to a perfume raw material ("PRM"), or a mixture of perfume raw materials ("PRMs"), that are used to impart an overall pleasant odor or fragrance profile to a composition. "Fragrance materials" can encompass any suitable perfume raw materials for fragrance uses, including materials such as, for example, alcohols, aldehydes, ketones, esters, ethers, acetates, nitriles, terpene hydrocarbons, nitrogenous or sulfurous heterocyclic compounds and essential oils. However, naturally occurring plant and animal oils and exudates comprising complex mixtures of various chemical components are also known for use as "fragrance materials". The individual perfume raw materials which comprise a known natural oil can be found by reference to Journals commonly used by those skilled in the art such as "Perfume and Flavourist" or "Journal of Essential Oil Research", or listed in reference texts such as the book by S. Arctander, Perfume and Flavor Chemicals, 1969, Montclair, New Jersey, USA and more recently re-published by Allured Publishing Corporation Illinois (1994). Additionally, some perfume raw materials are supplied by the fragrance houses (Firmenich, International Flavors & Fragrances, Givaudan, Symrise) as mixtures in the form of proprietary specialty accords. Non-limiting examples of the fragrance materials useful herein include pro-fragrances such as acetal pro-fragrances, ketal pro-fragrances, ester pro-fragrances, hydrolyzable inorganic-organic pro-fragrances, and mixtures thereof. The fragrance materials may be released from the pro-fragrances in a number of ways. For example, the fragrance may be released as a result of simple hydrolysis, or by a shift in an equilibrium reaction, or by a pH-change, or by enzymatic release.

As used herein, the term "fragrance profile" means the description of how the fragrance is perceived by the human nose at any moment in time. The fragrance profile may change over time. It is a result of the combination of the low, moderate and high volatile fragrance materials, if present, of a fragrance. A fragrance profile is composed of 2 characteristics: 'intensity' and 'character'. The 'intensity' relates to the perceived strength whilst 'character' refers to the odor impression or quality of the perfume, e.g., fruity, floral, woody, *etc.*

As used herein, the terms "modulator", and "fragrance modulator" are used interchangeably to designate an agent having the capacity to affect the fragrance profile, such as for example, by impacting the fragrance materials' evaporation rate. The modulator may mediate its effect by lowering the vapor pressure of the fragrance materials and increasing their adherence to the substrate (skin and/or hair) thus ensuring a longer-lasting impression of the fragrance. By incorporating the modulator, it is desired that the fragrance profile, preferably the fragrance components composition attributable to the high and moderate volatile fragrance materials, alone or individually, of the composition can be perceived by a a panel of experts or professional evaluators or individual experts or professional evaluators, over a longer period of time, or the perceived harshness of overdosing of the fragrance material is mitigated or absent, as compared to the same perception in the absence of the modulator. As used herein "overdose" can include overdosing a moderate volatile component or high volatile component in aggregate (e.g., greater than 30 wt% of the fragrance component). The term "overdose" can further include overdosing an individual component of the moderate volatile component or the high volatile component (e.g., if the high volatile component includes three oils at least one of the oils may account for a greater wt% of the high volatile component than would be present in a traditional fragrance or a fragrance that is free of the modulators described herein). Suitable examples of the modulator are provided herein below. However, as discovered by the inventors, simply adding modulators to a traditionally constructed fragrance composition (e.g., classical fragrance pyramid construction) will not ensure an improved or enhanced fidelity and/or longevity of the fragrance profile over time.

As used herein, the term "substantially non-odorous" means an agent that does not impart an odor of its own when added into a composition of the present invention. For example, a "substantially non-odorous fragrance modulator" does not impart a new odor that alters the character of the fragrance profile of the composition to which it is added. The term "substantially non-odorous" also encompasses an agent that may impart a minimal or slight odor of its own when added into a composition of the present invention. However, the odor imparted by the "substantially non-odorous fragrance modulator" is generally undetectable or tends to not substantively alter the character of the fragrance profile of the composition to which it is added initially or preferably over time. Furthermore, the term "substantially non-odorous" also includes materials that are perceivable only by a minority of people or those materials deemed "anosmic" to the majority of people. Furthermore, the term "substantially non-odorous" also includes materials that may, from particular suppliers, contain an odor due to impurities, such as when the materials contain the impurities at not more than about 5 wt%, preferably not more than 1 wt%, often even not more than 1 part per million (ppm). These impurities maybe removed by purification techniques known in the art as required to make them suitable for use in fragrance compositions of the present invention.

As used herein, the term "vapor pressure" means the partial pressure in air at a defined temperature (e.g., 25 °C) and standard atmospheric pressure (760 mmHg) for a given chemical species. It defines a chemical species' desire to be in the gas phase rather than the liquid or solid state. The higher the vapor pressure the greater the proportion of the material that will, at equilibrium, be found in a closed headspace. It is also related to the rate of evaporation of a fragrance material which is defined in an open environment where material is leaving the system. The vapor pressure is determined according to the reference program Advanced Chemistry Development (ACD/Labs) Software Version 14.02, or preferably the latest version update).

It is understood that the test methods that are disclosed in the Test Methods Section of the present application must be used to determine the respective values of the parameters of Applicants' inventions as described and claimed herein.

In all embodiments of the present invention, all percentages are by weight of the total composition, as evident by the context, unless specifically stated otherwise. All ratios are weight ratios, unless specifically stated otherwise, and all measurements are made at 25 °C, unless otherwise designated.

### Compositions

The inventive subject matter herein is directed towards top-heavy fragrance compositions or mixtures having improved fragrance profile fidelity and longevity Disclosed fragrance compositions can include at least a fragrance component and modulator. The fragrance component can include a wide variety of fragrance materials. The fragrance materials can be grouped in terms of their volatility. Generally, the materials can be grouped as low volatile fragrance materials, moderate volatile fragrance materials, and high volatile fragrance materials. Each group of materials can be associated with various perceptions by a a panel of experts or professional evaluators or individual experts or professional evaluators. While not so limited, a high volatile fragrance may be associated with a citrus character; a moderate volatile fragrance may be associated with a spicy character; and a low volatile fragrance may be associated with a woody character. Each group of fragrance materials can include synthetic materials or natural materials. The volatility of the fragrance materials can be in reference to an individual fragrance material. Alternatively, in cases where a combination of materials produce a fragrance, for example a natural oil, the volatility may be in reference to that aggregation.

In some examples, this disclosure shows that longer lasting fragrance profiles or at least initial fragrance profiles, may be enhanced through the presence of certain modulators.

With respect to the composition, the fragrance component can be present in an amount of from 0.04 wt% to 30 wt%, 1 wt% to 30 wt%, 5 wt% to 30 wt%, or equal to, or greater than 0.04 wt%, less than, equal to, or greater than 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, or less than or equal to 30 wt% relative to the composition.

Additionally with respect to the composition, the modulator can be present in an amount of from 0.1 wt% to 20 wt%, 0.5 wt% to 20 wt%, or equal to, or greater than 0.1 wt%, less than, equal to, or greater than 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, or less than or equal to 20 wt% relative to the composition.

As described herein, the "fragrance materials" have been classified as low, moderate or high volatile fragrance materials according to their volatility by their vapor pressure. This method of classifying fragrance materials by their vapor pressure avoids the problem of different classifications for the same fragrance material according to the traditional approach that relies on their subjective characteristic character. In the case that the fragrance materials are a natural oil, extract or absolute, which comprises a mixture of several compounds, the vapor pressure of the complete oil should be treated a mixture of the individual perfume raw material components using the reference program cited above. The individual components and their level, in any given natural oil or extract, can be determined by direct injection of the oil into a GC-MS column for analysis as known by one skilled in the art. In the scenario that the fragrance materials are a proprietary specialty accord, so called 'bases', the vapor pressure, using the reference program cited above, should preferably be obtained from the supplier. However, it is understood by one skilled in the art that they can physically analyze the composition of a full fragrance oil available commercially to identity the fragrance raw materials and their levels using standard GC-MS techniques. This would be irrespective of whether they had been added to the fragrance oil as individual chemicals, as components of naturals or from proprietary bases. Although proprietary bases and naturals are included in our examples, when analyzing a commercially available fragrance *via* GC-MS one could simply identify the components of the base or natural oil as part of the overall fragrance mixture and their levels, without being able to identify which proprietary base or natural oil the fragrance had come from.

### (i) Low Volatile Fragrance Materials

The fragrance component comprises at least one low volatile fragrance material. Individual low volatile fragrance materials or aggregate low volatile fragrance materials are those having a vapor pressure less than 0.001 Torr (0.000133 kPa) at 25 °C. According to some examples, the composition can include at least 3 low volatile fragrance materials, or at least 4 low volatile fragrance materials, or at least 5 low volatile fragrance materials, or at least 7 low volatile fragrance materials. The amount of the low fragrance material present in the fragrance component can vary depending on the specific application. The low volatile fragrance material can be present in an amount ranging from 1 wt% to 30 wt%, 5 wt% to 30 wt%, or equal to, or greater than 1 wt% or less than, equal to or greater than 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.25, 27, 27.5, 28, 28.5, 29 or 29.5, or less than or equal to 30 wt% relative to the total weight of the fragrance component.

If there are more than one low volatile fragrance materials, then the ranges provided hereinabove cover the total of all the low volatile fragrance materials. Examples of suitable low volatile fragrances materials are provided in Table 1A and 1B below.

Preferably, the low volatile fragrance material is selected from at least 1 material, or at least 2 materials, or at least 3 materials, or at least 5 materials, at least 7, at least 8, at least 10, or at least 12 low volatile fragrance materials as disclosed in Table 1A. Natural fragrance materials or oils having an aggregrate vapour pressure less than 0.001 Torr (0.000133 kPa) at 25 °C are provided in Table 1B. Low Volatile Natural Oils.

**Table 1A - Low Volatile Fragrance Materials**

| **No.** | **CAS Number** | **IUPAC Name** | **Common Name**** | **Vapor Pressure (Torr at 25°C)*** |
|---|---|---|---|---|
| 1. | 1211-29-6 | Cyclopentaneacetic acid, 3-oxo-2-(2Z)-2-penten-1-yl-, methyl ester, (1*R*,2*R*)*-* | Methyl jasmonate | 0.00096500 |
| 2. | 28219-60-5 | 2-Buten-1-ol, 2-methyl-4-(2,2,3-trimethyl-3-cyclopenten-1-yl)- | Hindinol | 0.00096100 |
| 3. | 93-08-3 | Ethanone, 1-(2-naphthalenyl)- | Methyl beta-naphthyl ketone | 0.00095700 |
| 4. | 67633-95-8 | 3-Decanone, 1-hydroxy- | Methyl Lavender Ketone | 0.00095100 |
| 5. | 198404-98-7 | Cyclopropanemethanol, 1-methyl-2-[(1,2,2-trimethylbicyclo[3.1.0]he x-3-yl)methyl]- | Javanol^{®} | 0.00090200 |
| 6. | 121-32-4 | Benzaldehyde, 3-ethoxy-4-hydroxy- | Ethyl vanillin | 0.00088400 |
| 7. | 72403-67-9 | 3-Cyclohexene-1-methanol, 4-(4-methyl-3-penten-1-yl)-, 1-acetate | Myraldylaceta te | 0.00087900 |
| 8. | 28940-11-6 | 2*H*-1,5-Benzodioxepin-3(4*H*)-one, 7-methyl- | Calone | 0.00083100 |
| 9. | 139504-68-0 | 2-Butanol, 1-[[2-(1,1-dimethylethyl)cyclohexyl ]oxy]- | Amber core | 0.00080300 |
| 10. | 502847-01-0 | Spiro[5.5]undec-8-en-1-one, 2,2,7,9-tetramethyl- | Spiro[5.5]und ec-8-en-1-one, 2,2,7,9-tetramethyl- | 0.00073100 |
| 11. | 2570-03-8 | Cyclopentaneacetic acid, 3-oxo-2-pentyl-, methyl ester, (1*R*,2*R*)-rel- | trans-Hedione | 0.00071000 |
| 12. | 24851-98-7 | Cyclopentaneacetic acid, 3-oxo-2-pentyl-, methyl ester | Methyl dihydrojasmo nate or alternatives *I* | 0.00071000 |
| 13. | 101-86-0 | Octanal, 2-(phenylmethylene)- | Hexyl cinnamic aldehyde | 0.00069700 |
| 14. | 365411-50-3 | Indeno[4,5-d]-1,3-dioxin, 4,4a,5,6,7,8,9,9b-octahydro-7,7,8,9,9-pentamethyl- | Nebulone | 0.00069200 |
| 15. | 37172-53-5 | Cyclopentanecarboxylic acid, 2-hexyl-3-oxo-, methyl ester | Dihydro Iso Jasmonate | 0.00067500 |
| 16. | 65113-99-7 | 3-Cyclopentene-1-butanol, α,β,2,2,3-pentamethyl- | Sandalore^{®} | 0.00062500 |
| 17. | 68133-79-9 | Cyclopentanone, 2-(3,7-dimethyl-2,6-octadien-1-vl)- | Apritone | 0.00062000 |
| 18. | 7212-44-4 | 1,6,10-Dodecatrien-3-ol, 3,7,11-trimethyl- | Nerolidol | 0.00061600 |
| 19. | 53243-59-7 | 2-Pentenenitrile, 3-methyl-5-phenyl-, (2Z)- | Citronitril | 0.00061500 |
| 20. | 134123-93-6 | Benzenepropanenitrile, 4-ethyl-α,α-dimethyl- | Fleuranil | 0.00057600 |
| 21. | 77-53-2 | 1*H*-3a,7-Methanoazulen-6-ol, octahydro-3,6,8,8-tetramethyl-, (3*R*,3a*S*,6*R*,7*R*,8a*S*)- | Cedrol Crude | 0.00056900 |
| 22. | 68155-66-8 | Ethanone, 1-(1,2,3,5,6,7,8,8a-octahydro-2,3,8,8-tetramethyl-2-naphthalenyl)- | Iso Gamma Super | 0.00056500 |
| 23. | 54464-57-2 | Ethanone, 1-(1,2,3,4,5,6,7,8-octahydro-2,3,8,8-tetramethyl-2-naphthalenyl)- | Iso-E Super^{®} | 0.00053800 |
| 24. | 774-55-0 | Ethanone, 1-(5,6,7,8-tetrahydro-2-naphthalenyl)- | Florantone | 0.00053000 |
| 25. | 141-92-4 | 2-Octanol, 8,8-dimethoxy-2,6-dimethyl- | Hydroxycitro nellal Dimethyl Acetal | 0.00052000 |
| 26. | 20665-85-4 | Propanoic acid, 2-methyl-, 4-formyl-2-methoxyphenyl ester | Vanillin isobutyrate | 0.00051200 |
| 27. | 79-78-7 | 1,6-Heptadien-3-one, 1-(2,6,6-trimethyl-2-cvclohexen-1-vl)- | Hexalon | 0.00049800 |
| 28. | 6259-76-3 | Benzoic acid, 2-hydroxy-, hexyl ester | Hexyl Salicylate | 0.00049100 |
| 29. | 93-99-2 | Benzoic acid, phenyl ester | Phenyl Benzoate | 0.00047900 |
| 30. | 153859-23-5 | Cyclohexanepropanol, 2,2,6-trimethyl-α-propyl-, (1*R*,6*S*)*-* | Norlimbanol | 0.00046900 |
| 31. | 70788-30-6 | Cyclohexanepropanol, 2,2,6-trimethyl-α-propyl- | Timberol / Norlimbanol | 0.00046900 |
| 32. | 68555-58-8 | Benzoic acid, 2-hydroxy-, 3-methyl-2-buten-1-yl ester | Prenyl Salicylate | 0.00045700 |
| 33. | 950919-28-5 | 2H-1,5-Benzodioxepin-3(4*H*)-one, 7-(1-methylethyl)- | Cascalone | 0.00045500 |
| 34. | 30168-23-1 | Butanal, 4-(octahydro-4,7-methano-5*H*-inden-5-vlidene)- | Dupical | 0.00044100 |
| 35. | 1222-05-5 | Cyclopenta[g]-2-benzopyran, 1,3,4,6,7,8- | Galaxolide^{®} | 0.00041400 |
| | | hexahydro-4,6,6,7,8,8-hexamethyl- | | |
| 36. | 4602-84-0 | 2,6,10-Dodecatrien-1-ol, 3,7,11-trimethyl- | Farnesol | 0.00037000 |
| 37. | 95962-14-4 | Cyclopentanone, 2-[2-(4-methyl-3-cyclohexen-1-yl)propyl]- | Nectaryl | 0.00036700 |
| 38. | 4674-50-4 | 2(3*H*)-Naphthalenone, 4,4a,5,6,7,8-hexahydro-4,4a-dimethyl-6-(1-methylethenyl)-, (4*R*,4a*S*,6*R*)- | Nootkatone | 0.00035800 |
| 39. | 3487-99-8 | 2-Propenoic acid, 3-phenyl-, pentyl ester | Amyl Cinnamate | 0.00035200 |
| 40. | 10522-41-5 | 2-hydroxy-2-phenylethyl acetate | Styrolyl Acetate | 0.00033900 |
| 41. | 118-71-8 | 4*H*-Pyran-4-one, 3-hydroxy-2-methyl- | Maltol | 0.00033700 |
| 42. | 128119-70-0 | 1-Propanol, 2-methyl-3-[(1,7,7-trimethylbicyclo[2.2.1]he pt-2-yl)oxy]- | Bornafix | 0.00033400 |
| 43. | 103614-86-4 | 1-Naphthalenol, 1,2,3,4,4a,5,8,8a-octahydro-2,2,6,8-tetramethyl- | Octalynol | 0.00033200 |
| 44. | 7785-33-3 | 2-Butenoic acid, 2-methyl-, (2*E*)-3,7-dimethyl-2,6-octadien-1-yl ester, (2*E*)- | Geranyl Tiglate | 0.00033200 |
| 45. | 117933-89-8 | 1,3-Dioxane, 2-(2,4-dimethyl-3-cyclohexen-1-yl)-5-methyl-5-(1-methylpropyl)- | Karanal | 0.00033100 |
| 46. | 629-92-5 | Nonadecane | Nonadecane | 0.00032500 |
| 47. | 67801-20-1 | 4-Penten-2-ol, 3-methyl-5-(2,2,3-trimethyl-3-cyclopenten-1-yl)- | Ebanol | 0.00028100 |
| 48. | 65416-14-0 | Propanoic acid, 2-methyl-, 2-methyl-4-oxo-4*H*-pyran-3-yl ester | Maltol Isobutyrate | 0.00028000 |
| 49. | 28219-61-6 | 2-Buten-1-ol, 2-ethyl-4-(2,2,3-trimethyl-3-cyclopenten-1-yl)- | Laevo Trisandol | 0.00028000 |
| 50. | 5986-55-0 | 1,6-Methanonaphthalen-1(2*H*)-ol, octahydro-4,8a,9,9-tetramethyl-, (1*R*,4*S*,4a*S*,6*R*,8a*S*)- | Healingwood | 0.00027800 |
| 51. | 195251-91-3 | 2*H*-1,5-Benzodioxepin-3(4*H*)-one, 7-(1,1-dimethylethyl)- | Transluzone | 0.00026500 |
| 52. | 3100-36-5 | 8-Cyclohexadecen-1-one | Cyclohexadec enone | 0.00025300 |
| 53. | 65405-77-8 | Benzoic acid, 2-hydroxy-, (3*Z*)-3-hexen-1-yl ester | cis-3-Hexenyl salicylate | 0.00024600 |
| 54. | 4940-11-8 | 4*H*-Pyran-4-one, 2-ethyl-3-hvdroxv- | Ethyl Maltol | 0.00022800 |
| 55. | 541-91-3 | Cyclopentadecanone, 3-methyl- | Muskone | 0.00017600 |
| 56. | 118-58-1 | Benzoic acid, 2-hydroxy-, phenylmethyl ester | Benzyl salicylate | 0.00017500 |
| 57. | 81783-01-9 | 6,8-Nonadien-3-one, 2,4,4,7-tetramethyl-, oxime | Labienoxime | 0.00017300 |
| 58. | 25485-88-5 | Benzoic acid, 2-hydroxy-, cyclohexyl ester | Cyclohexyl Salicylate | 0.00017300 |
| 59. | 91-87-2 | Benzene, [2-(dimethoxymethyl)-1-hepten-1-yl]- | Amyl Cinnamic Aldehyde Dimethyl Acetal | 0.00016300 |
| 60. | 104864-90-6 | 3-Cyclopentene-1-butanol, β,2,2,3-tetramethyl-δ-methylene- | Firsantol | 0.00016000 |
| 61. | 224031-70-3 | 4-Penten-1-one, 1-spiro[4.5]dec-7-en-7-yl- | Spirogalbanon e | 0.00015300 |
| 62. | 134-28-1 | 5-Azulenemethanol, 1,2,3,4,5,6,7,8-octahydro-α,α,3,8-tetramethyl-, 5-acetate, (3*S*,5*R*,8*S*)*-* | Guaiyl Acetate | 0.00013400 |
| 63. | 236391-76-7 | Acetic acid, 2-(1-oxopropoxy)-, 1-(3,3-dimethylcyclohexyl)ethyl ester | Romandolide ^{®} | 0.00012400 |
| 64. | 115-71-9 | 2-Penten-1-ol, 5-[(1*R*,3*R*,6*S*)-2,3-dimethyltricyclo[2.2.1.02, 6]hept-3-yl]-2-methyl-, (2*Z*)- | cis-alpha-Santalol | 0.00011800 |
| 65. | 107898-54-4 | 4-Penten-2-ol, 3,3-dimethyl-5-(2,2,3-trimethyl-3-cyclopenten-1-vl)- | Polysantol^{®} | 0.00011700 |
| 66. | 69486-14-2 | 5,8-Methano-2*H*-1-benzopyran-2-one, 6-ethylideneoctahydro- | Florex^{®} | 0.00011000 |
| 67. | 84697-09-6 | Heptanal, 2-[(4-methylphenyl)methylene] | Acalea | 0.00010100 |
| 68. | 14595-54-1 | 4-Cyclopentadecen-1-one, (4*Z*)- | Exaltenone | 0.00009640 |
| 69. | 32388-55-9 | Ethanone, 1-[(3*R*,3a*R*,7*R*,8a*S*)-2,3,4,7,8,8a-hexahydro-3,6,8,8-tetramethyl-1*H-*3a,7-methanoazulen-5-yl]- | Vertofix^{®} | 0.00008490 |
| 70. | 131812-67-4 | 1,3-Dioxolane, 2,4-dimethyl-2-(5,6,7,8-tetrahydro-5,5,8,8-tetramethyl-2-naphthalenyl)- | Okoumal^{®} | 0.00007600 |
| 71. | 106-02-5 | Oxacyclohexadecan-2-one | Exaltolide^{®} | 0.00006430 |
| 72. | 141773-73-1 | 1-Propanol, 2-[1-(3,3-dimethylcyclohexyl)etho xy]-2-methyl-, 1-propanoate | Helvetolide^{®} | 0.00005790 |
| 73. | 63314-79-4 | 5-Cyclopentadecen-1-one, 3-methyl- | Delta Muscenone | 0.00005650 |
| 74. | 77-42-9 | 2-Penten-1-ol, 2-methyl-5-[(1*S*,2*R*,4*R*)-2-methyl-3-methylenebicyclo[2.2.1]h ept-2-yl]-, (2Z)- | cis-beta-Santalol | 0.00004810 |
| 75. | 362467-67-2 | 2*H*-1,5-Benzodioxepin-3(4*H*)-one, 7-(3-methylbutyl)- | Azurone | 0.00004770 |
| 76. | 28371-99-5 | Ethanone, 1-(2,6,10-trimethyl-2,5,9-cvclododecatrien-1-vl)- | Trimofix O | 0.00004580 |
| 77. | 16223-63-5 | 1*H*-3a,6-Methanoazulene-3-methanol, octahydro-7,7-dimethyl-8-methylene-, (3*S*,3a*R*,6*R*,8a*S*)- | Khusimol | 0.00004400 |
| 78. | 10461-98-0 | Benzeneacetonitrile, α-cyclohexylidene- | Peonile | 0.00004290 |
| 79. | 90-17-5 | Benzenemethanol, α-(trichloromethyl)-, 1-acetate | Rosacetol | 0.00004240 |
| 80. | 50607-64-2 | Benzoic acid, 2-[(2-methylpentylidene)amino ]-, methyl ester | Mevantraal | 0.00004070 |
| 81. | 29895-73-6 | 5-Hydroxy-2-benzyl-1,3-dioxane | Acetal CD | 0.00004050 |
| 82. | 94-47-3 | Benzoic acid, 2-phenylethyl ester | Phenyl Ethyl Benzoate | 0.00003480 |
| 83. | 3100-36-5 | Cyclohexadec-8-en-1-one | Globanone^{®} | 0.00003310 |
| 84. | 37609-25-9 | 5-Cyclohexadecen-1-One | Ambretone | 0.00003310 |
| 85. | 66072-32-0 | Cyclohexanol, 4-(1,7,7-trimethylbicyclo[2.2.1]he pt-2-yl)- | Iso Bornyl Cyclohexanol | 0.00003010 |
| 86. | 31906-04-4 | 3-Cyclohexene-1-carboxaldehyde, 4-(4-hydroxy-4-methylpentyl)- | Lyral^{®} | 0.00002940 |
| 87. | 21145-77-7 | Ethanone, 1-(5,6,7,8-tetrahydro-3,5,5,6,8,8-hexamethyl-2-naphthalenyl)- | Musk Plus | 0.00002860 |
| 88. | 21145-77-7 | Ethanone, 1-(5,6,7,8-tetrahydro-3,5,5,6,8,8-hexamethyl-2-naphthalenyl)- | Fixolide | 0.00002860 |
| 89. | 22442-01-9 | 2-Cyclopentadecen-1-one, 3-methyl- | Muscenone | 0.00002770 |
| 90. | 109-29-5 | Oxacycloheptadecan-2-one | Silvanone Ci | 0.00002600 |
| 91. | 101-94-0 | Benzeneacetic acid, 4-methylphenyl ester | Para Cresyl Phenyl Acetate | 0.00002330 |
| 92. | 102-20-5 | Benzeneacetic acid, 2-phenylethyl ester | Phenyl Ethyl Phenyl Acetate | 0.00002300 |
| 93. | 118562-73-5 | Cyclododecaneethanol, β-methyl- | Hydroxyambr an | 0.00001800 |
| 94. | 103-41-3 | 2-Propenoic acid, 3-phenyl-, phenylmethyl ester | Benzyl Cinnamate | 0.00001050 |
| 95. | 4707-47-5 | Benzoic acid, 2,4-dihydroxy-3,6-dimethyl-, methyl ester | Veramoss / LRG201 / Evernvl | 0.00001050 |
| 96. | 183551-83-9 | Naphtho[2,1-b]furan-6(7*H*)-one, 8,9-dihydro-1,5,8-trimethyl-, (8*R*)- | Myrrhone | 0.00000977 |
| 97. | 102-17-0 | Benzeneacetic acid, (4-methoxyphenyl)methyl ester | Para Anisyl Phenyl Acetate | 0.00000813 |
| 98. | 120-11-6 | Benzene, 2-methoxy-1-(phenylmethoxy)-4-(1-propen-1-yl)- | Benzyl Iso Eugenol | 0.00000676 |
| 99. | 102-22-7 | Benzeneacetic acid, (2*E*)-3,7-dimethyl-2,6-octadien-1-vl ester | Geranyl Phenylacetate | 0.00000645 |
| 100. | 111879-80-2 | Oxacyclohexadec-12-en-2-one, (12*E*)- | Habanolide 100% | 0.00000431 |
| 101. | 87-22-9 | Benzoic acid, 2-hydroxy-, 2-phenylethyl ester | Phenyl Ethyl Salicylate | 0.00000299 |
| 102. | 78-37-5 | 2-Propenoic acid, 3-phenyl-, 1-ethenyl-1,5-dimethyl-4-hexen-1-yl ester | Linalyl Cinnamate | 0.00000174 |
| 103. | 28645-51-4 | Oxacycloheptadec-10-en-2-one | Ambrettolide | 0.00000139 |
| 104. | 123-69-3 | Oxacycloheptadec-8-en-2-one, (8*Z*)- | Ambrettolide | 0.00000136 |
| 105. | 3391-83-1 | 1,7-Dioxacycloheptadecan-8-one | Musk RI | 0.00000057 |
| 106. | 68527-79-7 | 7-Octen-2-ol, 8-(1*H-*indol-1-yl)-2,6-dimethyl- | Indolene | 0.000000445 |
| 107. | 89-43-0 | Methyl 2-[(7-hydroxy-3,7-dimethyloctylidene)amin o]benzoate | Aurantinol | 0.0000000100 |
| 108. | 54982-83-1 | 1,4-Dioxacyclohexadecane-5,16-dione | Zenolide | 0.00000000834 |
| 109. | 105-95-3 | 1,4-Dioxacycloheptadecane-5,17-dione | Ethylene Brassylate | 0.00000000313 |
| 110. | 3681-73-0 | Hexadecanoic acid, (2*E*)-3,7-dimethyl-2,6-octadien-1-vl ester | Hexarose | 0.00000000300 |
| 111. | 4159-29-9 | Phenol, 4-[3-(benzoyloxy)-1-propen-1-yl]-2-methoxy- | Coniferyl benzoate | 0.00000000170 |
| 112. | 144761-91-1 | Benzoic acid, 2-[(1-hydroxy-3-phenylbutyl)amino]-, methyl ester | Trifone DIPG | 0.00000000093 |

| | | | | |
|---|---|---|---|---|
| *¹* Non-limiting examples of alternative qualities from various suppliers can be purchased under the following tradenames: Kharismal^{®} Super (IFF), Kharismal^{®} (IFF), Hedione^{®} (Firmenich), Hedione^{®} HC (Firmenich), Paradisone (Firmenich), Cepionate (Zenon), Super cepionate (Zenon), Claigeon^{®} (Zenon). * Vapor Pressures are acquired as described in the Test Methods Section. ** Origin: The low volatile fragrance materials may be obtained from one or more of the following companies: Firmenich (Geneva, Switzerland), Symrise AG (Holzminden, Germany), Givaudan (Argenteuil, France), IFF (Hazlet, New Jersey), Bedoukian (Danbury, Connecticut), Sigma Aldrich (St. Louis, Missouri), Millennium Specialty Chemicals (Olympia Fields, Illinois), Polarone International (Jersey City, New Jersey), and Aroma & Flavor Specialties (Danbury, Connecticut). | | | | |

**Table 1B -Low Volatile Natural Oils.**

| **No.** | **Natural oil** | **Supplier** |
|---|---|---|
| 1. | Beeswax Absolute | Robertet |
| 2. | Cedarwood Sawdust SFE | Firmenich |
| 3. | Cedarwood Oil Rect | Firmenich |
| 4. | Cedarwood Texas Light | H. Revnaud & Fils |
| 5. | Ciste Absolute | IFF |
| 6. | Cocoa Colorless Oil | Robertet |
| 7. | Cypriol Coeur Essence | Robertet |
| 8.. | Guaiacwood Oil | Global Essence Inc |
| 9. | Incense Wood Natural | Robertet |
| 10. | Orris CO2 Extract | Mane |
| 11. | Patchouli Oil | IFF |
| 12. | Tolu Baume Res | Robertet |
| 13. | Vanilla Absolute | Robertet |
| 14. | Vanilla CO2 Absolute | Robertet |
| 15. | Vetivert Oil | IFF |
| 16. | Vetyvert Acetate | Robertet |

### Suppliers:

Firmenich, Geneva, Switzerland
Global Essence Inc, New Jersey, USA
H. Reynaud & Fils, Montbrun-les-Bains, France
IFF, Hazlet, New Jersey, USA
Mane, Le Bar-sur-Loup, France
Robertet, Grasse, France

Exemplary low volatile fragrance materials selected from the group of Tables 1A or 1B Low Volatile Fragrance Materials are preferred. However, it is understood by one skilled in the art that other low volatile fragrance materials, not recited in Tables 1A or 1B, would also fall within the scope of the present invention, so long as they have a vapor pressure less than 0.001 Torr (0.000133 kPa) at 25 °C.

### (ii) Moderate Volatile Fragrance Materials

The fragrance component includes at least one moderate volatile fragrance material or aggregate of volatile fragrance materials having a vapor pressure in the range of 0.1 Torr to 0.001 Torr (0.0133 kPa to 0.000133 kPa) at 25 °C. In some examples, the composition according to the present disclosure can include at least 3 moderate volatile fragrance materials, or at least 5 moderate volatile fragrance materials, or at least 7 moderate volatile fragrance materials. Compositions of the present invention can include high levels of the moderate volatile fragrance materials present in an amount of from 25 wt% to 65 wt%, 35 wt% to 60 wt%, or less than equal to, or greater than about 30 wt%, 35, 40, 45, 50, 55 or 60, or less than or equal to 65 wt% of the fragrance component. If there are more than one moderate volatile fragrance materials, then the ranges provided hereinabove cover the total of all of the moderate volatile fragrance materials. Suitable examples of moderate volatile fragrances materials are provided in Table 2A and 2B below.

Preferable examples of moderate volatile fragrance materials having a vapor pressure in the range of 0.1 Torr to 0.001 Torr (0.0133 kPa to 0.000133 kPa) at 25 °C are provided in Table 2A and 2B. Preferably, the moderate volatile fragrance material is selected from at least 1 material, or at least 2 materials, or at least 3 materials, or at least 5 materials, or at least 7 moderate volatile fragrance materials as disclosed in Table 2A. Natural fragrance materials or oils having an aggregrate vapour pressure between 0.1 Torr to 0.001 Torr (0.0133 kPa to 0.000133 kPa) at 25 °C are provided in Table 2B. Moderate Volatile Natural Oils.

**Table 2A - Moderate Volatile Fragrance Materials**

| **No.** | **CAS Number** | **IUPAC Name** | **Common Name**** | **Vapor Pressure (Torr at 25 °C)*** |
|---|---|---|---|---|
| 1. | 24168-70-5 | Pyrazine, 2-methoxy-3-(1-methylpropyl)- | Methoxyisobutylp yrazine | 0.09950000 |
| 2. | 89-79-2 | Cyclohexanol, 5-methyl-2-(1-methylethenyl)-, (1*R*,2*S*,5*R*)*-* | Iso-Pulegol | 0.09930000 |
| 3. | 112-12-9 | 2-Undecanone | Methyl Nonyl Ketone | 0.09780000 |
| 4. | 103-05-9 | Benzenepropanol, α,α-dimethyl- | Phenyl Ethyl Dimethyl Carbinol | 0.09770000 |
| 5. | 125-12-2 | Bicyclo[2.2.1]heptan -2-ol, 1,7,7-trimethyl-, 2-acetate, (1*R*,2*R*,4*R*)*-*rel*-* | Iso Bornyl Acetate | 0.09590000 |
| 6. | 78-70-6 | 1,6-Octadien-3-ol, 3,7-dimethyl- | Linalool | 0.09050000 |
| 7. | 101-97-3 | Benzeneacetic acid, ethyl ester | Ethyl Phenyl Acetate | 0.08970000 |
| 8. | 100-86-7 | Benzeneethanol, α,α-dimethyl- | Dimethyl Benzyl Carbinol | 0.08880000 |
| 9. | 188570-78-7 | Cyclopropanecarbox ylic acid, (3*Z*)-3-hexen-1-yl ester | Montaverdi | 0.08640000 |
| 10. | 67634-25-7 | 3-Cyclohexene-1-methanol, 3,5-dimethyl-, 1-acetate | Floralate | 0.08500000 |
| 11. | 112-44-7 | Undecanal | Undecyl Aldehyde | 0.08320000 |
| 12. | 32669-00-4 | Ethanone, 1-(3-cycloocten-1-yl)- | Tanaisone^{®} | 0.08150000 |
| 13. | 98-53-3 | Cyclohexanone, 4-(1,1-dimethylethyl)- | Patchi | 0.07780000 |
| 14. | 35854-86-5 | 6-Nonen-1-ol, (6*Z*)- | cis-6-None-1-ol | 0.07770000 |
| 15. | 5331-14-6 | Benzene, (2-butoxyethyl)- | Butyl phenethyl ether | 0.07760000 |
| 16. | 80-57-9 | Bicyclo[3.1.1]hept-3-en-2-one, 4,6,6-trimethyl- | Verbenone | 0.07730000 |
| 17. | 22471-55-2 | Cyclohexanecarboxy lic acid, 2,2,6-trimethyl-, ethyl ester, (1*R*,6*S*)-rel- | Thesaron | 0.07670000 |
| 18. | 60-12-8 | Benzeneethanol | Phenethyl alcohol | 0.07410000 |
| | | | | |
| 19. | 106-26-3 | 2,6-Octadienal, 3,7-dimethyl-, (2*Z*)- | Neral | 0.07120000 |
| 20. | 5392-40-5 | 2,6-Octadienal, 3,7-dimethyl- | Citral | 0.07120000 |
| 21. | 89-48-5 | Cyclohexanol, 5-methyl-2-(1-methylethyl)-, 1-acetate, (1*R*,2*S*,5*R*)-rel- | Menthyl Acetate | 0.07070000 |
| 22. | 119-36-8 | Benzoic acid, 2-hydroxy-, methyl ester | Methyl salicylate | 0.07000000 |
| 23. | 104-46-1 | Benzene, 1-methoxy-4-(1*E*)-1-propen-1-yl- | Anethol | 0.06870000 |
| 24. | 7549-37-3 | 2,6-Octadiene, 1,1-dimethoxy-3,7-dimethyl- | Citral Dimethyl Acetal | 0.06780000 |
| 25. | 25225-08-5 | Cyclohexanemethan ol, α,3,3-trimethyl-, 1-formate | Aphermate | 0.06780000 |
| 26. | 3913-81-3 | 2-Decenal, (2E)- | 2-Decene-1-al | 0.06740000 |
| 27. | 15373-31-6 | 3-Cyclopentene-1-acetonitrile, 2,2,3-trimethyl- | Cantryl^{®} | 0.06700000 |
| 28. | 6485-40-1 | 2-Cyclohexen-1-one, 2-methyl-5-(1-methylethenyl)-, (5R)- | Laevo carvone | 0.06560000 |
| 29. | 16587-71-6 | Cyclohexanone, 4-(1,1-dimethylpropyl)- | Orivone | 0.06490000 |
| 30. | 62406-73-9 | 6,10-Dioxaspiro[4.5]deca ne, 8,8-dimethyl-7-(1-methylethyl)- | Opalal CI | 0.06290000 |
| 31. | 3720-16-9 | 2-Cyclohexen-1-one, 3-methyl-5-propyl- | Livescone | 0.06270000 |
| 32. | 13816-33-6 | Benzonitrile, 4-(1-methylethyl)- | Cumin Nitrile | 0.06230000 |
| 33. | 67019-89-0 | 2,6-Nonadienenitrile | Violet Nitrile | 0.06200000 |
| | | | | |
| 34. | 53398-85-9 | Butanoic acid, 2-methyl-, (3Z)-3-hexen-1-vl ester | cis-3-Hexenyl Alpha Methyl Butyrate | 0.06130000 |
| 35. | 208041-98-9 | n/a | Jasmonitrile | 0.05920000 |
| 36. | 16510-27-3 | Benzene, 1-(cyclopropylmethyl) -4-methoxy- | Toscanol | 0.05870000 |
| 37. | 111-80-8 | 2-Nonynoic acid, methyl ester | Methyl Octine Carbonate | 0.05680000 |
| 38. | 103-45-7 | Acetic acid, 2-phenylethyl ester | Phenyl Ethyl Acetate | 0.05640000 |
| 39. | 2550-26-7 | 2-Butanone, 4-phenyl- | Benzyl Acetone | 0.05570000 |
| 40. | 13491-79-7 | Cyclohexanol, 2-(1,1-dimethylethyl)- | Verdol | 0.05430000 |
| 41. | 7786-44-9 | 2,6-Nonadien-1-ol | 2,6-Nonadien-1-ol | 0.05370000 |
| 42. | 103-28-6 | Propanoic acid, 2-methyl-, phenylmethyl ester | Benzyl Iso Butyrate | 0.05130000 |
| 43. | 104-62-1 | Formic acid, 2-phenylethyl ester | Phenyl Ethyl Formate | 0.05050000 |
| 44. | 28462-85-3 | Bicyclo[2.2.1]heptan -2-ol, 1,2,3,3-tetramethyl-, (1*R*,2*R*,4*S*)-rel*-* | Humus Ether | 0.04870000 |
| 45. | 122-03-2 | Benzaldehyde, 4-(1-methylethyl)- | Cuminic Aldehyde | 0.04820000 |
| 46. | 358331-95-0 | 2,5-Octadien-4-one, 5,6,7-trimethyl-, (2*E*)- | Pomarose | 0.04810000 |
| 47. | 562-74-3 | 3-Cyclohexen-1-ol, 4-methyl-1-(1-methylethyl)- | Terpinenol-4 | 0.04780000 |
| 48. | 68527-77-5 | 3-Cyclohexene-1-methanol, 2,4,6-trimethyl- | Isocyclogeraniol | 0.04640000 |
| 49. | 35852-46-1 | Pentanoic acid, (3*Z*)-3-hexen-1-yl ester | Cis-3-Hexenyl Valerate | 0.04580000 |
| | | | | |
| 50. | 2756-56-1 | Bicyclo[2.2.1]heptan -2-ol, 1,7,7-trimethyl-, 2-propanoate, (1*R*,2*R*,4*R*)-rel*-* | Iso Bornyl Propionate | 0.04540000 |
| 51. | 14374-92-6 | Benzene, 1-methyl-4-(1-methylethyl)-2-(1-propen-1-yl)- | Verdoracine | 0.04460000 |
| 52. | 6784-13-0 | 3-Cyclohexene-1-propanal, β,4-dimethyl- | Limonenal | 0.04380000 |
| 53. | 8000-41-7 | 2-(4-methyl-1-cyclohex-3-enyl)propan-2-ol | Alpha Terpineol | 0.04320000 |
| 54. | 41884-28-0 | 1-Hexanol, 5-methyl-2-(1-methylethyl)-, (2*R*)- | Tetrahydro Lavandulol | 0.04230000 |
| 55. | 22457-23-4 | 3-Heptanone, 5-methyl-, oxime | Stemone^{®} | 0.04140000 |
| 56. | 104-50-7 | 2(3*H*)-Furanone, 5-butyldihydro- | Gamma Octalactone | 0.04080000 |
| 57. | 143-08-8 | 1-Nonanol | Nonyl Alcohol | 0.04070000 |
| 58. | 3613-30-7 | Octanal, 7-methoxy-3,7-dimethyl- | Methoxycitronell al | 0.04020000 |
| 59. | 67634-00-8 | Acetic acid, 2-(3-methylbutoxy)-, 2-propen-1-yl ester | Allyl Amyl Glycolate | 0.04000000 |
| 60. | 464-45-9 | Bicyclo[2.2.1]heptan -2-ol, 1,7,7-trimethyl-, (1*S*,2*R*,4*S*)*-* | 1-Borneol | 0.03980000 |
| 61. | 124-76-5 | Bicyclo[2.2.1]heptan -2-ol, 1,7,7-trimethyl-, (1*R*,2*R*,4*R*)-rel*-* | 1.7.7-TrimethylBicyclo-1.2.2-Heptanol-2 | 0.03980000 |
| 62. | 67874-72-0 | Cyclohexanol, 2-(1,1-dimethylpropyl)-, 1-acetate | Coniferan | 0.03980000 |
| 63. | 80-26-2 | 3-Cyclohexene-1-methanol, α,α,4-trimethyl-, 1-acetate | Terpinyl Acetate | 0.03920000 |
| 64. | 498-81-7 | Cyclohexanemethan ol, α,α,4-trimethyl- | Dihydro Terpineol | 0.03920000 |
| 65. | 112-45-8 | 10-Undecenal | Undecylenic aldehyde | 0.03900000 |
| 66. | 35044-57-6 | 2,4-Cyclohexadiene-1-carboxylic acid, 2,6,6-trimethyl-, ethyl ester | Ethyl Safranate | 0.03880000 |
| 67. | 106-21-8 | 1-Octanol, 3,7-dimethyl- | Dimethyl Octanol | 0.03860000 |
| 68. | 84560-00-9 | Cyclopentanol, 2-pentyl- | Cyclopentol | 0.03790000 |
| 69. | 82461-14-1 | Furan, tetrahydro-2,4-dimethyl-4-phenyl- | Rhubafuran^{®} | 0.03780000 |
| 70. | 56011-02-0 | Benzene, [2-(3-methylbutoxy)ethyl] | Phenyl Ethyl Isoamyl Ether | 0.03690000 |
| 71. | 103-37-7 | Butanoic acid, phenylmethyl ester | Benzyl Butyrate | 0.03660000 |
| 72. | 6378-65-0 | Hexyl hexanoate | Hexyl hexanoate | 0.03490000 |
| 73. | 118-61-6 | Benzoic acid, 2-hydroxy-, ethyl ester | Ethyl salicylate | 0.03480000 |
| 74. | 98-52-2 | Cyclohexanol, 4-(1,1-dimethylethyl)- | Patchon | 0.03480000 |
| 75. | 115-99-1 | 1,6-Octadien-3-ol, 3,7-dimethyl-, 3-formate | Linalyl Formate | 0.03440000 |
| 76. | 112-54-9 | Dodecanal | Lauric Aldehyde | 0.03440000 |
| 77. | 53046-97-2 | 3,6-Nonadien-1-ol, (3*Z*,6*Z*)- | 3,6 Nonadien-1-ol | 0.03360000 |
| 78. | 76649-25-7 | 3,6-Nonadien-1-ol | 3,6-Nonadien-1-ol | 0.03360000 |
| 79. | 141-25-3 | 3,7-Dimethyloct-6-en-1-ol | Rhodinol | 0.03290000 |
| 80. | 1975-78-6 | Decanenitrile | Decanonitrile | 0.03250000 |
| 81. | 2216-51-5 | Cyclohexanol, 5-methyl-2-(1-methylethyl)-, (1*R*,2*S*,5*R*)*-* | L-Menthol | 0.03230000 |
| 82. | 3658-77-3 | 4-hydroxy-2,5-dimethylfuran-3-one | Pineapple Ketone | 0.03200000 |
| 83. | 103-93-5 | Propanoic acid, 2-methyl-, 4-methylphenyl ester | Para Cresyl iso-Butyrate | 0.03120000 |
| 84. | 24717-86-0 | Propanoic acid, 2-methyl-, (1*R*,2*S*,4*R*)-1,7,7-trimethylbicyclo[2.2. 1]hept-2-yl ester, rel- | Abierate | 0.03110000 |
| 85. | 67845-46-9 | Acetaldehyde, 2-(4-methylphenoxy)- | Aldehyde XI | 0.03090000 |
| 86. | 67883-79-8 | 2-Butenoic acid, 2-methyl-, (3*Z*)-3-hexen-1-yl ester, (2*E*)- | Cis-3-Hexenyl Tiglate | 0.03060000 |
| 87. | 33885-51-7 | Bicyclo[3.1.1]hept-2-ene-2-propanal, 6,6-dimethyl- | Pino Acetaldehyde | 0.03040000 |
| 88. | 105-85-1 | 6-Octen-1-ol, 3,7-dimethyl-, 1-formate | Citronellyl Formate | 0.03000000 |
| 89. | 70214-77-6 | 2-Nonanol, 6,8-dimethyl- | Nonadyl | 0.03010000 |
| 90. | 215231-33-7 | Cyclohexanol, 1-methyl-3-(2-methylpropyl)- | Rossitol | 0.02990000 |
| 91. | 120-72-9 | 1*H*-Indole | Indole | 0.02980000 |
| 92. | 2463-77-6 | 2-Undecenal | 2-Undecene-1-al | 0.02970000 |
| 93. | 675-09-2 | 2*H*-Pyran-2-one, 4,6-dimethyl- | Levistamel | 0.02940000 |
| 94. | 98-55-5 | 3-Cyclohexene-1-methanol, α,α,4-trimethyl- | Alpha-Terpineol | 0.02830000 |
| 95. | 81786-73-4 | 3-Hepten-2-one, 3,4,5,6,6-pentamethyl-, (3*Z*)- | Koavone | 0.02750000 |
| 96. | 122-97-4 | Benzenepropanol | Phenyl Propyl Alcohol | 0.02710000 |
| 97. | 39212-23-2 | 2(3*H*)-Furanone, 5-butyldihydro-4-methyl- | Methyl Octalactone | 0.02700000 |
| 98. | 53767-93-4 | 7-Octen-2-ol, 2,6-dimethyl-, 2-acetate | Dihydro Terpinyl Acetate | 0.02690000 |
| 99. | 35044-59-8 | 1,3-Cyclohexadiene-1-carboxylic acid, 2,6,6-trimethyl-, ethyl ester | Ethyl Safranate | 0.02660000 |
| 100. | 104-55-2 | 2-Propenal, 3-phenyl- | Cinnamic Aldehyde | 0.02650000 |
| 101. | 144-39-8 | 1,6-Octadien-3-ol, 3,7-dimethyl-, 3-propanoate | Linalyl Propionate | 0.02630000 |
| 102. | 61931-80-4 | 1,6-Nonadien-3-ol, 3,7-dimethyl-, 3-acetate | 3,7-Dimethyl-1,6-nonadien-3-yl acetate | 0.02630000 |
| 103. | 102-13-6 | Benzeneacetic acid, 2-methylpropyl ester | Iso Butyl Phenylacetate | 0.02630000 |
| 104. | 65443-14-3 | Cyclopentanone, 2,2,5-trimethyl-5-pentyl- | Veloutone | 0.02610000 |
| 105. | 141-12-8 | 2,6-Octadien-1-ol, 3,7-dimethyl-, 1-acetate, (2*Z*)- | Neryl Acetate | 0.02560000 |
| 106. | 105-87-3 | 2,6-Octadien-1-ol, 3,7-dimethyl-, 1-acetate, (2*E*)- | Geranyl acetate | 0.02560000 |
| 107. | 68141-17-3 | Undecane, 1,1-dimethoxy-2-methyl- | Methyl Nonyl Acetaldehyde Dimethyl Acetal | 0.02550000 |
| 108. | 2206-94-2 | Benzenemethanol, α-methylene-, 1-acetate | Indocolore | 0.02550000 |
| 109. | 10528-67-3 | Cyclohexanepropan ol, α-methyl- | Cyclohexylmagno 1 | 0.02550000 |
| 110. | 123-11-5 | Benzaldehyde, 4-methoxy- | Anisic Aldehyde | 0.02490000 |
| 111. | 57576-09-7 | Cyclohexanol, 5-methyl-2-(1-methylethenyl)-, 1-acetate, (1*R*,2*S*,5*R*)*-* | Iso Pulegol Acetate | 0.02480000 |
| 112. | 51566-62-2 | 6-Octenenitrile, 3,7-dimethyl- | Citronellyl Nitrile | 0.02470000 |
| 113. | 60335-71-9 | 2*H*-Pyran, 3,6-dihydro-4-methyl-2-phenyl- | Rosyrane Super | 0.02470000 |
| 114. | 30385-25-2 | 6-Octen-2-ol, 2,6-dimethyl- | Dihydromyrcenol | 0.02440000 |
| 115. | 101-84-8 | Benzene, 1,1'-oxybis- | Diphenyl Oxide | 0.02230000 |
| 116. | 136-60-7 | Benzoic acid, butyl ester | Butyl Benzoate | 0.02170000 |
| 117. | 93939-86-7 | 5,8-Methano-2*H*-1-benzopyran, 6-ethylideneoctahydro | Rhuboflor | 0.02120000 |
| 118. | 83926-73-2 | Cyclohexanepropan ol, α,α-dimethyl- | Coranol | 0.02100000 |
| 119. | 125109-85-5 | Benzenepropanal, β-methyl-3-(1-methylethyl)- | Florhydral | 0.02070000 |
| 120. | 104-21-2 | Benzenemethanol, 4-methoxy-, 1-acetate | Anisyl Acetate | 0.02050000 |
| 121. | 1365-19-1 | 2-Furanmethanol, 5-ethenyltetrahydro-α,α,5-trimethyl- | Linalool Oxide | 0.02050000 |
| 122. | 137-03-1 | Cyclopentanone, 2-heptyl- | Frutalone | 0.02040000 |
| 123. | 2563-07-7 | Phenol, 2-ethoxy-4-methyl- | Ultravanil | 0.02030000 |
| 124. | 1128-08-1 | 2-Cyclopenten-1-one, 3-methyl-2-pentyl- | Dihydrojasmone | 0.02020000 |
| 125. | 7493-57-4 | Benzene, [2-(1-propoxyethoxy)ethyl ]- | Acetaldehyde | 0.01990000 |
| 126. | 141-25-3 | 7-Octen-1-ol, 3,7-dimethyl- | Rhodinol | 0.01970000 |
| 127. | 216970-21-7 | Bicyclo[4.3.1]decan e, 3-methoxy-7,7-dimethyl-10-methylene- | 3-Methoxy-7,7-dimethyl-10-methylenebicyclo [4.3.1]decane | 0.01960000 |
| 128. | 319002-92-1 | Propanoic acid, 2-(1,1-dimethylpropoxy)-, propyl ester, (2S)- | Sclareolate^{®} | 0.01960000 |
| 129. | 85-91-6 | Benzoic acid, 2-(methylamino)-, methyl | Dimethyl anthranilate | 0.01930000 |
| 130. | 13828-37-0 | Cyclohexanemethan ol, 4-(1-methylethyl)-, cis- | Mayol | 0.01920000 |
| 131. | 26330-65-4 | (*E*)-6-ethyl-3-methyloct-6-en-1-ol | Super Muguet | 0.01850000 |
| 132. | 7540-51-4 | 6-Octen-1-ol, 3,7-dimethyl-, (3S)- | L-Citronellol | 0.01830000 |
| 133. | 106-22-9 | 6-Octen-1-ol, 3,7-dimethyl- | Citronellol | 0.01830000 |
| 134. | 543-39-5 | 7-Octen-2-ol, 2-methyl-6-methylene- | Myrcenol | 0.01820000 |
| 135. | 7775-00-0 | Benzenepropanal, 4-(1-methylethyl)- | Cyclemax | 0.01820000 |
| 136. | 18479-54-4 | 4,6-Octadien-3-ol, 3,7-dimethyl- | Muguol | 0.01800000 |
| 137. | 29214-60-6 | Octanoic acid, 2-acetyl-, ethyl ester | Gelsone | 0.01790000 |
| 138. | 1209-61-6 | 5-Oxatricyclo[8.2.0.04, 6]dodecane, 4,9,12,12-tetramethyl- | Tobacarol | 0.01730000 |
| 139. | 57934-97-1 | 2-Cyclohexene-1-carboxylic acid, 2- | Givescone | 0.01710000 |
| | | ethyl-6,6-dimethyl-, ethyl ester | | |
| 140. | 14901-07-6 | 3-Buten-2-one, 4-(2,6,6-trimethyl-1-cyclohexen-1-yl)-, (3*E*)- | Beta-Ionone | 0.01690000 |
| 141. | 64001-15-6 | 4,7-Methano-1H-inden-5-ol, octahydro-, 5-acetate | Dihydro Cyclacet | 0.01630000 |
| 142. | 95-41-0 | 2-Cyclopenten-1-one, 2-hexyl- | Iso Jasmone T | 0.01600000 |
| 143. | 134-20-3 | Benzoic acid, 2-amino-, methyl ester | Methyl Anthranilate | 0.01580000 |
| 144. | 100-06-1 | Ethanone, 1-(4-methoxyphenyl)- | Para Methoxy Acetophenone | 0.01550000 |
| 145. | 105-86-2 | 2,6-Octadien-1-ol, 3,7-dimethyl-, 1-formate, (2*E*)- | Geranyl Formate | 0.01540000 |
| 146. | 154171-77-4 | Spiro[1,3-dioxolane-2,8'(5*H*)-[2*H-*2,4a]methanonaphth alene], hexahydro-1',1',5',5'-tetramethyl-, (2'*S*,4'a*S*,8'a*S*)-(9CI) | Ysamber K^{®} | 0.01470000 |
| 147. | 154171-76-3 | Spiro[1,3-dioxolane-2,8' (5'*H*)-[2*H-*2,4a]methanonaphth alene], | Ysamber | 0.01470000 |
| 148. | 127-41-3 | 3-Buten-2-one, 4-(2,6,6-trimethyl-2-cyclohexen-1-yl)-, (3*E*)- | Alpha-Ionone | 0.01440000 |
| 149. | 151-05-3 | Benzeneethanol, α,α-dimethyl-, 1-acetate | Dimethyl Benzyl Carbinyl Acetate | 0.01390000 |
| 150. | 2500-83-6 | 4,7-Methano-1*H-*inden-5-ol, 3a,4,5,6,7,7a-hexahydro-, 5-acetate | Flor Acetate | 0.01370000 |
| 151. | 150-84-5 | 6-Octen-1-ol, 3,7-dimethyl-, 1-acetate | Citronellyl acetate | 0.01370000 |
| 152. | 30310-41-9 | 2*H*-Pyran, tetrahydro-2-methyl- | Pelargene | 0.01350000 |
| | | 4-methylene-6-phenyl- | | |
| 153. | 68845-00-1 | Bicyclo[3.3.1]nonan e, 2-ethoxy-2,6,6-trimethyl-9-methylene- | Boisiris | 0.01350000 |
| 154. | 106-24-1 | 2,6-Octadien-1-ol, 3,7-dimethyl-, (2*E*)- | Geraniol | 0.01330000 |
| 155. | 106-25-2 | 2,6-Octadien-1-ol, 3,7-dimethyl-, (2*Z*)- | Nerol | 0.01330000 |
| 156. | 75975-83-6 | Bicyclo[7.2.0]undec -4-ene, 4,11,11-trimethyl-8-methylene-, (1*R*,4*E*,9*S*)*-* | Vetyvenal | 0.01280000 |
| 157. | 19870-74-7 | 1*H*-3a,7-Methanoazulene, octahydro-6-methoxy-3,6,8,8-tetramethyl-, (3*R*,3a*S*,6*S*,7*R*,8a*S*)- | Cedryl methyl ether | 0.01280000 |
| 158. | 87-44-5 | Bicyclo[7.2.0]undec -4-ene, 4,11,11-trimethyl-8-methylene-, (1*R*,4*E*,9*S*)*-* | Caryophyllene Extra | 0.01280000 |
| 159. | 54440-17-4 | 1*H*-Inden-1-one, 2,3-dihydro-2,3,3-trimethyl- | Safraleine | 0.01260000 |
| 160. | 110-98-5 | 2-Propanol, 1,1'-oxybis- | Dipropylene Glycol | 0.01250000 |
| 161. | 41890-92-0 | 2-Octanol, 7-methoxy-3,7-dimethyl- | Osyrol^{®} | 0.01250000 |
| 162. | 71077-31-1 | 4,9-Decadienal, 4,8-dimethyl- | Floral Super | 0.01230000 |
| 163. | 65-85-0 | Benzoic Acid | Benzoic Acid | 0.01220000 |
| 164. | 61444-38-0 | 3-Hexenoic acid, (3*Z*)-3-hexen-1-yl ester, (3*Z*)- | cis-3-hexenyl-cis-3-hexenoate | 0.01220000 |
| 165. | 116044-44-1 | Bicyclo[2.2.1]hept-5-ene-2-carboxylic acid, 3-(1- | Herbanate | 0.01210000 |
| 166. | 104-54-1 | 2-Propen-1-ol, 3-phenyl- | Cinnamic Alcohol | 0.01170000 |
| 167. | 78-35-3 | Propanoic acid, 2-methyl-, 1-ethenyl-1,5-dimethyl-4-hexen-1-yl ester | Linalyl Isobutyrate | 0.01170000 |
| 168. | 23495-12-7 | Ethanol, 2-phenoxy-, 1-propanoate | Phenoxy Ethyl Propionate | 0.01130000 |
| 169. | 103-26-4 | 2-Propenoic acid, 3-phenyl-, methyl ester | Methyl Cinnamate | 0.01120000 |
| 170. | 67634-14-4 | Benzenepropanal, 2-ethyl-α,α-dimethyl- | Florazon (ortho-isomer) | 0.01110000 |
| 171. | 5454-19-3 | Propanoic acid, decyl ester | *N*-Decyl Propionate | 0.01100000 |
| 172. | 93-16-3 | Benzene, 1,2-dimethoxy-4-(1-propen-1-yl)- | Methyl Iso Eugenol | 0.01100000 |
| 173. | 81782-77-6 | 3-Decen-5-ol, 4-methyl- | 4-Methyl-3-decen-5-ol | 0.01070000 |
| 174. | 67845-30-1 | Bicyclo[2.2.2]oct-5-ene-2-carboxaldehyde, 6-methyl-8-(1-methylethyl)- | Maceal | 0.01060000 |
| 175. | 97-53-0 | Phenol, 2-methoxy-4-(2-propen-1-yl)- | Eugenol | 0.01040000 |
| 176. | 120-57-0 | 1,3-Benzodioxole-5-carboxaldehyde | Heliotropin | 0.01040000 |
| 177. | 93-04-9 | Naphthalene, 2-methoxy- | Beta Naphthyl Methyl Ether Extra 99 | 0.01040000 |
| 178. | 4826-62-4 | 2-Dodecenal | 2 Dodecene-1-al | 0.01020000 |
| 179. | 20407-84-5 | 2-Dodecenal, (2*E*)- | Aldehyde Mandarin | 0.01020000 |
| 180. | 5462-06-6 | Benzenepropanal, 4-methoxy-α-methyl- | Canthoxal | 0.01020000 |
| 181. | 94-60-0 | 1,4-Cyclohexanedicarbo | Dimethyl 1,4-cyclohexanedicar boxylate | 0.01020000 |
| | | xylic acid, 1,4-dimethyl ester | | |
| 182. | 57378-68-4 | 2-Buten-1-one, 1-(2,6,6-trimethyl-3-cyclohexen-1-yl)- | delta-Damascone | 0.01020000 |
| 183. | 17283-81-7 | 2-Butanone, 4-(2,6,6-trimethyl-1-cvclohexen-1-vl)- | Dihydro Beta Ionone | 0.01020000 |
| 184. | 1885-38-7 | 2-Propenenitrile, 3-phenyl-, (2*E*)- | Cinnamalva | 0.01010000 |
| 185. | 103-48-0 | Propanoic acid, 2-methyl-, 2-phenylethyl ester | Phenyl Ethyl Iso Butyrate | 0.00994000 |
| 186. | 488-10-8 | 2-Cyclopenten-1-one, 3-methyl-2-(2*Z*)-2-penten-1-yl- | Cis Jasmone | 0.00982000 |
| 187. | 7492-67-3 | Acetaldehyde, 2-[(3,7-dimethyl-6-octen-1-yl)oxy]- | Citronellyloxyace taldehyde | 0.00967000 |
| 188. | 68683-20-5 | 1-Cyclohexene-1-ethanol, 4-(1-methylethyl)-, 1-formate | Iso Bergamate | 0.00965000 |
| 189. | 3025-30-7 | 2,4-Decadienoic acid, ethyl ester, (2*E*,4*Z*)- | Ethyl 2,4-Decadienoate | 0.00954000 |
| 190. | 103-54-8 | 2-Propen-1-ol, 3-phenyl-, 1-acetate | Cinnamyl Acetate | 0.00940000 |
| 191. | 18127-01-0 | Benzenepropanal, 4-(1,1-dimethylethyl)- | Bourgeonal | 0.00934000 |
| 192. | 3738-00-9 | Naphtho[2,1-b]furan, dodecahydro-3a,6,6,9a-tetramethyl- | Ambrox^{®} or Cetalox^{®} or Synambran | 0.00934000 |
| 193. | 51519-65-4 | 1,4-Methanonaphthalen-5(1*H*)-one, 4,4a,6,7,8,8a-hexahydro- | Tamisone | 0.00932000 |
| 194. | 148-05-1 | Dodecanoic acid, 12-hydroxy-, λ-lactone (6CI,7CI); 1,12- | Dodecalactone | 0.00931000 |
| 195. | 6790-58-5 | (3a*R*,5a*S*,9a*S*,9b*R*)-3a,6,6,9a-tetramethyl- | Ambronat^{®} or Ambroxan^{®} | 0.00930000 |
| | | 2,4,5,5a,7,8,9,9b-octahydro-1*H-*benzo[e][1]benzofur an | | |
| 196. | 86-26-0 | 1,1'-Biphenyl, 2-methoxy- | Methyl Diphenyl Ether | 0.00928000 |
| 197. | 68738-94-3 | 2-Naphthalenecarboxa ldehyde, octahydro-8,8-dimethyl | Cyclomyral^{®} | 0.00920000 |
| 198. | 2705-87-5 | Cyclohexanepropan oic acid, 2-propen-1-yl ester | Allyl Cyclohexane Propionate | 0.00925000 |
| 199. | 7011-83-8 | 2(3*H*)-Furanone, 5-hexyldihydro-5-methyl- | Lactojasmone^{®} | 0.00885000 |
| 200. | 61792-11-8 | 2,6-Nonadienenitrile, 3,7-dimethyl- | Lemonile^{®} | 0.00884000 |
| 201. | 692-86-4 | 10-Undecenoic acid, ethyl ester | Ethyl Undecylenate | 0.00882000 |
| 202. | 103-95-7 | Benzenepropanal, α-methyl-4-(1-methylethyl)- | Cymal | 0.00881000 |
| 203. | 13019-22-2 | 9-Decen-1-ol | Rosalva | 0.00879000 |
| 204. | 94201-19-1 | 1-Oxaspiro[4.5]decan-2-one, 8-methyl- | Methyl Laitone 10% TEC | 0.00872000 |
| 205. | 104-61-0 | 2(3*H*)-Furanone, dihydro-5-pentyl- | γ-Nonalactone | 0.00858000 |
| 206. | 706-14-9 | 2(3*H*)-Furanone, 5-hexyldihydro- | γ -Decalactone | 0.00852000 |
| 207. | 24720-09-0 | 2-Buten-1-one, 1-(2,6,6-trimethyl-2-cyclohexen-1-yl)-, (2*E*)- | α-Damascone | 0.00830000 |
| 208. | 39872-57-6 | 2-Buten-1-one, 1-(2,4,4-trimethyl-2-cyclohexen-1-yl)-, (2*E*)- | Isodamascone | 0.00830000 |
| 209. | 705-86-2 | 2*H*-Pyran-2-one, tetrahydro-6-pentyl- | Decalactone | 0.00825000 |
| 210. | 67634-15-5 | Benzenepropanal, 4-ethyl-α,α-dimethyl- | Floralozone | 0.00808000 |
| 211. | 40527-42-2 | 1,3-Benzodioxole, 5-(diethoxymethyl)- | Heliotropin Diethyl Acetal | 0.00796000 |
| 212. | 56973-85-4 | 4-Penten-1-one, 1-(5,5-dimethyl-1-cyclohexen-1-yl)- | Neobutenone α | 0.00763000 |
| 213. | 128-51-8 | Bicyclo[3.1.1]hept-2-ene-2-ethanol, 6,6-dimethyl-, 2-acetate | Nopyl Acetate | 0.00751000 |
| 214. | 103-36-6 | 2-Propenoic acid, 3-phenyl-, ethyl ester | Ethyl Cinnamate | 0.00729000 |
| 215. | 5182-36-5 | 1,3-Dioxane, 2,4,6-trimethyl-4-phenyl- | Floropal^{®} | 0.00709000 |
| 216. | 42604-12-6 | Cyclododecane, (methoxymethoxy)- | Boisambrene | 0.00686000 |
| 217. | 33885-52-8 | Bicyclo[3.1.1]hept-2-ene-2-propanal, α,α,6,6-tetramethyl- | Pinyl Iso Butyrate Alpha | 0.00685000 |
| 218. | 92015-65-1 | 2(3H)-Benzofuranone, hexahydro-3,6-dimethyl- | Natactone | 0.00680000 |
| 219. | 63767-86-2 | Cyclohexanemethan ol, α-methyl-4-(1-methylethyl)- | Mugetanol | 0.00678000 |
| 220. | 3288-99-1 | Benzeneacetonitrile, 4-(1,1-dimethylethyl)- | Marenil CI | 0.00665000 |
| 221. | 35044-68-9 | 2-Buten-1-one, 1-(2,6,6-trimethyl-1-cyclohexen-1-yl)- | beta-Damascone | 0.00655000 |
| 222. | 41724-19-0 | 1,4-Methanonaphthalen-6(2*H*)-one, octahydro-7-methyl | Plicatone | 0.00652000 |
| 223. | 75147-23-8 | Bicyclo[3.2.1]octan-8-one, 1,5-dimethyl-, oxime | Buccoxime^{®} | 0.00647000 |
| 224. | 25634-93-9 | 2-Methyl-5-phenylpentan-1-ol | Rosaphen^{®} 600064 | 0.00637000 |
| 225. | 55066-48-3 | 3-Methyl-5-phenylpentanol | Phenyl Hexanol | 0.00637000 |
| | | | | |
| 226. | 495-62-5 | Cyclohexene, 4-(1,5-dimethyl-4-hexen-1-vlidene)-1-methyl- | Bisabolene | 0.00630000 |
| 227. | 2785-87-7 | Phenol, 2-methoxy-4-propyl- | Dihydro Eugenol | 0.00624000 |
| 228. | 87-19-4 | Benzoic acid, 2-hydroxy-, 2-methylpropyl ester | Iso Butyl Salicylate | 0.00613000 |
| 229. | 4430-31-3 | 2*H*-1-Benzopyran-2-one, octahydro- | Octahydro Coumarin | 0.00586000 |
| 230. | 38462-22-5 | Cyclohexanone, 2-(1-mercapto-1-methylethyl)-5-methyl- | Ringonol 50 TEC | 0.00585000 |
| 231. | 77-83-8 | 2-Oxiranecarboxylic acid, 3-methyl-3-phenyl-, ethyl ester | Ethyl Methyl Phenyl Glycidate | 0.00571000 |
| 232. | 37677-14-8 | 3-Cyclohexene-1-carboxaldehyde, 4-(4-methyl-3-penten-1-yl)- | Iso Hexenyl Cyclohexenyl Carboxaldehyde | 0.00565000 |
| 233. | 103-60-6 | Propanoic acid, 2-methyl-, 2-phenoxvethyl ester | Phenoxy Ethyl iso-Butyrate | 0.00562000 |
| 234. | 18096-62-3 | Indeno[1,2-d]-1,3-dioxin, 4,4a,5,9b-tetrahydro- | Indoflor^{®} | 0.00557000 |
| 235. | 63500-71-0 | 2*H*-Pyran-4-ol, tetrahydro-4-methyl-2-(2-methylpropyl)- | Florosa Q/ Florol | 0.00557000 |
| 236. | 65405-84-7 | Cyclohexanebutanal, α,2,6,6-tetramethyl- | Cetonal^{®} | 0.00533000 |
| 237. | 171102-41-3 | 4,7-Methano-1*H-*inden-6-ol, 3a,4,5,6,7,7a-hexahydro-8,8-dimethyl-, 6-acetate | Flor Acetate | 0.00530000 |
| 238. | 10339-55-6 | 1,6-Nonadien-3-ol, 3,7-dimethyl- | Ethyl linalool | 0.00520000 |
| 239. | 23267-57-4 | 3-Buten-2-one, 4-(2,2,6-trimethyl-7-oxabicyclo[4.1.0]he pt-1-vl)- | Ionone Epoxide Beta | 0.00520000 |
| 240. | 97-54-1 | Phenol, 2-methoxy-4-(1-propen-1-yl)- | Isoeugenol | 0.00519000 |
| 241. | 67663-01-8 | 2(3*H*)-Furanone, 5-hexyldihydro-4-methyl- | Peacholide | 0.00512000 |
| 242. | 33885-52-8 | Bicyclo[3.1.1]hept-2-ene-2-propanal, α,α,6,6-tetramethyl- | Pinyl Iso Butyrate Alpha | 0.00512000 |
| 243. | 23696-85-7 | 2-Buten-1-one, 1-(2,6,6-trimethyl-1,3-cyclohexadien-1-yl)- | Damascenone | 0.00503000 |
| 244. | 80-71-7 | 2-Cyclopenten-1-one, 2-hydroxy-3-methyl- | Maple Lactone | 0.00484000 |
| 245. | 67662-96-8 | Propanoic acid, 2,2-dimethyl-, 2-phenylethyl ester | Pivarose Q | 0.00484000 |
| 246. | 2437-25-4 | Dodecanenitrile | Clonal | 0.00480000 |
| 247. | 141-14-0 | 6-Octen-1-ol, 3,7-dimethyl-, 1-propanoate | Citronellyl Propionate | 0.00469000 |
| 248. | 54992-90-4 | 3-Buten-2-one, 4-(2,2,3,6-tetramethylcyclohex vl)- | Myrrhone | 0.00460000 |
| 249. | 55066-49-4 | Benzenepentanal, β-methyl- | Mefranal | 0.00455000 |
| 250. | 7493-74-5 | Acetic acid, 2-phenoxy-, 2-propen-1-vl ester | Allyl Phenoxy Acetate | 0.00454000 |
| 251. | 80-54-6 | Benzenepropanal, 4-(1,1-dimethylethyl)-α-methyl- | Lilial^{®} | 0.00444000 |
| 252. | 86803-90-9 | 4,7-Methano-1*H-*indene-2-carboxaldehyde, octahydro-5-methoxy- | Scentenal^{®} | 0.00439000 |
| 253. | 68991-97-9 | 2-Naphthalenecarboxa ldehyde, 1,2,3,4,5,6,7,8-octahydro-8,8-dimethyl- | Melafleur | 0.00436000 |
| 254. | 18871-14-2 | Pentitol, 1,5-anhydro-2,4-dideoxy-2-pentyl-, 3-acetate | Jasmal | 0.00434000 |
| 255. | 58567-11-6 | Cyclododecane, (ethoxymethoxy)- | Boisambren Forte | 0.00433000 |
| 256. | 94400-98-3 | Naphth[2,3-b]oxirene, 1a,2,3,4,5,6,7,7a-octahydro-1a,3,3,4,6,6-hexamethyl-, (1a*R*,4*S*,7a*S*)-rel- | Molaxone | 0.00425000 |
| 257. | 79-69-6 | 3-Buten-2-one, 4-(2,5,6,6-tetramethyl-2-cyclohexen-1-yl)- | alpha-Irone | 0.00419000 |
| 258. | 65442-31-1 | Quinoline, 6-(1-methylpropyl)- | Iso Butyl Quinoline | 0.00408000 |
| 259. | 87731-18-8 | Carbonic acid, 4-cycloocten-1-yl methyl ester | Violiff | 0.00401000 |
| 260. | 173445-65-3 | 1*H*-Indene-5-propanal, 2,3-dihydro-3,3-dimethyl- | Hivernal (A-isomer) | 0.00392000 |
| 261. | 23911-56-0 | Ethanone, 1-(3-methyl-2-benzofuranvl)- | Nerolione | 0.00383000 |
| 262. | 52474-60-9 | 3-Cyclohexene-1-carboxaldehyde, 1-methyl-3-(4-methyl-3-penten-1-yl)- | Precyclemone B | 0.00381000 |
| 263. | 139539-66-5 | 6-Oxabicyclo [3.2.1]octane, 5-methyl-1-(2,2,3-trimethyl-3-cyclopenten-1-yl)- | Cassifix | 0.00381000 |
| 264. | 80858-47-5 | Benzene, [2-(cyclohexyloxy)ethy l]- | Phenafleur | 0.00380000 |
| 265. | 32764-98-0 | 2*H*-Pyran-2-one, tetrahydro-6-(3-penten-1-yl)- | Jasmolactone | 0.00355000 |
| 266. | 78417-28-4 | 2,4,7-Decatrienoic acid, ethyl ester | Ethyl 2,4,7-decatrienoate | 0.00353000 |
| 267. | 140-26-1 | Butanoic acid, 3-methyl-, 2-phenylethyl ester | Beta Phenyl Ethyl Isovalerate | 0.00347000 |
| 268. | 105-90-8 | 2,6-Octadien-1-ol, 3,7-dimethyl-, 1-propanoate, (2E)- | Geranyl Propionate | 0.003360000 |
| 269. | 41816-03-9 | Spiro[1,4-methanonaphthalene -2(1*H*),2'-oxirane], 3,4,4a,5,8,8a-hexahydro-3',7-dimethyl- | Rhubofix^{®} | 0.00332000 |
| 270. | 7070-15-7 | Ethanol, 2-[[(1*R*,2*R*,4*R*)-1,7,7-trimethylbicyclo[2.2. 1]hept-2-yl]oxy]-, rel- | Arbanol | 0.00326000 |
| 271. | 93-29-8 | Phenol, 2-methoxy-4-(1-propen-1-yl)-, 1-acetate | Iso Eugenol Acetate | 0.00324000 |
| 272. | 476332-65-7 | 2*H*-Indeno[4,5-b]furan, decahydro-2,2,6,6,7,8,8-heptamethyl- | Amber Xtreme Compound 1 | 0.00323000 |
| 273. | 68901-15-5 | Acetic acid, 2-(cyclohexyloxy)-, 2-propen-1-yl ester | Cyclogalbanate | 0.00323000 |
| 274. | 107-75-5 | Octanal, 7-hydroxy-3,7-dimethyl- | Hydroxycitronella l | 0.00318000 |
| 275. | 68611-23-4 | Naphtho[2,1-b]furan, 9b-ethyldodecahydro-3a,7,7-trimethyl- | Grisalva | 0.00305000 |
| 276. | 313973-37-4 | 1,6-Heptadien-3-one, 2-cyclohexyl- | Pharaone | 0.00298000 |
| 277. | 137-00-8 | 5-Thiazoleethanol, 4-methyl- | Sulfurol | 0.00297000 |
| 278. | 7779-30-8 | 1-Penten-3-one, 1-(2,6,6-trimethyl-2-cyclohexen-1-yl)- | Methyl Ionone | 0.00286000 |
| 279. | 127-51-5 | 3-Buten-2-one, 3-methyl-4-(2,6,6-trimethyl-2-cyclohexen-1-yl)- | Isoraldeine Pure | 0.00282000 |
| 280. | 72903-27-6 | 1,4-Cyclohexanedicarbo | Fructalate^{™} | 0.00274000 |
| | | xylic acid, 1,4-diethyl ester | | |
| 281. | 7388-22-9 | 3-Buten-2-one, 4-(2,2-dimethyl-6-methylenecyclohexy l)-3-methyl- | Ionone Gamma Methyl | 0.00272000 |
| 282. | 104-67-6 | 2(3H)-Furanone, 5-heptyldihydro- | gamma-Undecalactone (racemic) | 0.00271000 |
| 283. | 1205-17-0 | 1,3-Benzodioxole-5-propanal, α-methyl- | Helional | 0.00270000 |
| 284. | 33704-61-9 | 4*H*-Inden-4-one, 1,2,3,5,6,7-hexahydro-1,1,2,3,3-pentamethyl- | Cashmeran | 0.00269000 |
| 285. | 36306-87-3 | Cyclohexanone, 4-(1-ethoxyethenyl)-3,3,5,5-tetramethyl- | Kephalis | 0.00269000 |
| 286. | 97384-48-0 | Benzenepropanenitri le, α-ethenyl-α-methyl- | Citrowanil^{®} B | 0.00265000 |
| 287. | 141-13-9 | 9-Undecenal, 2,6,10-trimethyl- | Adoxal | 0.00257000 |
| 288. | 2110-18-1 | Pyridine, 2-(3-phenylpropyl)- | Corps Racine VS | 0.00257000 |
| 289. | 27606-09-3 | Indeno[1,2-d]-1,3-dioxin, 4,4a,5,9b-tetrahydro-2,4-dimethyl- | Magnolan | 0.00251000 |
| | 57082-24-3 | Caryophyllene acetate | Caryophyllene acetate | 0.00025000 |
| 290. | 67634-20-2 | Propanoic acid, 2-methyl-, 3a,4,5,6,7,7a-hexahydro-4,7-methano-1*H*-inden-5-vl ester | Cyclabute | 0.00244000 |
| 291. | 65405-72-3 | 1-Naphthalenol, 1,2,3,4,4a,7,8,8a-octahydro-2,4a,5,8a-tetramethyl-, 1-formate | Oxyoctaline Formate | 0.00236000 |
| 292. | 122-40-7 | Heptanal, 2-(phenylmethylene)- | Amyl Cinnamic Aldehyde | 0.00233000 |
| 293. | 103694-68-4 | Benzenepropanol, β,β,3-trimethyl- | Majantol^{®} | 0.00224000 |
| 294. | 13215-88-8 | 2-Cyclohexen-1-one, 4-(2-buten-1-ylidene)-3,5,5-trimethyl- | Tabanone Coeur | 0.00223000 |
| 295. | 25152-85-6 | 3-Hexen-1-ol, 1-benzoate, (3Z)- | Cis-3-Hexenyl Benzoate | 0.00203000 |
| 296. | 406488-30-0 | 2-Ethyl-*N*-methyl-*N-*(m-tolyl)butanamide | Paradisamide | 0.00200000 |
| 297. | 121-33-5 | Benzaldehyde, 4-hydroxy-3-methoxy- | Vanillin | 0.00194000 |
| 298. | 77-54-3 | 1*H*-3a,7-Methanoazulen-6-ol, octahydro-3,6,8,8-tetramethyl-, 6-acetate, (3*R*,3a*S*,6*R*,7*R*,8a*S*)- | Cedac | 0.00192000 |
| 299. | 76842-49-4 | 4,7-Methano-1*H-*inden-6-ol, 3a,4,5,6,7,7a-hexahydro-8,8-dimethyl-, 6-propanoate | Frutene | 0.00184000 |
| 300. | 121-39-1 | 2-Oxiranecarboxylic acid, 3-phenyl-, ethyl ester | Ethyl Phenyl Glycidate | 0.00184000 |
| 301. | 211299-54-6 | 4*H*-4a,9-Methanoazuleno [5,6 -d]-1,3-dioxole, octahydro-2,2,5,8,8,9a-hexamethyl-, (4a*R*,5*R*,7a*S*,9*R*)- | Ambrocenide^{®} | 0.00182000 |
| 302. | 285977-85-7 | (2,5-Dimethyl-1,3-dihydroinden-2-vl)methanol | Lilyflore | 0.00180000 |
| 303. | 10094-34-5 | Butanoic acid, 1,1-dimethyl-2-phenylethyl ester | Dimethyl Benzyl Carbinyl Butyrate | 0.00168000 |
| 304. | 40785-62-4 | Cyclododeca[c] furan, 1,3,3a,4,5,6,7, 8,9,10,11,13a-dodecahydro- | Muscogene | 0.00163000 |
| 305. | 75490-39-0 | Benzenebutanenitril e, α,α,γ-trimethyl- | Khusinil | 0.00162000 |
| | | | | |
| 306. | 55418-52-5 | 2-Butanone, 4-(1,3-benzodioxol-5-yl)- | Dulcinyl | 0.00161000 |
| 307. | 3943-74-6 | Benzoic acid, 4-hydroxy-3-methoxy-, methyl ester | Carnaline | 0.00157000 |
| 308. | 72089-08-8 | 3-Cyclopentene-1-butanol, β,2,2,3-tetramethyl- 2-Methyl-4-(2,2,3-trimethyl-3-cyclopenten-1-yl)butanol | Brahmanol^{®} | 0.00154000 |
| 309. | 3155-71-3 | 2-Butenal, 2-methyl-4-(2,6,6-trimethyl-1-cyclohexen-1-vl)- | Boronal | 0.00147000 |
| 310. | 2050-08-0 | Benzoic acid, 2-hydroxy-, pentyl ester | Amyl Salicylate | 0.00144000 |
| 311. | 41199-20-6 | 2-Naphthalenol, decahydro-2,5,5-trimethyl- | Ambrinol | 0.00140000 |
| 312. | 12262-03-2 | ndecanoic acid, 3-methylbutyl ester | Iso Amyl Undecylenate | 0.00140000 |
| 313. | 107-74-4 | 1,7-Octanediol, 3,7-dimethyl- | Hydroxyol | 0.00139000 |
| 314. | 91-64-5 | 2*H*-1-Benzopyran-2-one | Coumarin | 0.00130000 |
| 315. | 68901-32-6 | 1,3-Dioxolane, 2-[6-methyl-8-(1-methylethyl)bicyclo[ 2.2.2]oct-5-en-2-yl]- | Glycolierral | 0.00121000 |
| 316. | 68039-44-1 | Propanoic acid, 2,2-dimethyl-, 3a,4,5,6, 7,7a-hexahydro-4,7-methano-1*H*-inden-6-vl ester | Pivacyclene | 0.00119000 |
| 317. | 106-29-6 | Butanoic acid, (2*E*)-3,7-dimethyl-2,6-octadien-1-vl ester | Geranyl Butyrate | 0.00116000 |
| 318. | 5471-51-2 | 2-Butanone, 4-(4-hydroxyphenyl)- | Raspberry ketone | 0.00106000 |
| 319. | 109-42-2 | 10-Undecenoic acid, butyl ester | Butyl Undecylenate | 0.00104000 |
| 320. | 2785-89-9 | 4-Ethyl-2-methoxyphenol | 4-Ethylguaiacol | 0.02000000 |
| | 27538-10-9 | 2-ethyl-4-hydroxy-5-methylfuran-3-one | Homofuronol | 0.01210000 |

| | | | | |
|---|---|---|---|---|
| * Vapor Pressures are acquired as described in the Test Methods Section. ** Origin: Same as for Table 1 hereinabove. | | | | |

**Table 2B--Moderate Volatile Natural Oils.**

| **No.** | **Natural oil** | **Supplier** |
|---|---|---|
| 1. | Bay Oil Terpeneless | IFF |
| 2. | Cade Oil | H. Revnaud & Fils |
| 3. | Cedar Atlas Oil | Robertet |
| 4. | Cinnamon Bark Oil | Robertet |
| 5. | Cinnamon Oleoresin | Citrus & Allied Essences |
| 6. | Clove Bud Oil | Robertet |
| 7. | Clove Leaf Oil Rectified | H. Reynaud & Fils |
| 8. | Clove Stem Oil | H. Revnaud & Fils |
| 9. | Davana Oil | Robertet |
| 10. | Geranium Bourbon | Robertet |
| 11. | Ginger Oil Fresh Madagascar | IFF |
| 12. | Hay Absolute MD 50 PCT | IFF |
| 13. | Juniperberry Oil T'less | Robertet |
| 14. | Papyrus Oil | Robertet |
| 15. | Rose Absolute Oil | Robertet |
| 16. | Tonka Bean Absolute | Robertet |
| 17. | Wormwood Oil | Robertet |

### Suppliers:

Citrus & Allied Essences, New York, USA
H. Reynaud & Fils, Montbrun-les-Bains, France
IFF, Hazlet, New Jersey, USA
Robertet, Grasse, France

Moderate volatile fragrance materials can eselected from the group of Tables 2A or 2B. However, it is understood by one skilled in the art that other moderate volatile fragrance materials, not recited in Tables 2A or 2B, would also fall within the scope of the present invention, so long as they have a vapor pressure of 0.1 to 0.001 Torr at 25 °C.

### (iii) High Volatile Fragrance Materials

The fragrance component includes at least one high volatile fragrance material having a vapor pressure greater than 0.1 Torr (0.0133 kPa) at 25 °C. In some examples, the high volatile fragrance material can include at least 2 high volatile fragrance materials, 3 high volatile fragrance materials, or at least 5 high volatile fragrance materials, or at least 7 high volatile fragrance materials. The high volatile fragrance material can be present in an amount greater than 30 wt% of the fragrance component, greater than 40 wt%, greater than 50 wt%, greater than 60 wt%, 31 wt% to 60 wt%, 40 wt% to 50 wt%, or equal to, or greater than 30 wt%, or less than, equal to, or greater than 31, 35, 40, 45, 50, 55, 60, 65, or 70 wt%. If there are more than one high volatile fragrance materials, then the ranges provided hereinabove cover the total of all of the high volatile fragrance materials. Suitable examples of high volatile fragrances materials are provided in Tables 3A and 3B below.

Preferably, the high volatile fragrance material is selected from at least 1 material, or at least 2 materials, or at least 3 materials, or at least 5 materials, at least 7 materials, or at least 9 high volatile fragrance materials as disclosed in Table 3A. Natural fragrance materials or oils having an aggregrate vapour pressure greater than 0.1 Torr (0.0133 kPa) at 25 °C are provided in Table 3B. Moderate Volatile Natural Oils.

**Table 3A - High Volatile Fragrance Materials**

| **No.** | **CAS Number** | **IUPAC Name** | **Common Name**** | **Vapor Pressure (Torr at 25 °C)*** |
|---|---|---|---|---|
| 1. | 107-31-3 | Formic acid, methyl ester | Methyl Formate | 732.00000000 |
| 2. | 75-18-3 | Methane, 1,1'-thiobis- | Dimethyl Sulfide 1.0% In DEP | 647.00000000 |
| 3. | 141-78-6 | Acetic acid ethyl ester | Ethyl Acetate | 112.00000000 |
| 4. | 105-37-3 | Propanoic acid, ethyl ester | Ethyl Propionate | 44.50000000 |
| 5. | 110-19-0 | Acetic acid, 2-methylpropyl ester | Isobutyl Acetate | 18.00000000 |
| 6. | 105-54-4 | Butanoic acid, ethyl ester | Ethyl Butyrate | 13.90000000 |
| 7. | 14765-30-1 | 1-Butanol | Butyl Alcohol | 8.52000000 |
| 8. | 7452-79-1 | Butanoic acid, 2-methyl-, ethyl ester | Ethyl-2-Methyl Butyrate | 7.85000000 |
| 9. | 123-92-2 | 1-Butanol, 3-methyl-, 1-acetate | Iso Amyl Acetate | 5.68000000 |
| 10. | 66576-71-4 | Butanoic acid, 2-methyl-, 1-methylethyl ester | Iso Propyl 2-Methylbutyrate | 5.10000000 |
| 11. | 110-43-0 | 2-Heptanone | Methyl Amyl Ketone | 4.73000000 |
| 12. | 6728-26-3 | 2-Hexenal, (2*E*)- | Trans-2 Hexenal | 4.62000000 |
| 13. | 123-51-3 | 1-Butanol, 3-methyl- | Isoamyl Alcohol | 4.16000000 |
| 14. | 1191-16-8 | 2-Buten-1-ol, 3-methyl-, 1-acetate | Prenyl acetate | 3.99000000 |
| 15. | 57366-77-5 | 1,3-Dioxolane-2-methanamine, *N-*methyl- | Methyl Dioxolan | 3.88000000 |
| 16. | 7785-70-8 | Bicyclo[3.1.1]he pt-2-ene, 2,6,6-trimethyl-, (1*R*,5*R*)*-* | Alpha Pinene | 3.49000000 |
| 17. | 79-92-5 | Bicyclo[2.2.1]he ptane, 2,2-dimethyl-3-methylene- | Camphene | 3.38000000 |
| 18. | 94087-83-9 | 2-Butanethiol, 4-methoxy-2-methyl- | 4-Methoxy-2-Methyl-2-Butanenthiol | 3.31000000 |
| 19. | 39255-32-8 | Pentanoic acid, 2-methyl-, ethyl ester | Manzanate | 2.91000000 |
| 20. | 3387-41-5 | Bicyclo[3.1.0]he xane, 4-methylene-1-(1-methylethyl)- | Sabinene | 2.63000000 |
| 21. | 127-91-3 | Bicyclo[3.1.1]he ptane, 6,6-dimethyl-2-methylene- | Beta Pinene | 2.40000000 |
| 22. | 105-68-0 | 1-Butanol, 3-methyl-, 1-propanoate | Amyl Propionate | 2.36000000 |
| 23. | 123-35-3 | 1,6-Octadiene, 7-methyl-3-methylene- | Myrcene | 2.29000000 |
| 24. | 124-13-0 | Octanal | Octyl Aldehyde | 2.07000000 |
| 25. | 7392-19-0 | 2*H*-Pyran, 2-ethenyltetrahydr o-2,6,6-trimethyl- | Limetol | 1.90000000 |
| 26. | 111-13-7 | 2-Octanone | Methyl Hexyl Ketone | 1.72000000 |
| 27. | 123-66-0 | Hexanoic acid, ethyl ester | Ethyl Caproate | 1.66000000 |
| 28. | 470-82-6 | 2-Oxabicyclo[2.2.2 ]octane, 1,3,3-trimethyl- | Eucalyptol | 1.65000000 |
| 29. | 99-87-6 | Benzene, 1-methyl-4-(1-methylethyl)- | Para Cymene | 1.65000000 |
| 30. | 104-93-8 | Benzene, 1-methoxy-4-methyl- | Para Cresyl Methyl Ether | 1.65000000 |
| 31. | 13877-91-3 | 1,3,6-Octatriene, 3,7-dimethyl- | Ocimene | 1.56000000 |
| 32. | 138-86-3 | Cyclohexene, 1-methyl-4-(1-methylethenyl)- | dl-Limonene | 1.54000000 |
| 33. | 5989-27-5 | Cyclohexene, 1-methyl-4-(1-methylethenyl)-, (4*R*)*-* | d-limonene | 1.54000000 |
| 34. | 106-68-3 | 3-Octanone | Ethyl Amyl Ketone | 1.50000000 |
| 35. | 110-41-8 | Undecanal, 2-methyl- | Methyl Nonyl Acetaldehyde | 1.43000000 |
| 36. | 142-92-7 | Acetic acid, hexyl ester | Hexyl acetate | 1.39000000 |
| 37. | 110-93-0 | 5-Hepten-2-one, 6-methyl- | Methyl Heptenone | 1.28000000 |
| 38. | 81925-81-7 | 2-Hepten-4-one, 5-methyl- | Filbertone 1% in TEC | 1.25000000 |
| 39. | 3681-71-8 | 3-Hexen-1-ol, 1-acetate, (3*Z*)- | cis-3-Hexenyl acetate | 1.22000000 |
| 40. | 97-64-3 | Propanoic acid, 2-hydroxy-, ethyl ester | Ethyl Lactate | 1.16000000 |
| 41. | 586-62-9 | Cyclohexene, 1-methyl-4-(1-methylethylidene )- | Terpineolene | 1.13000000 |
| 42. | 51115-64-1 | Butanoic acid, 2-methylbutyl ester | Amyl butyrate | 1.09000000 |
| 43. | 106-27-4 | Butanoic acid, 3-methylbutyl ester | Amyl Butyrate | 1.09000000 |
| 44. | 99-85-4 | 1,4-Cyclohexadiene, 1-methyl-4-(1-methylethyl)- | Gamma Terpinene | 1.08000000 |
| 45. | 18640-74-9 | Thiazole, 2-(2-methylpropyl)- | 2-Isobutylthiazole | 1.07000000 |
| 46. | 928-96-1 | 3-Hexen-1-ol, (3Z)- | cis-3-Hexenol | 1.04000000 |
| 47. | 100-52-7 | Benzaldehyde | Benzaldehyde | 0.97400000 |
| 48. | 141-97-9 | Butanoic acid, 3-oxo-, ethyl ester | Ethyl Acetoacetate | 0.89000000 |
| 49. | 928-95-0 | 2-Hexen-1-ol, (2*E*)- | Trans-2-Hexenol | 0.87300000 |
| 50. | 928-94-9 | 2-Hexen-1-ol, (2Z)- | Beta Gamma Hexenol | 0.87300000 |
| 51. | 24691-15-4 | Cyclohexane, 3-ethoxy-1,1,5-trimethyl-, cis-(9CI) | Herbavert | 0.85200000 |
| 52. | 19872-52-7 | 2-Pentanone, 4-mercapto-4-methyl- | 4-Methyl-4-Mercaptopentan-2-one 1ppm TEC | 0.84300000 |
| 53. | 3016-19-1 | 2,4,6-Octatriene, 2,6-dimethyl-, (4*E*,6*E*)- | Allo-Ocimene | 0.81600000 |
| 54. | 69103-20-4 | Oxirane, 2,2-dimethyl-3-(3-methyl-2,4-pentadien-1-yl)- | Myroxide | 0.80600000 |
| 55. | 189440-77-5 | 4,7-Octadienoic acid, methyl ester, (4E)- | Anapear | 0.77700000 |
| 56. | 67633-96-9 | Carbonic acid, (3*Z*)-3-hexen-1-yl methyl ester | Liffarome^{™} | 0.72100000 |
| 57. | 123-68-2 | Hexanoic acid, 2-propen-1-yl ester | Allyl Caproate | 0.67800000 |
| 58. | 106-72-9 | 5-Heptenal, 2,6-dimethyl- | Melonal | 0.62200000 |
| 59. | 106-30-9 | Heptanoic acid, ethyl ester | Ethyl Oenanthate | 0.60200000 |
| 60. | 68039-49-6 | 3-Cyclohexene-1-carboxaldehyde, 2,4-dimethyl- | Ligustral or Triplal | 0.57800000 |
| 61. | 101-48-4 | Benzene, (2,2-dimethoxyethyl)- | Phenyl Acetaldehyde Dimethyl Acetal | 0.55600000 |
| 62. | 16409-43-1 | 2*H*-Pyran, tetrahydro-4-methyl-2-(2-methyl-1-propen-1-yl)- | Rose Oxide | 0.55100000 |
| 63. | 925-78-0 | 3-Nonanone | Ethyl Hexyl Ketone | 0.55100000 |
| 64. | 100-47-0 | Benzonitrile | Benzyl Nitrile | 0.52400000 |
| 65. | 589-98-0 | 3-Octanol | Octanol-3 | 0.51200000 |
| 66. | 58430-94-7 | 1-Hexanol, 3,5,5-trimethyl-, 1-acetate | Iso Nonyl Acetate | 0.47000000 |
| 67. | 10250-45-0 | 4-Heptanol, 2,6-dimethyl-, 4-acetate | Alicate | 0.45400000 |
| 68. | 105-79-3 | Hexanoic acid, 2-methylpropyl ester | Iso Butyl Caproate | 0.41300000 |
| 69. | 2349-07-7 | Propanoic acid, 2-methyl-, hexyl ester | Hexyl isobutyrate | 0.41300000 |
| 70. | 23250-42-2 | Cyclohexanecarb oxylic acid, 1,4-dimethyl-, methyl ester, trans- | Cyprissate | 0.40500000 |
| 71. | 122-78-1 | Benzeneacetalde hyde | Phenyl acetaldehyde | 0.36800000 |
| 72. | 5405-41-4 | Butanoic acid, 3-hydroxy-, ethyl ester | Ethyl-3-Hydroxy Butyrate | 0.36200000 |
| 73. | 105-53-3 | Propanedioic acid, 1,3-diethyl ester | Diethyl Malonate | 0.34400000 |
| 74. | 93-58-3 | Benzoic acid, methyl ester | Methyl Benzoate | 0.34000000 |
| 75. | 16356-11-9 | 1,3,5-Undecatriene | Undecatriene | 0.33600000 |
| 76. | 65405-70-1 | 4-Decenal, (4*E*)- | Decenal (Trans-4) | 0.33100000 |
| 77. | 54546-26-8 | 1,3-Dioxane, 2-butyl-4,4,6-trimethyl- | Herboxane | 0.33000000 |
| 78. | 13254-34-7 | 2-Heptanol, 2,6-dimethyl- | Dimethyl-2 6-Heptan-2-ol | 0.33000000 |
| 79. | 98-86-2 | Ethanone, 1-phenyl- | Acetophenone | 0.29900000 |
| 80. | 93-53-8 | Benzeneacetalde hyde, α-methyl- | Hydratropic aldehyde | 0.29400000 |
| 81. | 80118-06-5 | Propanoic acid, 2-methyl-, 1,3-dimethyl-3-buten-1-yl ester | Iso Pentyrate | 0.28500000 |
| 82. | 557-48-2 | 2,6-Nonadienal, (2*E*,6*Z*)- | E Z-2,6-Nonadien-1-al | 0.28000000 |
| | | | | |
| 83. | 24683-00-9 | Pyrazine, 2-methoxy-3-(2-methylpropyl)- | 2-Methoxy-3-Isobutyl Pyrazine | 0.27300000 |
| 84. | 104-57-4 | Formic acid, phenylmethyl ester | Benzyl Formate | 0.27300000 |
| 85. | 104-45-0 | Benzene, 1-methoxy-4-propyl- | Dihydroanethole | 0.26600000 |
| 86. | 491-07-6 | Cyclohexanone, 5-methyl-2-(1-methylethyl)-, (2*R*,5*R*)-rel- | Iso Menthone | 0.25600000 |
| 87. | 89-80-5 | Cyclohexanone, 5-methyl-2-(1-methylethyl)-, (2*R*,5*S*)-rel*-* | Menthone Racemic | 0.25600000 |
| 88. | 2463-53-8 | 2-Nonenal | 2 Nonen-1-al | 0.25600000 |
| 89. | 55739-89-4 | Cyclohexanone, 2-ethyl-4,4-dimethyl- | Thuyacetone | 0.25000000 |
| 90. | 150-78-7 | Benzene, 1,4-dimethoxy- | Hydroquinone Dimethyl Ether | 0.25000000 |
| 91. | 64988-06-3 | Benzene, 1-(ethoxymethyl)-2-methoxy- | Rosacene | 0.24600000 |
| 92. | 76-22-2 | Bicyclo[2.2.1]he ptan-2-one, 1,7,7-trimethyl- | Camphor gum | 0.22500000 |
| 93. | 67674-46-8 | 2-Hexene, 6,6-dimethoxy-2,5,5-trimethyl- | Methyl Pamplemousse | 0.21400000 |
| 94. | 112-31-2 | Decanal | Decyl Aldehyde | 0.20700000 |
| 95. | 16251-77-7 | Benzenepropanal, β-methyl- | Trifernal | 0.20600000 |
| 96. | 93-92-5 | Benzenemethano 1, α-methyl-, 1-acetate | Methylphenylcarbi nol Acetate | 0.20300000 |
| 97. | 143-13-5 | Acetic acid, nonyl ester | Nonyl Acetate | 0.19700000 |
| 98. | 122-00-9 | Ethanone, 1-(4-methylphenyl)- | Para Methyl Acetophenone | 0.18700000 |
| | | | | |
| 99. | 24237-00-1 | 2H-Pyran, 6-butyl-3,6-dihydro-2,4-dimethyl- | Gyrane | 0.18600000 |
| 100. | 41519-23-7 | Propanoic acid, 2-methyl-, (3Z)-3-hexen-1-yl ester | Hexenyl Isobutyrate | 0.18200000 |
| 101. | 93-89-0 | Benzoic acid, ethyl ester | Ethyl Benzoate | 0.18000000 |
| 102. | 20780-48-7 | 3-Octanol, 3,7-dimethyl-, 3-acetate | Tetrahydro Linalyl Acetate | 0.18000000 |
| 103. | 101-41-7 | Methyl 2-phenylacetate | Methylphenyl acetate | 0.17600000 |
| 104. | 40853-55-2 | 1-Hexanol, 5-methyl-2-(1-methylethyl)-, 1-acetate | Tetrahydro Lavandulyl Acetate | 0.17300000 |
| 105. | 933-48-2 | Cyclohexanol, 3,3,5-trimethyl-, *(1R,5R)-rel-* | Trimethylcyclohexa nol | 0.17300000 |
| 106. | 35158-25-9 | 2-Hexenal, 5-methyl-2-(1-methylethyl)- | Lactone of Cis Jasmone | 0.17200000 |
| 107. | 18479-58-8 | 7-Octen-2-ol, 2,6-dimethyl- | Dihydromyrcenol | 0.16600000 |
| 108. | 140-11-4 | Acetic acid, phenylmethyl ester | Benzyl acetate | 0.16400000 |
| 109. | 14765-30-1 | Cyclohexanone, 2-(1-methylpropyl)- | 2-sec-Butyl Cyclo Hexanone | 0.16300000 |
| 110. | 20125-84-2 | 3-Octen-1-ol, (3Z)- | Octenol | 0.16000000 |
| 111. | 142-19-8 | Heptanoic acid, 2-propen-1-yl ester | Allyl Heptoate | 0.16000000 |
| 112. | 100-51-6 | Benzenemethano 1 | Benzyl Alcohol | 0.15800000 |
| 113. | 10032-15-2 | Butanoic acid, 2-methyl-, hexyl ester | Hexyl-2-Methyl Butyrate | 0.15800000 |
| 114. | 695-06-7 | 2(3H)-Furanone, 5-ethyldihydro- | Gamma Hexalactone | 0.15200000 |
| 115. | 21722-83-8 | Cyclohexaneetha nol, 1-acetate | Cyclohexyl Ethyl Acetate | 0.15200000 |
| 116. | 111-79-5 | 2-Nonenoic acid, methyl ester | Methyl-2-Nonenoate | 0.14600000 |
| 117. | 16491-36-4 | Butanoic acid, (3Z)-3-hexen-1-vl ester | Cis 3 Hexenyl Butyrate | 0.13500000 |
| 118. | 111-12-6 | 2-Octynoic acid, methyl ester | Methyl Heptine Carbonate | 0.12500000 |
| 119. | 59323-76-1 | 1,3-Oxathiane, 2-methyl-4-propyl-, (2R,4S)-rel- | Oxane | 0.12300000 |
| 120. | 62439-41-2 | Heptanal, 6-methoxy-2,6-dimethyl- | Methoxy Melonal | 0.11900000 |
| 121. | 13851-11-1 | Bicyclo[2.2.1]he ptan-2-ol, 1,3,3-trimethyl-, 2-acetate | Fenchyl Acetate | 0.11700000 |
| 122. | 115-95-7 | 1,6-Octadien-3-ol, 3,7-dimethyl- , 3-acetate | Linalyl acetate | 0.11600000 |
| 123. | 18479-57-7 | 2-Octanol, 2,6-dimethyl- | Tetra-Hydro Mvrcenol | 0.11500000 |
| 124. | 78-69-3 | 3,7-dimethyloctan-3-ol | Tetra-Hydro Linalool | 0.11500000 |
| 125. | 111-87-5 | 1-Octanol | Octyl Alcohol | 0.11400000 |
| 126. | 71159-90-5 | 3-Cyclohexene-1-methanethiol, α,α,4-trimethyl- | Grapefruit mercaptan | 0.10500000 |
| 127. | 80-25-1 | Cyclohexanemet hanol, α,α,4-trimethyl-, 1-acetate | Menthanyl Acetate | 0.10300000 |
| 128. | 88-41-5 | Cyclohexanol, 2-(1,1-dimethylethyl)-, 1-acetate | Verdox^{™} | 0.10300000 |
| 129. | 32210-23-4 | Cyclohexanol, 4-(1,1-dimethylethyl)-, 1-acetate | Vertenex | 0.10300000 |
| 130. | 112-44-7 | Undecanal | n-Undecanal | 0.10200000 |
| 131. | 124-19-6 | Nonanal | Nonanal Aldehyde C-9 | 0.53200000 |
| 132. | 929253-05-4 | 6-methoxy-2,6-dimethyloctanal | 6-methoxy-2,6-dimethyl octanal | 0.04020000 |
| 133. | 68039-47-4 | 2-propan-2-yloxyethylbenze ne | Phenethyl Isopropyl Ether | 0.24900000 |
| 134. | 6413-10-1 | ethyl 2-(2-methyl-1,3-dioxolan-2-yl)acetate | Apple Ketal | 0.21900000 |
| 135. | 106-23-0 | 3,7-dimethyloct-6-enal | citronellal | 0.21500000 |
| 136. | 14667-55-1 | Trimethyl Pvrazine-2,3,5 | Trimethyl Pyrazine-2,3,5 | 1.72400000 |

| | | | | |
|---|---|---|---|---|
| * Vapor Pressures are acquired as described in the Test Methods Section. ** Origin: Same as for Table 1 hereinabove. | | | | |

**Table 3B- High Volatile Fragrance Materials**

| **No.** | **Natural oil** | **Supplier** |
|---|---|---|
| 1. | Angelica Seeds Oil | Robertet |
| 2. | Basil Oil Grand Vert | IFF |
| 3. | Bergamot Oil Reggio Early New Crop | Capua |
| 4. | Black Pepper Oil | Robertet |
| 5. | Blackcurrant Buds Absolute | Robertet |
| 6. | Cardamom Guatamala Extract CO2 | IFF |
| 7. | Cardamom Oil Guatemala | IFF |
| 8. | Cedarleaf Oil | Kerrv |
| 9. | citronella oil | H. Reynaud & Fils |
| 10. | Clary Sage Oil French | IFF |
| 11. | Coffee Extract CO2 | Firmenich |
| 12. | Cucumber Extract | Firmenich |
| 13. | Cumin Oil | Robertet |
| 14. | Cypress Oil | IFF |
| 15. | Elemi Coeur Oil | Robertet |
| 16. | Ginger oil India | IFF |
| 17. | Grapefruit Zest | Citrus & Allied Essences |
| 18. | It. Bergamot Oil | Capua |
| 19. | Labdanum Cistus Absolute | Biolandes |
| 20. | Lavandin Grosso Oil | H. Revnaud & Fils |
| 21. | Lemon Oil Winter | Capua |
| 22. | Green Mandarin Oil | Simone Gatto |
| 23. | Nutmeg Oil | Robertet |
| 24. | Oil Orange Sinensal | Citrus & Allied Essences |
| 25. | Olibanum Oil Pyrogenous | Firmenich |
| 26. | Pepper Black CO2 Oil | Firmenich |
| 27. | Petitgrain Mandarinier Oil | Misitano & Stracuzzi |
| 28. | Pink Pepper CO2 OIL | Firmenich |
| 29. | Rum CO2 Oil | Firmenich |
| 30. | Sichuan Pepper CO2 oil | Firmenich |
| 31. | Styrax Resoid | IFF |
| 32. | Tangerine Oil | Robertet |
| 33. | Thym Oil | IFF |
| 34. | Violet Leaves Absolute | Robertet |

### Suppliers

Biolandes, Le Sen, France
Capua, Campo Calabro, Italy
Citrus & Allied Essences, New York, USA
Firmenich, Geneva, Switzerland
Global Essence Inc, New Jersey, USA
H. Reynaud & Fils, Montbrun-les-Bains, France
IFF, Hazlet, New Jersey, USA
Kerry, Co. Kerry, Ireland
Mane, Le Bar-sur-Loup, France
Misitano & Stracuzzi, Messina, Italy
Robertet, Grasse, France
Simone Gatto, San Pierre Niceto, Italy

Exemplary high volatile fragrance materials selected from the group of Tables 3A or 3B are preferred. However, it is understood by one skilled in the art that other high volatile fragrance materials, not recited in Tables 3A or 3B, would also fall within the scope of the present invention, so long as they have a vapor pressure of greater than 0.1 Torr (0.0133 kPa) at 25 °C.

### (iv) Fragrance Modulators

The composition further comprises at least one substantially non-odorous fragrance modulator as described herein below. Examples of substantially non-odorous fragrance modulators are provided in Table 4 below.

The substantially non-odorous fragrance modulator can be present in an amount of from 0.1 wt% to 20 wt% relative to the total weight of the composition of the composition, 0.5 wt% to 18 wt%, 2.5 wt% to 15 wt%, or equal to, or greater than 0.1 wt%, or less than, equal to, or greater than 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19 or 19.5 wt% or less than or equal to 20 wt%. If there are more than one substantially non-odorous fragrance modulators, then the ranges provided hereinabove cover the total of all of the substantially non-odorous fragrance modulators.

The substantially non-odorous fragrance modulator can be a liquid at temperatures lower than 100 °C, such as at ambient temperature. The substantially non-odorous fragrance modulators may be fully miscible with the fragrance materials to form a single phase liquid. However, if the fragrance materials are not entirely miscible, or are immiscible, then cosolvents (e.g., dipropylene glycol (DPG), triethyl citrate, or others well known to those skilled in the art) can be added to aid in the solubility of the fragrance materials.

According to various examples, the effect of the substantially non-odorous fragrance modulator on the fragrance profile, particularly the characters of the fragrance profile which is attributable to the high and moderate volatile fragrance materials, can be improved. By "improved" it is meant that the fragrance profile of the composition, particular the components contributed by at least one of the high and moderate volatile fragrance materials, can be perceived by the a panel of experts or professional evaluators or individual experts or professional evaluators at later time points such as, for example, 15 mins, 30 mins, 1 hour, 2 hours, 3 hours, 4 hours, 5 hours, 6 hours, 7 hours, 8 hours, 10 hours, and possibly all the way up to 24 hrs after application as compared to controls, e.g., lacking any of the disclosed non-odorous fragrance modulators such as PPG-20 Methyl Glucose Ether or an equivalent traditional fragrance construction.

Alternatively, by "improved" it can mean that the perception, by the a panel of experts or professional evaluators or individual experts or professional evaluators, of the fidelity of the fragrance profile contributed by the high and moderate volatile fragrance materials is markedly increased or enhanced as compared to the controls. "Increased" or "enhanced" means that the a panel of experts or professional evaluators or individual experts or professional evaluators perceives the fragrance profile, preferably the characters attributable to the high and/or moderate volatile fragrance materials, of a composition as not changing from its initial impression or the changes are minimal from when the composition was first applied to when it dissipates. In other words, the fidelity of the perceived fragrance profile of the composition is maintained over time. In contrast the composition lacking any of the disclosed nom-odorous fragrance modulators or an equivelant traditional fragrance construction will undergo a rapid loss of the characters attributable to the high and/or moderate volatile fragrance materials.

Such a solution as presented herein provides enhanced or improved fidelity and/or longevity of the fragrance profile, particularly amongst those composition formulated from volatile fragrance materials having moderate to high vapor pressure ranges (greater than or equal to 0.001 Torr (0.000133 kPa) at 25 °C), without having to rely on the presence or significant amounts of the low volatile fragrance materials, which has a tendency to overpower and alter the overall fragrance profile, particularly over time. As a result, the present disclosure provides the perfumer options to formulate compositions having new fragrance profiles not possible before.

Additionally, according to some embodiments, the perceived harshness of overdosing of the fragrance material is mitigated or absent, as compared to the same perception in a fragrance in the absence of the modulator.

The non-odorous modulator according to the claimed invention is chosen between methyl glucoside polyol, ethyl glucoside polyol, propyl glucoside polyol, or mixtures thereof.

**Tables 4(a) and 4(b) provide lists of non-odorous fragrance modulators. Table 4(a): Substantially Non-Odorous Fragrance Modulators**

| **No.** | **Group** | **Chemical Name** | **CAS Number** | **Supplier** |
|---|---|---|---|---|
| 1. | | PPG-10 Methyl Glucose Ether | 61849-72-7 | Lubrizol |
| 2. | (a) | PPG-20 Methyl Glucose Ether *¹* | 61849-72-7 | |
| 3. | | Ethoxylated Methyl Glucose Ether *²* | 68239-42-9 | |
| 4. | | Caprylyl/Capryl Glucoside *³* | 68515-73-1 | BASF |
| 5. | | Undecyl Glucoside *^{3a}* | -- | SEPPIC (France) |
| 6. | (b) | Isocetyl Alcohol *⁴* | 36653-82-4 | Ashland Speciality Ingredients |
| 7. | (c) | PPG-3 Myristyl Ether *⁵* | -- | Evonik |
| 8. | | Neopentyl Glycol Diethylhexanoate *⁶* | 28510-23-8 | Lubrizol |
| 9. | (d) | Sucrose Laurate | 25339-99-5 | Alfa Chemicals Ltd. (UK) |
| 10. | | Sucrose dilaurate | 25915-57-5 | Alfa Chemicals Ltd. (UK) |
| 11. | | Sucrose Myristate | 27216-47-3 | Mitsubishi Chemicals |
| 12. | | Sucrose Palmitate | 26446-38-8 | Alfa Chemicals Ltd. (UK) |
| 13. | | Sucrose Stearate | 25168-73-4 | |
| 14. | | Sucrose Distearate | 27195-16-0 | Mitsubishi Chemicals (JP) |
| 15. | | Sucrose Tristearate | 27923063-3 | Mitsubishi Chemicals (JP) |
| 16. | (e) | (E)-1-(2,2,6-trimethylcyclohexyl)oct-1-en-3-one *⁸* | -- | Takasago (Japan) |
| 17. | (f) | 2-(1-menthoxy)ethane-1-ol *⁹* | -- | Takasago (Japan) |
| 18. | | 1-(1-menthoxy)propane-2-ol *⁹* | -- | |
| 19. | | 3-(1-menthoxy)propane-1-ol *⁹* | -- | |
| 20. | | 3-(1-menthoxy)propane-1,2-diol *⁹* | -- | |
| 21. | | 2-methyl-3-(1-menthoxy)propane-1,2-diol *⁹* | -- | |
| 22. | | 4-(1-menthoxy) butane-1-ol *⁹* | -- | |
| 23. | (g) | **1, 1,4,4-tetramethyl-6-acetyl-7-**formyl-1,2,3,4-tetrahydronaphthalene *¹⁰* | -- | Givaudan (Switzerland ) |
| 24. | | 1,1,2,4,4-pentamethyl-6-acetyl-7-formyl-1,2,3,4-tetrahydronaphthalene *¹⁰* | -- | |
| 25. | (h) | Hyaluronic acid disaccharide sodium salt *11* | 9004-61-9 | Sigma Aldrich (UK) |
| 26. | | Sodium Hyaluronate *11* | 9067-32-7 | |
| 27. | | Mono-o-(linalyl)-glucopyranose *12* | -- | |
| 28. | | Di-o-(linalyl)-glucopyranose *¹²* | -- | |
| 29. | | Tri-o-(linalyl)-glucopyranose *¹²* | -- | |
| 30. | | Tetra-o-(linalyl)-glucopyranose *¹²* | -- | |
| 31. | | Penta-o-(linalyl)-glucopyranose *¹²* | -- | |
| 32. | (i) | Mono-o-(cis-3-hexenyl)-glactopyranose *¹²* | -- | Kanebo (Japan) |
| 33. | | Di-o-(cis-3-hexenyl)-glactopyranose *¹²* | -- | |
| 34. | | Tri-o-(cis-3-hexenyl)-glactopyranose *¹²* | -- | |
| 35. | | Tetra-o-(cis-3-hexenyl)-glactopyranose *¹²* | -- | |
| 36. | | Penta-o-(cis-3-hexenyl)-glactopyranose *¹²* | -- | |
| 37. | (j) | Bis-O-(3,6-dioxadecanyl)-glucopyranose *¹³* | -- | |
| 38. | | Tris-O-(3,6-dioxadecanyl)-glucopyranose *¹³* | -- | |
| 39. | | Tetrakis-O-(3,6-dioxadecanyl)-glucopyranose *¹³* | -- | |
| 40. | | Pentakis-O-(3,6-dioxadecanyl)-glucopyranose*¹³* | -- | |
| 41. | | Bis-O-(3,6-dioxaoctanyl)-galactopyranose *¹³* | -- | |
| 42. | | Tris-O-(3,6-dioxaoctanyl)-galactopyranose *¹³* | -- | |
| 43. | | Tetrakis-O-(3,6-dioxaoctanyl)-galactopyranose*¹³* | -- | |
| 44. | | Pentakis-O-(3,6-dioxaoctanyl)-galactopyranose *¹³* | -- | |
| 45. | | Bis-O-(3,6-dioxaheptanyl)-xylopyranose *¹³* | -- | |
| 46. | | Tris-O-(3,6-dioxaheptanyl)-xylopyranose *¹³* | -- | |
| 47. | | Tetrakis-O-(3,6-dioxaheptanyl)-xylopyranose *¹³* | -- | |
| 48. | | Bis-O-(3,6-dioxadodecanyl)-glucopyranose *¹³* | -- | |
| 49. | | Tris-O-(3,6-dioxadodecanyl)-glucopyranose *¹³* | -- | |
| 50. | | Tetrakis-O-(3,6-dioxadodecanyl)-glucopyranose *¹³* | -- | |
| 51. | | Pentakis-O-(3,6-dioxadodecanyl)-glucopyranose*¹³* | -- | |
| 52. | (k) | Hydroquinone beta-D-glycoside *¹⁴* | 497-76-7 | Shiseido |
| 53. | (l) | Propylene Glycol Propyl Ether | 1569-01-3 | |
| 54. | | Dicetyl Ether | 4113-12-6 | Sigma Aldrich (UK) |
| 55. | | Polyglycerin-4 Ethers | 25618-55-7 | Solvay Chemicals |
| 56. | | Isoceteth-5 | 69364-63-2 | Nihon Emulsion Company Ltd. |
| 57. | | Isoceteth-7 | 69364-63-2 | |
| 58. | | Isoceteth-10 | 69364-63-2 | |
| 59. | | Isoceteth-12 | 69364-63-2 | |
| 60. | | Isoceteth-15 | 69364-63-2 | |
| 61. | | Isoceteth-20 | 69364-63-2 | |
| 62. | | Isoceteth-25 | 69364-63-2 | |
| 63. | | Isoceteth-30 | 69364-63-2 | |
| 64. | | Disodium Lauroamphodipropionate | 68929-04-4 | Rhodia |
| 65. | | Hexaethylene glycol monododecyl ether *^{14b}* | 3055-96-7 | Sigma Aldrich (UK) |
| 66. | (m) | Neopentyl Glycol Diisononanoate *¹⁵* | 27841-07-2 | Symrise (Germany) |
| 67. | | Cetearyl Ethylhexnoate *¹⁶* | 90411-68-0 | |
| 68. | (n) | 2-ethylhexyloxypropanediol *¹⁷* | 70455-33-9 | Takasago (JP) |
| 69. | (o) | Panthenol Ethyl Ether *¹⁸* | 667-83-4 | DSM Nutritional Products, Inc. (USA) |
| 70. | | DL-Panthenol | 16485-10-2 | Roche Inc. (USA) |
| 71. | (p) | Diisobutyl Adipate *¹⁹* | 141-04-8 | Sigma Aldrich (UK) |
| 72. | | Diisoamyl Adipate *¹⁹* | 6624-70-0 | |
| 73. | (q) | PPG-11 Stearyl Ether *^{19a}* | 25231-21-4 | Kao (JP) |
| 74. | (r) | N-hexadecyl n-nonanoate *^{19b}* (e.g., cetyl nonanoate) | 72934-15-7 | Symrise (Germany) |
| 75. | | Noctadecyl n-nonanoate *^{19b}* (e.g., stearyl nonanoate) | 107647-13-2 | |
| 76. | | methanone, (morphonyl) tricyclo[3.3.1.1^{3,7}]dec-1-yl- *²⁰* | -- | |
| 77. | | methanone, (piperidinyl) tricyclo[3.3.1.1^{3,7}]dec-1-yl- *²⁰* | -- | |
| 78. | | methanone, (pyrrolidinyl) tricyclo[3.3.1.1^{3,7}]dec-1-yl *²⁰* | -- | |
| 79. | | methanone, (azetidinyl) tricyclo[3.3.1.1^{3,7}]dec-1-yl- *²⁰* | -- | |
| 80. | | methanone, (hexahydroazepinyl) tricyclo[3.3.1.1^{3,7}]dec-1-yl-*²⁰* | -- | |
| 81. | (s) | methanone, (4-cyano-piperidinyl)tricyclo[3.3.1.1^{3,7}]de c-1-yl- *²⁰* | -- | Unilever (UK) |
| 82. | | methanone, (4-amido-piperidinyl)tricyclo[3.3.1.1^{3,7}]de c-1-yl- *²⁰* | -- | |
| 83. | | methanone, (Tricyclo[3.3.1.1^{3,7}]decanyl)-*N-*tricyclo[3.3.1.1^{3,7}]dec-1-yl- *²⁰* | -- | |
| 84. | | methanone, (decahydroisoquinolinyl)tricycl o[3.3.1.1^{3,7}]dec-1-yl- *²⁰* | -- | |
| 85. | | methanone, (decahydroisoquinolinyl)tricycl o[3.3.1.1^{3,7}]dec-1-yl- *²⁰* | -- | |
| 86. | | methanone, (decahydroquinolinyl)tricyclo[3 .3.1.1^{3,7}]dec-1-yl-*20* | -- | |
| 87. | | methanone, (3,3-dimethyl-1-piperidinyl)tricyclo[3.3.1.1^{3,7}] dec-1-yl- *²⁰* | -- | |
| 88. | | methanone, (2-methyl-1-piperidinyl)tricyclo[3.3.1.1^{3,7}] dec-1-yl- *²⁰* | -- | |
| 89. | | methanone, (4-methyl-1-piperidinyl)tricyclo[3.3.1.13,7] dec-1-yl- *²⁰* | -- | |
| 90. | | methanone, (3-methyl-1-piperidinyl)tricyclo[3.3.1.1^{3,7}] dec-1-yl- *²⁰* | -- | |
| 91. | | methanone, (3,5-dimethyl-1-piperidinyl)tricyclo[3.3.1.1^{3,7}] dec-1-yl- *²⁰* | -- | |
| 92. | | methanone, (4-methyl-4-ethy-piperidinyl)tricyclo[3.3.1.1^{3,7}] dec-1-yl- *²⁰* | -- | |
| 93. | | methanone, (3,3-diethyl-1-pyrrolidinyl)tricyclo[3.3.1.1^{3,7}] dec-1-yl- *²⁰* | -- | |
| 94. | | methanone, (N,N-diisopropyl) tricyclo[3.3.1.1^{3,7}]dec-1-yl- *²⁰* | -- | |
| 95. | | methanone, (3,3-dimethylbutylaminyl) tricyclo[3.3.1.1^{3,7}]dec-1-yl- *²⁰* | -- | |
| 96. | | methanone, (2,2-dimethylpropylaminyl) tricyclo[3.3.1.1^{3,7}]dec-1-yl- *²⁰* | -- | |
| 97. | | methanone, (1,1-dimethyl-3,3-dimethylbutylaminyl) tricyclo[3.3.1.1^{3,7}]dec-1-yl- *²⁰* | -- | |
| 98. | | methanone, (1,3-dimethyl-butylaminyl) tricycle[3.3.1.1^{3,7}]dec-1-yl- *²⁰* | -- | |
| 99. | (t) | Bis-methoxy PEG-13 PEG-438/PPG-110 SMDI Copolymer *²¹* | 936645-35-1 | PolymerExp ert S.A. (Pessac, France) |
| 100. | (u) | propyl {4-[2-(diethylamino)-2-oxoethoxy]-3-methoxyphenyl}acetate *²²* | 61791-12-6 | Sigma Aldrich (US) |
| 101. | (v) | 3-((2-ethylhexyl)oxy)propane-1,2-diol *²³* | 70445-33-9 | - |
| 102. | | 3-((2-propylheptyl)oxy)propane-1,2-diol *²³* | - | - |
| 103. | | 1-amino-3-((2-ethylhexyl)oxy)propan-2-ol *²³* | 99509-00-9 | - |

| | | | | |
|---|---|---|---|---|
| *¹* available as GLUCAM^{™} P-20. *²* available as Glucam^{™} E-20. *³* available as Plantacare^{®} 810 UP. *^{3a}* available as Simulsol^{®} SL 11W. ⁴ available as CERAPHYL^{®} ICA. ³ available as Tegosoft^{®} APM. *⁶* available as Schercemol^{™} NGDO. *⁷* disclosed in U.S. Patent No. 6,737,396B2 (Firmenich), column 1, lines 43-47. *⁸* diclosed as compound 1'i in U.S. Patent No. 6,440,400B1 (Takasago Int. Corp.), col. 5. *^{8a}* diclosed in U.S. Patent No. 4,313,855 (Dragoco Gerberding & Co. GmbH), col. 1, lines 12-13. *⁹* disclosed in U.S. Patent No. 7,538,081B2 (Takasago Int. Corp.), column 7, lines 50-53. *¹⁰* disclosed in U.S. Patent No. 6,147,049 (Givaudan Roure), col. 5, line 24, to col. 6, line 17. *¹¹* disclosed in PCT Publication No. WO85/04803 (Diagnostic), pg. 2, line 1 to pg. 4, line 2. *¹²* disclosed in JP Patent No. 61-083114 (Kanebo). *¹³* disclosed in JP Patent No. 61-063612 (Kanebo). *¹⁴* disclosed in JP Patent No. 62-084010 (Shiseido). *^{14b}* available as: Laureth-6. *¹⁵* disclosed in U.S. Patent Publication No. 2011/0104089A1 (Symrise), para. [0001]. *¹⁶* available as PCL-Liquid^{®} 100. *¹⁷* disclosed in U.S. Patent No. 7,196,052 (Takasago Int. Corp.), col. 4, lines 34-35. *¹⁸* disclosed in EP Patent Publication No. 616800A2 (Givaudan), pg. 2, lines 12-25. *¹⁹* disclosed in U.S. Patent No. 4,110,626 (Shiseido), column 3, lines 54-56. *^{19a}* disclosed in PCT Publication No. WO2014/155019 (LVMH). *^{19b}* disclosed in U.S. Patent No. 9,050,261 (Symrise). *²⁰* disclosed as compounds C1-C22 in WO2014/139952 (Unilever). *²¹* available as Expert Gel^{®} EG56. *²²* available as Kolliphor^{®} EL. *²³* disclosed in U.S. Patent No. 9,050,261 (Symrise). | | | | |

Further examples of non-odorous fragrance modulator is selected from the group of materials disclosed in Table 4(b).

**Table 4(b): Substantially Non-Odorous Fragrance Modulators**

| **No.** | **Chemical or INCI Name** | **Trade Name** | **CAS Number** | **Supplier** |
|---|---|---|---|---|
| 1. | C12-14 Sec-Pareth-3 | Tergitol^{®} 15-S-7 | 68131-40-8 | Sigma Aldrich (UK) |
| 2. | Poly(ethylene glycol-ran-propylene glycol) monobutyl ether | PPG-7-Buteth-10 | 9038-95-3 | Sigma Aldrich (UK) |
| 3. | PPG-4-Ceteth-10 | Nikkol PBC-33 | 37311-01-6 | Chemical Navi |
| 4. | Deceth-4 | Ethal DA-4 | 5703-94-6 | Ethox Chemicals, Inc. |
| 5. | PPG-5-Ceteth-20 | AEC PPG-5-Ceteth-20 | 9087-53-0 | A & E Connock (Perfumery & Cosmetics) Ltd. |
| 6. | C14-15 Pareth-7 | Neodol 45-7 alcohol ethoxylate | 68951-67-7 | Shell Chemical Company |
| 7. | Linear alcohol (C12-15) Pareth-3ethoxylate, POE-7 | Bio-soft N25-7 | 68131-39-5 | Stephan Company (USA) |
| 8. | Linear alcohol (C12-13) Pareth-3ethoxylated, POE-6.5) | Bio-soft N23-6.5 | 66455-14-9 | |
| 9. | Polyethylene glycol 1100 mono(hexadecyl/octade cyl) ether | Cremophor^{®} A 25 | 68439-49-6 | Sigma Aldrich (UK) |
| 10. | Linear alcohol (C9-11) ethoxylated POE -8 Pareth-3 | Bio-soft N91-8 | 68439-46-3 | Stephan Company (USA) |
| 11. | Coceth-10 or Polyoxyethylene (10) dodecyl ether | Genapol^{®} C-100 | 61791-13-7 | Sigma Aldrich (UK) |
| 12. | Alcohols, C12-14, ethoxylated | Rhodasurf^{®} LA 30 | 68439-50-9 | Solvay Solutions Italia S.p.A. |
| 13. | Poly(ethylene glycol) methyl ether | Poly(ethylene glycol) methyl ether | 9004-74-4 | Sigma Aldrich (UK) |
| 14. | C10-16 Pareth-1 | Neodol^{®} PC 110 | 68002-97-1 | Shell Chemical Company |
| 15. | PPG-11 Stearyl Ether | Arlamol^{™} PS11E | 25231-21-4 | Croda (UK) |
| | | | | |
| 16. | Steareth-100 | Brij^{®} S100 | 9005-00-9 | Sigma Aldrich (UK) |
| 17. | Polyethylene glycol hexadecyl ether | Brij^{®} C-58 | 9004-95-9 | Sigma Aldrich (UK) |
| 18. | Pluronic^{®} F-127 | Pluronic^{®} F-127 | 9003-11-6 | Sigma Aldrich (UK) |
| 19. | Linear Alcohol (C11) Ethoxylate, POE-5 | Bio-soft N1-5 | 34398-01-1 | Stepan Canada Inc. |
| 20. | Laureth-10 | Intrasol FA 12/18/10 | 6540-99-4 | Evonik Industries AG |
| 21. | Decaethylene glycol mono-dodecyl ether | Polyoxyethylen e (10) lauryl ether | 9002-92-0 | Sigma Aldrich (UK) |
| 22. | Ethylene glycol monomethyl ether | 2-Methoxyethanol | 109-86-4 | Sigma Aldrich (UK) |
| 23. | Myreth-4 | Homulgator 920 G | 27306-79-2 | Grau Aromatics GmbH & Company KG |
| 24. | Oleth-16 Alkoxylated Alcohols | Pegnol O-16A | 25190-05-0 | Toho Chemical Industry Co., Ltd. |
| 25. | Isosteareth-5 | Emalex 1805 | 52292-17-8 | Nihon Emulsion Company, Ltd. |
| 26. | PPG-10 Cetyl Ether | Arlamol^{™} PC10 | 9035-85-2 | Croda (UK) |
| 27. | Polyoxy(ethylene glycol) (18) tridecyl ether | Poly(ethylene glycol) (18) tridecyl ether | 24938-91-8 | Sigma-Aldrich (UK) |
| 28. | Poly(oxy-1,2-ethanediyl), a-decyl-w-hydroxy- | ALFONIC^{®} 10-8 Ethoxylate | 26183-52-8 | Sasol Chemicals (USA) LLC |
| 29. | Laureth- 1 | Mackam^{™} 2LSF | 4536-30-5 | Rhodia (DE) |
| 30. | PEG-5 Hydrogenated Tallow Amine | Ethox HTAM-5 | 61791-26-2 | Ethox Chemicals, Inc. |
| 31. | PEG-15 Oleamine | Nikkol TAMNO-15 | 26635-93-8 | Nikko Chemicals Co., Ltd. |
| 32. | Polyoxyethylene (20) oleyl ether | Brij^{®} O20-SS | 9004-98-2 | Sigma Aldrich (UK) |
| 33. | Cetoleth-10 | Brij^{®} CO10 | 8065-81-4 | Croda, Inc. |
| 34. | Talloweth-7 | Emulmin 70 | 61791-28-4 | Sanyo Chemical Industries Ltd. |
| 35. | Isobutoxypropanol Alcohols | Isobutoxypropa nol | 34150-35-1 | MolPort |
| 36. | Isobutoxypropanol Alcohols | Isobutoxypropa nol | 23436-19-3 | AKos Consulting & Solutions |
| 37. | Diethylene Glycol | Twincide EDG | 111-46-6 | Roda |
| 38. | Methoxyethanol | Hisolve MC | 109-86-4 | Toho Chemical Industry Co., Ltd. |
| 39. | Ethoxyethanol Alcohols | 2-Ethoxyethanol | 110-80-5 | Sigma-Aldrich (UK) |
| 40. | Methoxyisopropanol Alcohols | Dowanol^{™} PM | 107-98-2 | The Dow Chemical Company |
| 41. | Methoxyethanol | Hisolve MC | 32718-54-0 | Toho Chemical Industry Co., Ltd. |
| 42. | Methylal Ethers | Dimethoxymeth ane | 109-87-5 | Sigma-Aldrich (UK) |
| 43. | 3-Methoxybutanol | Methoxybutano 1 | 2517-43-3 | Hans Schwarzkopf GmbH / Co. KG |
| 44. | Butoxyethanol | Butyl OXITOL | 111-76-2 | Shell Chemical Company |
| 45. | Propylene Glycol n-Butyl Ether | Dowanol^{™} PnB | 5131-66-8/29387-86-8 | The Dow Chemical Company |
| 46. | Propylene Glycol Butyl Ether | Propylene Glycol Butyl Ether | 15821-83-7 | Sigma Aldrich (UK) |
| 47. | 2-(2-butoxyethoxy)ethanol | Diethylene glycol butyl ether | 112-34-5 | Sigma Aldrich (UK) |
| 48. | Deceth-4 Phosphate | Crodafos^{™} D4A | 52019-36-0 | Croda, Inc. |
| 49. | 2-(Hexadecyloxy)ethanol | Ethylene glycol monohexadecyl ether | 2136-71-2 | Sigma-Aldrich (UK) |
| 50. | Poly(propylene glycol) monobutyl ether | Poly(propylene glycol) monobutyl ether | 9003-13-8 | Sigma-Aldrich (UK) |
| 51. | Propylene Glycol Propyl Ether | Dowanol^{™} PnP | 30136-13-1 | The Dow Chemical Company |
| 52. | Propylene Glycol n-Butyl Ether | Dowanol^{™} PnB | 29387-86-8/5131-66-8 | The Dow Chemical Company |
| 53. | Dipropylene glycol monomethyl ether | Di(propylene glycol) methyl ether, mixture of isomers | 34590-94-8 | Sigma Aldrich (UK) |
| 54. | Dipropylene Glycol Dimethyl Ether | Proglyde^{™} DMM | 111109-77-4 | The Dow Chemical Company |
| 55. | PPG-2 Methyl Ether | Dowanol^{™} DPM | 13429-07-7 | The Dow Chemical Company |
| 56. | Methoxydiglycol Ethers | OriStar DEGME | 111-77-3 | Orient Stars LLC |
| 57. | Diethylene glycol ethyl ether | Di(ethylene glycol) ethyl ether | 111-90-0 | Sigma Aldrich (UK) |
| 58. | Dimethoxydiglycol Ethers | Dimethyldiglyc ol | 111-96-6 | H&V Chemicals |
| 59. | PPG-3 Methyl Ether | Dowanol^{™} TPM | 37286-64-9 | The Dow Chemical Company |
| 60. | Methyl Morpholine Oxide Amine Oxides | 224286 ALDRICH 4-Methylmorpholi ne N-oxide | 7529-22-8 | Sigma-Aldrich (UK) |
| 61. | Oleth-3 | Brij^{®} O3 | 5274-66-8 | Croda Europe, Ltd. |
| 62. | Tri(propylene glycol) n-butyl ether | Dowanol^{™} TPnB | 55934-93-5 | Sigma-Aldrich (UK) |
| 63. | Tripropylene Glycol | Tripropylene Glycol | 24800-44-0 | Sigma-Aldrich (UK) |
| 64. | PPG-3 Methyl Ether Alkoxylated Alcohols | Dowanol^{™} TPM | 25498-49-1 | The Dow Chemical Company |
| 65. | Triethylene glycol | Triglycol | 112-27-6 | Sigma Aldrich (UK) |
| 66. | PEG-3 Methyl Ether | Hymol^{™} | 112-35-6 | Toho Chemical Industry Co., Ltd. |
| 67. | Laureth-3 | AEC Laureth-3 | 3055-94-5 | A & E Connock (Perfumery & Cosmetics) Ltd. |
| 68. | Ethylhexylglycerin | AG-G-75008 | 70445-33-9 | Angene Chemical |
| 69. | Tetra(ethylene glycol) | Tetraethylene glycol | 112-60-7 | Sigma Aldrich (UK) |
| 70. | Steareth-3 | Isoxal 5 | 4439-32-1 | Vevy Europe SpA |
| 71. | Ceteth-3 | Emalex 103 | 4484-59-7 | Nihon Emulsion Company, Ltd. |
| 72. | Myreth-3 | Isoxal 5 | 26826-30-2 | Vevy Europe SpA |
| 73. | Trideceth-3 | Alfonic^{®} TDA-3 Ethoxylate | -- | Sasol North America, Inc. |
| 74. | Ceteth-2 | Brij^{®} C2 | 5274-61-3 | Croda Europe, Ltd. |
| 75. | Oleth-2 | Brij^{®} O2 | 5274-65-7 | Croda, Inc. |
| 76. | Steareth-2 | Brij^{®} S2 | 16057-43-5 | Croda, Inc. |
| 77. | Cetoleth-10 | Brij^{®} CO10 | 8065-81-4 | Croda, Inc. |
| 78. | Trimethyl Pentanol Hydroxyethyl Ether Alcohols | Trimethyl Pentanol Hydroxyethyl Ether | 68959-25-1 | Angene Chemical |
| 79. | Steareth-10 Allyl Ether | Salcare^{®} SC80 | 109292-17-3 | BASF |
| 80. | TEA-Lauryl Ether | material ID-AG-J-99109 | 1733-93-3 | Angene Chemical |
| 81. | Polyglyceryl-2 Oleyl Ether | Chimexane NB | 71032-90-1 | Chimex |
| 82. | Batyl Alcohol | B402 ALDRICH | 544-62-7 | Sigma-Aldrich (UK) |
| 83. | Octaethylene Glycol | 15879 ALDRICH | 5117-19-1 | Sigma-Aldrich (UK) |
| 84. | Triglycerol diisostearate | Cithrol^{™} | 66082-42-6 | Croda (UK) |
| 85. | Diglycerin | Diglycerin 801 | 59113-36-9 | Sakamoto Yakuhin Kogyo Co., Ltd. |
| 86. | Polyglycerin #310 | Polyglycerin #310 | 25618-55-7 | Sakamoto Yakuhin Kogyo Co., Ltd. |
| 87. | Distearyl Ether | Cosmacol^{®} SE | 6297-03-6 | Sasol Germany GmbH |
| 88. | Caprylyl Glyceryl Ether | Caprylyl Glyceryl Ether | 10438-94-5 | AKos Consulting & Solutions |
| 89. | Chimyl Alcohol | Chimyl Alcohol | 506-03-6 | Nikko Chemicals Co., Ltd. |
| 90. | Dipentaerythrityl Hexacaprylate/Hexacap rate | Liponate^{®} DPC-6 | 68130-24-5 | Lipo Chemicals, Inc. |
| 91. | Morpholine | 394467 ALDRICH | 110-91-8 | Sigma-Aldrich (UK) |
| 92. | Dimethyl Oxazolidine | OXABAN^{™} -A | 51200-87-4 | The Dow Chemical Company |
| 93. | Ethyl Hydroxymethyl Oleyl Oxazoline | 4-Oxazolemethan ol | 68140-98-7 | Angene Chemical |
| 94. | Methyl Hydroxymethyl Oleyl Oxazoline | Adeka Nol GE-RF | 14408-42-5 | Adeka Corporation |
| 95. | Pramoxine HCl | OriStar PMHCL | 637-58-1 | Orient Stars LLC |
| 96. | Allantoin Ascorbate | Allantoin Ascorbate | 57448-83-6 | ABI Chem |
| 97. | Stearamidopropyl Morpholine Lactate | Mackalene^{™} 326 | 55852-14-7 | Rhodia Inc. |
| 98. | Dioxolane | Elcotal DX | 646-06-0 | Lambiotte & CIE S.A. |
| 99. | Glycerol Formal | Glycerol Formal | 5464-28-8 | Sigma Aldrich (UK) |
| 100. | Stearamidopropyl Morpholine | Mackine 321 | 55852-13-6 | Rhodia Inc. |
| 101. | 2,4,6-Tris[bis(methoxymethy 1)amino]-1,3,5-triazine | Poly(melamine-co- | 68002-20-0 | Sigma-Aldrich (UK) |
| | | formaldehyde) methylated | | |
| 102. | Poloxamine 1307 | Pluracare^{®} 1307 | 11111-34-5 | BASF |
| 103. | Nonoxynol-8 | Igepal^{®} CO-610 | 27177-05-5 | Rhodia Inc. |
| 104. | Nonoxynol-10 | Igepal^{®} CO-710 | 27177-08-8 | Rhodia Inc. |
| 105. | Octoxynol-10 | Nikkol OP-10 | 2315-66-4 | Nikko Chemicals Co., Ltd. |
| 106. | Nonoxynol-9 | Igepal^{®} CO-630 | 68987-90-6 | Rhodia Inc. |
| 107. | Nonoxynol-9 Iodine | Nonoxynol-9 iodine | 94349-40-3 | Angene Chemical |
| 108. | Octylphenoxy poly(ethyleneoxy)ethan ol, branched | Igepal^{®} CA-630 | 68987-90-6 | Rhodia Inc. |
| 109. | Sodium Octoxynol-2 Ethane Sulfonate | Triton^{™} X-200 | 55837-16-6 | The Dow Chemical Company |
| 110. | Benzylhemiformal | Preventol D2 | 14548-60-8 | Lanxess Corporation |
| 111. | Nonoxynol-2 | Igepal^{®} CO-210 | 27176-93-8 | Rhodia Inc. |
| 112. | Octoxynol-3 | Igepal^{®} CA-420 | 2315-62-0 | The Dow Chemical Company |
| 113. | Nonoxynol-3 | Marlophen NP 3 | 27176-95-0 | Sasol Germany GmbH |
| 114. | Alkoxylated Alcohols | Alkasurf NP-4 | 7311-27-5 | Rhodia Inc. |
| 115. | Nonoxynol-3 | Triethylene Glycol Mono(p-nonylphenyl) Ether | 51437-95-7 | Santa Cruz Biotechnology |
| 116. | Nonoxynol-7 | Lowenol 2689 | 27177-03-3 | Jos. H. Lowenstein & Sons, Inc. |
| 117. | Nonoxynol-6 | Igepal^{®} CO-530 | 27177-01-1 | Rhodia Inc. |
| 118. | Nonoxynol-5 | Igepal^{®} CO-520 | 20636-48-0 | Rhodia Inc. |
| 119. | Nonoxynol-5 | Igepal^{®} CO-520 | 26264-02-8 | Rhodia Inc. |
| 120. | Nonoxynol-4 | Alkasurf NP-4 | 27176-97-2 | Rhodia Inc. |
| 121. | Polyglyceryl-10 Trioleate | Nikkol Decaglyn 3-OV | 102051-00-3 | Nikko Chemicals Co., Ltd. |
| 122. | Polyglyceryl-10 Dioleate | Nikkol Decaglyn 2-O | 33940-99-7 | Nikko Chemicals Co., Ltd. |
| | | | | |
| 123. | Polyglyceryl-10 Tetraoleate | Caprol 10G40 | 34424-98-1 | Abitec Corporation |
| 124. | Polyglyceryl-10 Stearate | Nikkol Decaglyn 1-SV EX | 79777-30-3 | Nikko Chemicals Co., Ltd. |
| 125. | Polyglyceryl-10 Oleate | S-Face O-1001 P | 79665-93-3 | Sakamoto Yakuhin Kogyo Co., Ltd. |
| 126. | Polyglyceryl-10 Myristate | Nikkol Decaglyn 1-MV EX | 87390-32-7 | Nikko Chemicals Co., Ltd. |
| 127. | Dermofeel^{®} G 10 L | Dermofeel^{®} G 10L | 34406-66-1 | Dr. Straetmans |
| 128. | Polyglyceryl-6 Laurate | NIKKOL Hexaglyn 1-L | 51033-38-6 | Chemical Navi |
| 129. | Polyglyceryl-6 Isostearate | S-Face IS-601 P | 126928-07-2 | Sakamoto Yakuhin Kogyo Co., Ltd. |
| 130. | Choleth-10 | Emalex CS-10 | 27321-96-6 | Nihon Emulsion Company, Ltd. |
| 131. | Steareth-10 Allyl Ether/Acrylates Copolymer | Salcare^{®} SC80 | 109292-17-3 | BASF |
| 132. | Polyvinyl Stearyl Ether | Giovarez^{®}1800 | 9003-96-7 | Phoenix Chemical, Inc. |
| 133. | Dicetyl Ether | Cosmacol Ether 16 | -- | Sasol Germany GmbH |
| | | | | |
| 134. | PPG-23-Steareth-34 | Unisafe 34S-23 | 9038-43-1 | Pola Chemical Industries, Inc. |
| 135. | Stearoxypropyl Dimethylamine | Farmin DM E-80 | 17517-01-0 | Kao Corp. |
| 136. | Distearyl Ether | Cosmacol SE | 6297-03-6 | Sasol Germany GmbH |
| 137. | Polyquaternium-10 | AEC Polyquaternium -10 | 55353-19-0 | A & E Connock (Perfumery & Cosmetics) Ltd. |
| 138. | Octyl ether | Dioctyl ether | 629-82-3 | Sigma Adlrich (UK) |
| 139. | Ethyl Ether | Diethyl Ether | 60-29-7 | EMD Chemicals |
| 140. | Methyl Hexyl Ether Ethers | methyl hexyl ether | 4747-07-3 | TCI AMERICA |
| 141. | Ceteth-12 | Emalex 112 | 94159-75-8 | Nihon Emulsion Company, Ltd. |
| 142. | Ceteth-10 or cetyl alcohol POE-10 | Jeecol CA-10 | 14529-40-9 | Jeen International |
| 143. | Steareth-10 | Jeecol SA-10 | 13149-86-5 | Jeen International |
| 144. | Nonaethylene glycol monododecyl ether | Nonaethylene glycol | 3055-99-0 | Sigma Aldrich (UK) |
| | | monododecyl ether | | |
| 145. | Oleth-10 | Brij^{®} O10 | 71976-00-6 | Croda, Inc. |
| 146. | Oleth-10 | Brij^{®} O10 | 24871-34-9 | Croda, Inc. |
| 147. | PEG-12 | Carbowax^{™} PEG 600 | 6790-09-6 | The Dow Chemical Company |
| 148. | PEG-9 | Sabopeg 400 | 3386-18-3 | Sabo s.p.a. |
| 149. | PEG-10 | DECAETHYL ENE GLYCOL | 5579-66-8 | MolPort |
| 150. | PEG-6 | Carbowax^{™} PEG 300 | 2615-15-8 | The Dow Chemical Company |
| 151. | Glycerol propoxylate | Glycerol propoxylate | 25791-96-2 | Sigma Aldrich (UK) |
| 152. | Glycerol ethoxylate | Glycerol ethoxylate | 31694-55-0 | Sigma Aldrich (UK) |
| 153. | Laureth-8 | AEC Laureth-8 | 3055-98-9 | A & E Connock (Perfumery & Cosmetics) Ltd. |
| 154. | Oleth-8 | Emalex 508 | 27040-03-5 | Nihon Emulsion Company, Ltd. |
| 155. | Laureth-7 | Alfonic 1216CO-7 Ethoxylate | 3055-97-8 | Sasol North America, Inc. |
| 156. | Steareth-7 | Polyoxyethylen e (7) stearyl ether | 66146-84-7 | Sigma Aldrich |
| 157. | Deceth-6 | Alfonic 1012-6.0 Ethoxylate | 5168-89-8 | Sasol North America, Inc. |
| 158. | Steareth-6 | Emalex 606 | 2420-29-3 | Nihon Emulsion Company, Ltd. |
| 159. | Hexaethylene glycol monododecyl ether | Hexaethylene glycol monododecyl ether | 3055-96-7 | Sigma-Aldrich (UK) |
| 160. | Hexaethylene glycol monohexadecyl ether | Hexaethylene glycol monohexadecyl ether | 5168-91-2 | Sigma-Aldrich (UK) |
| 161. | Beheneth-5 | Nikkol BB-5 | 136207-49-3 | Nikko Chemicals Co., Ltd. |
| 162. | Myreth-5 | Isoxal 12 | 92669-01-7 | Vevy Europe SpA |
| 163. | Steareth-5 | Jeecol SA-5 | 71093-13-5 | Jeen International Corporation |
| 164. | Ceteth-5 | Emalex 105 | 4478-97-1 | Nihon Emulsion Company, Ltd. |
| | Oleth-5 | Brij^{®} O5 | 5353-27-5 | Croda, Inc. |
| 165. | | | | |
| 166. | Laureth-5 | Safol^{®} 23E5 Ethoxylate | 3055-95-6 | Sasol North America, Inc. |
| 167. | Steareth-4 | Jeecol SA-4 | 59970-10-4 | Jeen International Corporation |
| 168. | Laureth-4 | Brij^{®} L4 | 5274-68-0 | Croda, Inc. |
| 169. | Myreth-4 | Homulgator 920 G | 39034-24-7 | Grau Aromatics GmbH & Company KG |
| 170. | Ceteth-4 | Procol CA-4 | 5274-63-5 | Protameen Chemicals |
| 171. | Oleth-4 | Chemal OA-4 | 5353-26-4 | Chemax, Inc. |
| 172. | Oleth-4 | Chemal OA-4 | 103622-85-1 | Chemax, Inc. |
| 173. | Polyimide-1 | Aquaflex^{™} XL-30 | 497926-97-3 | Chemwill |
| 174. | Polymethoxy Bicyclic Oxazolidine | Caswell No. 494CA | 56709-13-8 | Angene Chemical |
| 175. | Hydroxymethyl Dioxoazabicyclooctane | Zoldine^{™} ZT | 6542-37-6 | Angus Chemical Company |
| 176. | Dihydro-7a-ethyloxazolo[3,4-c]oxazole | 5-Ethyl-1-aza-3,7-dioxabicyclo[3. 3.0]octane | 7747-35-5 | Sigma Aldrich (UK) |
| 177. | Dibenzylidene Sorbitol | Disorbene^{®} | 32647-67-9 | Roquette America, Inc. |
| 178. | Dimethyldibenzylidene Sorbitol | Millad^{®} 3988 | 135861-56-2 | Milliken Chemicals |
| 179. | Laureth-2 | Alfonic 1216CO-2 Ethoxylate | 3055-93-4 | Sasol North America, Inc. |
| 180. | 2-(2-Butoxyethoxy)ethyl (6-propylpiperonyl) ether | Piperonyl Butoxide | 51-03-6 | Sigma-Aldrich (UK) |
| 181. | Menthone Glycerin Acetal | Frescolat^{®} MGA | 63187-91-7 | Symrise |
| 182. | Propylene Glycol Caprylate | Mackaderm PGC | 68332-79-6 | Rhodia Inc. |
| 183. | Diethoxynonadiene | SBB016951 | 67674-36-6 | Ambinter |
| 184. | Menthoxypropanediol Alcohols | Coolact^{®} 10 | 87061-04-9 | Takasago International Corporation |
| 185. | 2-Diphenylmethoxy-N,N-dimethylethylamine hydrochloride | Diphenhydrami ne HCl | 147-24-0 | Sigma-Aldrich (UK) |
| 186. | 3-((2-ethylhexyl)oxy)propan e-1,2-diol | -- | 70445-33-9 | -- |
| 187. | 3-((2-propylheptyl)oxy)propa ne-1,2-diol | -- | -- | -- |
| 188. | 1-amino-3-((2-ethylhexyl)oxy)propan-2-ol | -- | 99509-00-9 | -- |
| 189. | 1-(1-Methyl-2-propoxyethoxy)-2-propanol | Di(propylene glycol) propyl ether | 29911-27-1 | Sigma Aldrich (UK) |

The compounds as described above in Tables 4(a) and 4(b) that are methyl glucoside polyol, ethyl glucoside polyol, propyl glucoside polyol, or mixtures thereof. act as a substantially non-odorous fragrance modulator of the perceived fidelity and/or longevity of the fragrance profile of the composition of the present invention. For example, the substantially non-odorous fragrance modulators, with a fragrance component having a top-heavy fragrance construction, act to prolong the duration during which the fragrance profile, preferably the characters attributable from the high and moderate volatile fragrance materials, can be perceived as compared to a control composition in the absence of the modulators or having the traditional fragrance pyramid three-tiered structure. As another example, the substantially non-odorous fragrance modulators, with a fragrance component having a top-heavy fragrance construction, can improve the fidelity of the fragrance profile, preferably the characters attributable from the high volatile fragrance materials, such that it remains significantly the same from initial impression to the end as compared to a control composition in the absence of the modulators or having the traditional fragrance pyramid three-tiered structure. While not wishing to be bound by theory, it is believed that the substantially non-odorous fragrance modulators associate to the fragrance materials and retard evaporation. Additionally, without wishing to be bound by theory, it is believed that the substantially non-odorous fragrance modulators associate to the high and moderate-volatility fragrance materials to allow for high wt% while mitigating or eliminating a perceived harshness of the composition by the user.

### Volatile Solvents

The composition according to the present invention, can include a volatile solvent present in the amount of from about 20 wt% to about 99 wt% relative to the total weight of the composition, about 30 wt% to about 80 wt%, about 55 wt% to about 75 wt%, or less than, equal to, or greater than about 20 wt%, 25, 30, 35, 40, 45, 50, 55, 60, 65, 70, 75, 80, 85, 90, 95, or about 99 wt%, and wherein the solvent is a branch or unbranched C₁ to C₁₀ alkyl, akenyl or alkynyl group having at least one alcohol moiety, preferably ethanol, or isopropanol, or other alcohols (e.g., methanol, propanol, isopropanol, butanol, and mixtures thereof) commonly found in commercial fine fragrance products.

Accordingly, ethanol may be present in any of the compositions of the present invention, and more specifically, it will form from about 5 wt% to about 95 wt%, or even from about 10 wt% to about 80 wt%, 25 wt% to about 75 wt% of the composition, or combinations thereof, relative to the total weight of the composition. Alternatively, ethanol may be present in an amount of from about 10 wt% or 25 wt% to about 75 wt% or 80 wt%, relative to the total weight of the composition. The ethanol useful in the present invention may be any acceptable quality of ethanol, compatible and safe for the specific intended use of the composition such as, for example, topical applications of fine fragrance or cosmetic compositions.

### Water

In some examples (e.g., those including a volatile solvent), water may be present in any of the compositions of the present invention, and more specifically, it may not exceed about 95 wt% relative to the total weight of the composition, about 90 wt% or less, about 85 wt% or less, about 80 wt% or less, about 75 wt% or less, about 70 wt% or less, about 65 wt% or less, about 60 wt% or less, about 55 wt% or less, about 50 wt% or less, about 45 wt% or less, about 40 wt% or less, about 35 wt% or less, about 30 wt% or less, about 20 wt% or less, about 10 wt%, or less than, equal to, or greater than about 95 wt%, 90, 85, 80, 75, 70, 65, 60, 55, 50 ,45, 40, 30, 35, 30, 25, 20, 15, 10, or 5 wt%. Alternatively, water may be present in an amount of from about 5 wt% or about 95 wt% When the composition is a cosmetic composition the level of water should not be so high that the product becomes cloudy thus negatively impacting the product aesthetics. It is understood that the amount of water present in the composition may be from the water present in the volatile solvent (e.g., ethanol) used in the composition, as the case may be.

### Non-Volatile Solvents

The composition may comprise a non-volatile solvent or a mixture of non-volatile solvents. Non-limiting examples of non-volatile solvents include benzyl benzoate, diethyl phthalate, isopropyl myristate, propylene glycol, dipropylene glycol, triethyl citrate, and mixtures thereof. These solvents often are introduced to the product via the perfume oil as many perfume raw materials may be purchased as a dilution in one of these solvents. Where non-volatile solvents are present, introduced either with the perfume materials or separately, then for the purposes of calculating the proportion of fragrance component having a vapor pressure of less than 0.001 Torr (0.000133 kPa) at 25 °C the total fragrance components does not include non-volatile solvents. Where non-volatile solvents are present, introduced either with the perfume materials or separately, then for the purposes of calculating the total level of fragrance component this does not include non-volatile solvents. In addition, if present with cyclic oligosacchrides, the non-volatile solvent may be included at a weight ratio of the non-volatile solvent to the cyclic oligosaccharide of less than 1:1, less than 1:2, less than 1:10, or less than 1:100.

### Entrapment Materials

In other examples, compositions of the present invention can include an entrapment material at a level such that the weight ratio of the entrapment material to the fragrance materials is in the range of from about 1:20 to about 20:1. in some examples, the composition may comprise an entrapment material present in the amount of from about 0.001 wt% to about 40 wt%, from about 0.1 wt% to about 25 wt%, from about 0.3 wt% to about 20 wt%, from about 0.5 wt% to about 10 wt%, or from about 0.75 wt% to about 5 wt%, relative to the total weight of the composition. The compositions disclosed herein may include from 0.001 wt% to 40%, from 0.1 wt% to 25 wt%, from 0.3 wt% to 20 wt%, from 0.5 wt% to 10 wt% or from 0.75 wt% to 5 wt%, relative to the total weight of the composition, of a cyclic oligosaccharide.

Suitable entrapment materials for use herein are selected from polymers; capsules, microcapsules and nanocapsules; liposomes, absorbents; cyclic oligosaccharides and mixtures thereof. Preferred are absorbents and cyclic oligosaccharides and mixtures thereof. Highly preferred are cyclic oligosaccharides (*see* PCT Publication Nos. WO2000/67721 (Procter & Gamble); and WO2000/67720 (Procter & Gamble); and U.S. Patent No. 6,893,647 (Procter & Gamble)).

As used herein, the term "cyclic oligosaccharide" means a cyclic structure comprising six or more saccharide units. Preferred for use herein are cyclic oligosaccharides having six, seven or eight saccharide units and mixtures thereof, more preferably six or seven saccharide units and even more preferably seven saccharide units. It is common in the art to abbreviate six, seven and eight membered cyclic oligosaccharides to α, β and γ respectively.

The cyclic oligosaccharide of the compositions used for the present invention may comprise any suitable saccharide or mixtures of saccharides. Examples of suitable saccharides include, but are not limited to, glucose, fructose, mannose, galactose, maltose and mixtures thereof. However, preferred for use herein are cyclic oligosaccharides of glucose. The preferred cyclic oligosaccharides for use herein are α-cyclodextrins or β-cyclodextrins, or mixtures thereof, and the most preferred cyclic oligosaccharides for use herein are β-cyclodextrins.

The cyclic oligosaccharide, or mixture of cyclic oligosaccharides, for use herein may be substituted by any suitable substituent or mixture of substituents. Herein the use of the term "mixture of substituents" means that two or more different suitable substituents can be substituted onto one cyclic oligosaccharide. The derivatives of cyclodextrins consist mainly of molecules wherein some of the OH groups have been substituted. Suitable substituents include, but are not limited to, alkyl groups; hydroxyalkyl groups; dihydroxyalkyl groups; (hydroxyalkyl)alkylenyl bridging groups such as cyclodextrin glycerol ethers; aryl groups; maltosyl groups; allyl groups; benzyl groups; alkanoyl groups; cationic cyclodextrins such as those containing 2-hydroxy-3-(dimethylamino) propyl ether; quaternary ammonium groups; anionic cyclodextrins such as carboxyalkyl groups, sulphobutylether groups, sulphate groups, and succinylates; amphoteric cyclodextrins; and mixtures thereof.

The substituents may be saturated or unsaturated, straight or branched chain. Preferred substituents include saturated and straight chain alkyl groups, hydroxyalkyl groups and mixtures thereof. Preferred alkyl and hydroxyalkyl substituents are selected from C₁-C₈ alkyl or hydroxyalkyl groups or mixtures thereof, more preferred alkyl and hydroxyalkyl substituents are selected from C₁-C₆ alkyl or hydroxyalkyl groups or mixtures thereof, even more preferred alkyl and hydroxyalkyl substituents are selected from C₁-C₄ alkyl or hydroxyalkyl groups and mixtures thereof. Especially preferred alkyl and hydroxyalkyl substituents are propyl, ethyl and methyl, more especially hydroxypropyl and methyl and even more preferably methyl.

Suitable cyclic oligosaccharides for use in the present invention are unsubstituted, or are substituted by only saturated straight chain alkyl, or hydroxyalkyl substituents. Therefore, preferred examples of cyclic oligosaccharides for use herein are α-cyclodextrin, β-cyclodextrin, methyl-α-cyclodextrin, methyl-β-cyclodextrin, hydroxypropyl-α-cyclodextrin and hydroxypropyl-β-cyclodextrin. Most preferred examples of cyclic oligosaccharides for use herein are methyl-α-cyclodextrin and methyl-β-cyclodextrin. These are available from Wacker-Chemie GmbH Hanns-Seidel-Platz 4, Munchen, DE under the tradename Alpha W6 M and Beta W7 M respectively.

The cyclic oligosaccharides of the compositions used for the present invention can be soluble in water, ethanol, or both water and ethanol. As used herein "soluble" means at least about 0.1 g of solute dissolves in 100 mL of solvent, at 25 °C and 1 standard atmospheric pressure (760 mmHg). The cyclic oligosaccharides for use herein have a solubility of at least about 1 g/100 mL, at 25 °C and 1 atm of pressure. In some examples, cyclic oligosaccharides are only present at levels up to their solubility limits in a given composition at room temperature. A person skilled in the art will recognize that the levels of cyclic oligosaccharides used in the present invention will also be dependent on the components of the composition and their levels, for example the solvents used or the exact fragrance oils, or combination of fragrance oils, present in the composition. Therefore, although the limits stated for the entrapment material are preferred, they are not exhaustive.

### Propellants

The compositions described herein may include a propellant. Some examples of propellants include compressed air, nitrogen, inert gases, carbon dioxide, and mixtures thereof. Propellants may also include gaseous hydrocarbons like propane, n-butane, isobutene, cyclopropane, and mixtures thereof. Halogenated hydrocarbons like 1,1-difluoroethane may also be used as propellants. Some non-limiting examples of propellants include 1,1,1,2,2-pentafluoroethane, 1,1,1,2-tetrafluoroethane, 1,1,1,2,3,3,3-heptafluoropropane, trans-1,3,3,3-tetrafluoroprop-1-ene, dimethyl ether, dichlorodifluoromethane (propellant 12), 1,1-dichloro-1,1,2,2-tetrafluoroethane (propellant 114), 1-chloro-1,1-difluoro-2,2-trifluoroethane (propellant 115), 1-chloro-1,1-difluoroethylene (propellant 142B), 1,1-difluoroethane (propellant 152A), monochlorodifluoromethane, and mixtures thereof. Some other propellants suitable for use include, but are not limited to, A-46 (a mixture of isobutane, butane and propane), A-31 (isobutane), A-17 (n-butane), A-108 (propane), AP70 (a mixture of propane, isobutane and n-butane), AP40 (a mixture of propane, isobutene and n-butane), AP30 (a mixture of propane, isobutane and n-butane), and 152A (1,1 diflouroethane). The propellant may have a concentration from about 15%, 25%, 30%, 32%, 34%, 35%, 36%, 38%, 40%, or 42% to about 70%, 65%, 60%, 54%, 52%, 50%, 48%, 46%, 44%, or 42% by weight of the total fill of materials stored within the container.

### Antiperspirant Active

The compositions described herein may be free of, substantially free of, or may include an antiperspirant active (e.g., any substance, mixture, or other material having antiperspirant activity). Examples of antiperspirant actives include astringent metallic salts, like the inorganic and organic salts of aluminum, zirconium and zinc, as well as mixtures thereof. Such antiperspirant actives include, for example, the aluminum and zirconium salts, such as aluminum halides, aluminum hydroxyhalides, zirconyl oxyhalides, zirconyl hydroxyhalides, and mixtures thereof.

### Other Ingredients

In yet another aspect, the composition consists essentially of the recited ingredients but may contain small amounts (not more than about 10 wt%, preferably no more than 5 wt%, or preferably no more than 2 wt% thereof, relative to the total weight of the composition) of other ingredients that do not impact on the fragrance profile, particularly the evaporation rate and release of the fragrance materials. For example, a fine fragrance composition may comprise stabilizing or anti-oxidant agents, UV filters or quenchers, or colouring agents, commonly used in perfumery. There are a number of other examples of additional ingredients that are suitable for inclusion in the present compositions, particularly in compositions for cosmetic use. These include, but are not limited to, alcohol denaturants such as denatonium benzoate; UV stabilizers such as benzophenone-2; antioxidants such as tocopheryl acetate; preservatives such as phenoxyethanol, benzyl alcohol, methyl paraben, and propyl paraben; dyes; pH adjusting agents such as lactic acid, citric acid, sodium citrate, succinic acid, phosphoric acid, sodium hydroxide, and sodium carbonate; deodorants and anti-microbials such as farnesol and zinc phenolsulphonate; humectants such as glycerine; oils; skin conditioning agents such as allantoin; cooling agents such as trimethyl isopropyl butanamide and menthol; silicones; solvents such as hexylene glycol; hair-hold polymers such as those described in PCT Publication No. WO94/08557 (Procter & Gamble); salts in general, such as potassium acetate and sodium chloride and mixtures thereof.

In yet another aspect, the composition of the present invention, depending on its intended use, is a mixture of fragrance materials possibly together with other ingredients such as, for example, perfume carriers. By the term "perfume carrier", it is meant to include materials which are practically neutral from a perfumery point of view, e.g., which does not significantly alter the organoleptic properties of perfuming components. The perfume carrier may be a compatible liquid or solid fillers, diluents, and the like. The term "compatible", as used herein, means that the components of the compositions of this invention are capable of being combined with the primary actives of the present invention, and with each other, in a manner such that there is no interaction which would substantially reduce the efficacy of the composition under ordinary use situations. The type of carrier utilized in the present invention depends on the type of product desired and may comprise, but are not limited to, solutions, aerosols, emulsions (including oil-in-water or water-in-oil), gels, and liposomes. Preferably, the carrier is a liquid and will be a solvent such as, for example, dipropyleneglycol, diethyl phthalate, isopropyl myristate, benzyl benzoate, 2-(2-ethoxyethoxy)-1-ethanol, or ethyl citrate (triethyl citrate).

In yet another aspect, the compositions for use in the present invention may take any form suitable for use, such as for perfumery or cosmetic use. These include, but are not limited to, vapor sprays, aerosols, emulsions, lotions, liquids, creams, gels, sticks, ointments, pastes, mousses, powders, granular products, substrates, cosmetics (e.g., semi-solid or liquid makeup, including foundations) and the like. In some examples, the compositions for use in the present invention take the form of a vapor spray. Compositions of the present invention can be further added as an ingredient to other compositions, preferably fine fragrance or cosmetic compositions, in which they are compatible. As such they can be used within solid composition or applied substrates etc. Examples of products including the composition can include a fabric care product, an air care product, a home care
product, a beauty care product, or a mixture thereof. Specific examples of products can include a perfume, an eau de toilette, an eau de parfum, a cologne, a body splash, a lotion, a cream, a shampoo, a conditioner, a hair mist, a body oil, a deodorant, a solid fragrance, or a body spray. The composition can be contacted with skin, hair, or a fabric.

### Article of Manufacture

The composition may be included in an article of manufacture comprising a spray dispenser. The spray dispenser may comprise a vessel for containing the composition to be dispensed. The spray dispenser may comprise an aerosolized composition (e.g., a composition comprising a propellant) within the vessel as well. Other non-limiting examples of spray dispensers include non-aerosol dispensers (e.g., vapor sprays), manually activated dispensers, pump-spray dispensers, or any other suitable spray dispenser available in the art.

### Methods of Using the Compositions

The composition of the present invention according to any embodiments described herein is a useful perfuming composition, which can be advantageously used as consumer products intended to perfume any suitable substrate. As used herein, the term "substrate" means any surface to which the composition of the present invention may be applied to without causing any undue adverse effect. For example, this can include a wide range of surfaces including human or animal skin or hair, paper (fragranced paper), air in a room (air freshener or aromatherapy composition), fabric, furnishings, dishes, hard surfaces and related materials. Preferred substrates include body surfaces such as, for example, hair and skin, most preferably skin.

The composition of the present invention may be used in a conventional manner for fragrancing a substrate. An effective amount of the composition, such as from about 1 µL to about 100 mL, preferably from about 10 µL to about 1,000 µL, more preferably from about 25 µL to about 500 µL, from about 50 µL to about 100 µL, from about 100 µL to about 20 mL, or combinations thereof, is applied to the suitable substrate. Alternatively, an effective amount of the composition of the present invention is less than, equal to, or greater than about 1 µL, 10 µL, 25 µL or 50 µL to about 100 µL, 500 µL, 1,000 µL, 10,000 µL, 10 mL, 20 mL, 25 mL, 30 mL, 40 mL, 50 mL, 60 mL, 70 mL, 80 mL, 90 mL, or 100 mL. The composition may be applied by hand or applied utilizing a delivery apparatus such as, for example, vaporizer or atomizer. Preferably, the composition is allowed to dry after its application to the substrate. The scope of the present invention should be considered to cover one or more distinct applications of the composition or the continuous release of a composition *via a* vaporizer or other type of atomizer.

The present disclosure provides a method for imparting, intensifying, or modifying an odor on human skin or human hair, comprising applying to human skin and/or human hair the composition of the present invention. Examples of notes or characters that can be enhanced include any of those of: citrus-type note, green-type note, watery-type notes, aromatic-type notes, herbal-type notes, mint-type notes, lavender-type notes, rosemary-type notes, spicy-type notes, cinnamon-type notes, clove-type notes, pepper-type notes, cumin-type notes, ginger-type notes, fougere-type note, patchouli-type notes, floral-type notes, gourmand-type notes, sweet-type notes, vanilla-type notes, amber-type notes, woody-type notes, cedarwood-type notes, sandalwood type notes, vetyver-type notes and mixtures thereof.

Preferably, the fragrance profile or character of the composition of the present invention is detectable by a a panel of experts or professional evaluators or individual experts or professional evaluators at later time points such as, for example, 15 mins, 30 mins, 1 hour, 2 hours, 3 hours, 4 hours, 5 hours, 6 hours, 7 hours, 8 hours, 10 hours, and possibly all the way up to 24 hours after application of the composition to a substrate as compared to controls (e.g., those without modulators).

In another aspect, the present invention is also directed to a method of producing a consumer product comprising bringing into contact or mixing into the product an organoleptically active quantity of a composition of the present invention.

### TEST METHODS

The following assays set forth must be used in order that the invention described and claimed herein may be more fully understood.

### Test Method 1: Determining Vapor Pressure

In order to determine the vapor pressure for the fragrance materials, go to the website *https:*//*scifinder.cas.org*/*scifinder*/*view*/*scifinder*/*scifinderExplore.jsf* and follow these steps to acquire the vapor pressure.

Input the CAS registry number for the particular fragrance material.

### Select the vapor pressure from the search results.

Record the vapor pressure (given in Torr at 25 °C).

SciFinder uses Advanced Chemistry Development (ACD/Labs) Software Version 11.02. (^{©} 1994-2018). If the CAS number for the particular fragrance material is unknown or does not exist, you can utilize the ACD/Labs reference program to directly determine the vapor pressure. Vapor Pressure is expressed in 1 Torr, which is equal to 0.133 kilopascal (kPa).

### Test Method 2a: Olfactory Tests a

In order to show the effect of the substantially non-odorous fragrance modulators and fragrance component of the present invention on the perception of fragrance profile in a composition of the present invention, test compositions are made, as described in the Example section, and given to expert panelists to evaluate.

At the testing facility, 50 µL samples of the compositions and the controls are applied to glass slides and placed on a hot plate at 32 °C to represent skin temperature for varying durations. It is important that glass slides of samples that are to be later compared are prepared at the same time. The panelists are asked to evaluate the perceived fragrance profile (intensity and/or character) of each glass slide sample at a given time point. Slides are presented coded so that their identity is not known by the panelists. Within a given time point panelists evaluate the slides in a random order and are able to revisit their assessment as they work through the slides at that time point. Their assessments are recorded. In the subsequent analysis, the data for strength and character comparisons are drawn from the independent assessments carried out at a given time point. Only when using the character difference scale below are any 2 products physically directly compared to each other. Panelists are selected from individuals who are either trained to evaluate fragrances according to the scales below or who have experience of fragrance evaluation in the industry. Typically, around 4-6 expert panelists are used to evaluate a given product and its control.

### (a) Fragrance Intensity:

The panelists are asked to give a score on a scale of 0 to 5 for perceived fragrance intensity according to the odour intensity scale set out in Table 5 herein below.

**Table 5 - Odour Intensity Scale**

| **Score** | **Fragrance Intensity** |
|---|---|
| 0 | None |
| 1 | Very Weak |
| 2 | Weak |
| 3 | Moderate |
| 4 | Strong |
| 5 | Very Strong |

### (b) Fragrance Character:

The panelists are asked to assess the fragrance character in one of 2 ways:
i)
   (i) a score on a scale of 0 to 3 for the dominance of particular characters that are relevant to that particular fragrance, e.g.: harsh, green, watery, floral, rose, muguet, fruity, apple, berry, citrus, creamy, woody, balsamic, amber, musk just to name a few, according to the odour grading scale set out in Table 6(i) herein below;
   (ii) a score on a scale of 1 to 5 for changes in the perceived fragrance profile change for the test compositions versus the controls according to the odour grading scale set out in Table 6(ii) herein below.

**Table 6(i) - Character Dominance Odour Grading Scale**

| **Score** | **Fragrance Character Dominance** |
|---|---|
| 0 | Not noticeable |
| 1 | Slight presence of the character |
| 2 | Moderate presence of the character |
| 3 | Dominance of the character |

**Table 6(ii) - Character Difference Odour Grading Scale**

| **Score** | **Fragrance Profile Change** |
|---|---|
| 1 | Fragrance profile is unchanged, *i.e.,* no difference between the sample vs. the control. |
| 2 | Slight fragrance profile change when compared directly with the control. |
| 3 | Moderate fragrance profile but similar character to the control, |
| 4 | Large difference in fragrance profile from the control. |
| 5 | Total difference in the fragrance profile from the control. |

The results of the panelists are averaged and if sufficient panelists are available, typically around 10, then the data can be analyzed using Analysis of Variance methods. The model treats the subject as a random effect and looks at the impact of product, time and the interaction between product and time. From the analysis, the least square means for the product and time interaction are obtained. These means (as well as their confidence intervals) are then plotted to enable comparisons between products at each time point. It should be noted that the confidence levels plotted are intended as a guide, and not as a statistical comparison, as they do not take into account that multiple testing has been performed. As well as a graphical assessment, statistical comparisons between the two products at each of the time points are performed with a Sidak correction for multiple comparisons. The p-values for the product differences are obtained, with p-values < 0.05 indicating a statistical difference between the two products at 5% significance (or 95% confidence). Typically for assessments by expert panelists of evaluators and perfumers there are 4-6 participants. In these cases a full statistical analysis is not possible and typically we observe that an difference in means of 0.75 on the scales used is considered to be meaningful, i.e.: 3 out of 4 experts gave the products grades with a difference of 1.

### Test Method 2b: Olfactory Tests b

In order to show the effect of the substantially non-odorous fragrance modulators and fragrance component on the perception of fragrance profile in a composition of the present invention, test compositions are made, as described in the Example section, and given to panelists to evaluate and describe.

At the testing facility, 20 µL samples of the compositions and the controls are applied to glass slides and placed on a hot plate at 32 °C to represent skin temperature for varying durations. Glass slides of samples that are to be later compared are prepared at the same time. The panelists are asked to evaluate the perceived fragrance profile (intensity and/or character) of each glass slide sample at a given time point. Slides are presented coded so that their identity is not known by the panelists. Within a given time point, panelists evaluate the slides in a random order and are able to revisit their assessment as they work through the slides at that time point. Their assessments are recorded. The experiments are run in duplicate on 2 different days and the data combined. In the subsequent analysis, the data for strength and character comparisons are drawn from the independent assessments carried out at a given time point.

Panelists are individuals who are specifically trained to evaluate fragrances according to the scale below using odour standards for calibration. Calibration standards are selected from perfume materials that represent specific families, for example, without being exhaustive, for the woody family panelists are calibrated with cedarwood, vetivert oil, patchouli oil, iso-E super, Norlimbanol and sandalwood or for harshness , for example, without being exhaustive, panelists are calibrated with pyrazines, Vertocitral, Aldehyde phenylacetic, Cedar Atlas and Cuminic aldehyde. Around 10-15 panelists are used to evaluate a given product and its control. Panelists assess the samples according to 2 scales:

### (a) Fragrance Intensity:

The panelists give a score on a unlabeled continuous scale where 0 is no perceptible odour and 10 is very strong odour.

### (b) Fragrance Character:

The panelists assess the fragrance character according to a number of pre-defined attributes e.g.: citrus, green, aromatic, floral, fruity, spicy, musk, woody, fresh, harsh just to name a few, scoring each one on unlabeled continuous scale where 0 is no perceptible odor and 10 is very strong odor.

The results of the panelists are analyzed using three-way analysis of variance (replicate / sample / panellist) with interaction followed by Duncan post-hoc multiple comparison test. The p-values for the product differences are obtained, with p-values < 0.05 indicating a statistical difference between products at 5% significance (or 95% confidence) and with p-values < 0.10 indicating a statistical difference between products at 10% significance (or 90% confidence). The data is represented graphically in bar or line charts showing the average for each attribute at a given time point with 95% confidence intervals as error bars.

### Test Method 3: Analytical Evaporation Tests

The following test is carried out to demonstrate the improved or enhanced longevity of a fragrance profile of a composition of the present invention vs. a control. In particular, the test measures the effect of a substantially non-odorous fragrance modulator on the evaporation rate of one or more fragrance materials formulated in a composition. The evaporation response of the fragrance materials to the modulator, as a function of time, is measured through the use of gas chromatography ("GC").
1. A test composition may comprise a substantially non-odorous fragrance modulator (any one of the modulators as disclosed in Tables 4(a) and 4(b)) with either: (i) a fragrance material (any one of the moderate volatile fragrance materials as disclosed in Table 2 and 2a and high volatile fragrance materials as disclosed in Table 3 and 3a, or (ii) a blend of fragrance materials from Tables 1a, 1b, 2a, 2b, 3a and 3c (as disclosed as Fragrance Examples 1 to 6 ). The test compositions also contain ethanol, and deionized water. Samples test compositions are provided in Tables 13, 14, 15 and 15 (d). All of the ingredients are admixed until evenly distributed in the test compositions.
2. A control composition to the test composition described in 1 above, without the substantially non-odorous fragrance modulator is made in a similar manner to Step 1, except that the missing substantially non-odorous modulator is replaced by deionized water. Sample control compositions are provided in Tables 13, 14, 15 and 15 (d)
3. An internal standard is needed to correct for variations of the amount of composition dispensed in the evaporation test as well as loss during the GC analysis. The internal standard has a vapor pressure of less than 0.001 Torr (0.000133 kPa) at 25 °C and is soluble in the composition and fragrance material. Alternatively the internal standard has a vapor pressure of less than 0.001 Torr (0.000133 kPa) at 25 °C and is soluble in ethanol or an ethanol/water solvent mixture to prepare an internal standard solution, which is then added to the fragrance material or composition. Suitable non-limiting examples of internal standards are triethyl citrate or denatonium benzoate. The internal standard and fragrance material, or blend of fragrance materials, are admixed until evenly distributed at a level of 90 to 95 parts by weight of fragrance material and the required amount of internal standard to reach 100 parts. This mixture is then used to prepare the sample compositions in Step 1 and 2. Alternatively, the internal standard is dissolved and diluted with ethanol or an ethanol/water mixture to obtain a solution. The internal standard solution and test or control composition are admixed until evenly distributed such that the resultant solution contains between 0.25 and 1.5% by weight of internal standard This resultant solution is used in subsequent steps.
4. A hotplate is set to a temperature of 32 °C. An aluminum container, such as TA Instruments T-Zero^{™} pan, is placed on the hotplate. 20 µL of the test or control composition is introduced in the aluminum container using a micropipette. Alternatively, the aluminum container may be filled with the test or control composition to its full capacity. The time at which this takes place is determined to be time zero (*i.e.,* T = 0). Multiple aluminum containers are prepared and left at the set temperature for pre-determined periods of time, such as for example 15 mins, 30 mins, 1 hr, 2 hrs, 3 hrs, 4 hrs, 5 hrs, 6 hrs, 8 hrs and up to 12 hrs.
5. The aluminum container is removed from the hotplate at the end of the pre-determined time period and transferred by being inserted into a 4 mL glass vial already containing at least 2 mL of highly volatile solvent, such as high purity ethanol or hexane.
6. The glass vial is mixed using a Heidolph multi REAX shaker, or equivalent, for 5 to 10 mins to extract the fragrance materials into the solvent phase. 1 mL of the resultant solution is transferred to a 2 mL GC vial.
7. The GC vial is analysed on an Agilent GC system 6890 equipped with an autosampler, or equivalent. A GC column such as a DB-5MS, ZB-5 lMSi models or equivalent phase, with a length of 30 m, an inner diameter of 0.25 mm and a film thickness of 1 µm is used. The GC parameters are set to the values indicated as follows:

**Table 6(iii) - GC Parameters**

| | |
|---|---|
| Injector temperature: | 250 °C |
| Initial gas velocity: | 25 to 40 cm/sec (for Helium as the carrier gas) |
| Initial oven temperature: | 50 °C |
| Temperature ramp: | 7 °C/min |
| Final oven temperature: | 325 °C |

Gas chromatograp hy with flame ionization detection ("FID") or with mass spectrometry ("MS") can be used for the identification and quantification of fragrance material in the compositions. Either detection system can be used in conjunction with GC. The column dimensions as well as GC settings described in this method, such as injector temperature, carrier gas velocity, temperature ramp and final oven temperature can be adjusted to optimize the response of the fragrance material and internal standard being monitored. The detection system settings, such as FID gas flows and temperature or MS parameters, should be optimized by a trained analyst to enable the precise detection and quantification of the analytes of interest.
8. The peak area of the fragrance material and internal standard are recorded. The peak area ratio of the fragrance material and the internal standard is calculated at each time point for each sample composition. The % of non-evaporated fragrance material remaining from T = 0 is calculated at each time point for each sample composition. The % fragrance material remaining in each composition is plotted to give an evaporation profile over time. This is done for both the test and control compositions. Significance is determined by comparison of the evaporation profile for the same fragrance material or same fragrance mixture in the test and control compositions. In addition, the sum of peak areas for a group of fragrance materials, such as high or moderate volatile fragrance materials, is calculated and used to determine the percentage of such materials remaining in each composition. Unidentified peaks were excluded. From the generated data the decay of the high volatility fragrance materials were analyzed. To determine the error, tests were run in triplicate and an average of the % fragrance materials remaining and a standard deviation value were calculated. An example of data output is shown in Table 6(iv). The output shows the standard deviation at 180 minutes, 120 minutes, 60 minutes, 30 minutes, and 15 minutes. Modulators tested include iso cetyl alcohol (ICA), Glucam, and a reference product (REF).

**Table 6(iv):**

| | | | | | |
|---|---|---|---|---|---|
| % remaining | 180 | 120 | 60 | 30 | 15 |
| ICA Average | 2.09 | 3.14 | 5.06 | 10.63 | 22.34 |
| ICA SD | 0.50 | 0.90 | 0.90 | 0.52 | 1.54 |
| REF Average | 0.11 | 0.27 | 1.34 | 3.67 | 6.60 |
| REF SD | 0.03 | 0.07 | 0.07 | 0.22 | 1.74 |
| Glucam average | 1.37 | 2.22 | 4.11 | 6.91 | 13.07 |
| Glucam SD | 0.93 | 0.99 | 1.33 | 1.87 | 3.25 |

### EXAMPLES

### Example 1 - Fragrance Oils

Fragrance examples 1, 2, 3, 4, 5, and 6 are provided below in Tables 7-12, respectively, as non-limiting examples of formulations of fragrance materials intended to form the fragrance component of the compositions of the present inventionif the modulator is chosen from methyl glucoside polyol, ethyl glucoside polyol, propyl glucoside polyol, or mixtures thereof.

Fragrance examples 1A, 2A, 3A, 4A, 5A and 6A provided in Tables 7-12, respectively, below are examples of traditional formulations of fragrance materials that fall outside the scope of the present invention.

The following fragrance formulations are made by mixing the listed ingredients in the listed proportions (wt%) at room temperature, wherein the wt% is relative to the total weight of the fragrance component.

**Table 7 - Fragrance Example 1**

| CAS Number | Perfume Material | Example 1 Parts (Weight %) | Comparative Example 1A (Weight %) | Vapour Pressure (Torr at 25 °C) | Volatility |
|---|---|---|---|---|---|
| Natural | Tangerine Oil | 6.700-7.000 | 4.900 | | High |
| Natural | Elemi Coeur Oil | 9.500-10.500 | 7.000 | | High |
| Natural | Lemon Oil Winter | 2.200-2.500 | 1.600 | | High |
| Natural | Bergamot Oil Reggio Early New Crop | 4.400-4.800 | 3.300 | | High |
| 68039-49-6 | LIGUSTRAL OR TRIPLAL | 0.010-0.0160 | 0.010 | 0.57800 | High |
| Natural | Cumin Oil | 0.500-0.6500 | 0.424 | | High |
| Natural | Pepper Black Oil | 1.300-1.500 | 1.000 | | High |
| Natural | Pink Pepper CO2 Oil | 1.300-1.500 | 1.000 | | High |
| 67674-46-8 | Methyl Pamplemousse | 2.600-2.800 | 2.000 | 0.21400 | High |
| 93-92-5 | Methyl Phenyl Carbinol Acetate | 0.260-0.290 | 0.200 | 0.20300 | High |
| 115-95-7 | linalyl acetate | 8.000-8.400 | 6.266 | 0.11600 | High |
| Natural | Clary Sage Oil French | 0.550-0.580 | 0.400 | | High |
| 88-41-5 | verdox | 1.350-1.400 | 1.000 | 0.10300 | High |
| 120-72-9 | Indole | 0.010-0.160 | 0.010 | 0.02980 | Moderate |
| 134-20-3 | Methyl anthranilate | 1.300-1.400 | 0.100 | 0.01580 | Moderate |
| 19870-74-7 | CEDRYL METHYL ETHER | 2.600-2.900 | 2.000 | 0.01280 | Moderate |
| 54440-17-4 | Safraleine | 0.050-0.060 | 0.039 | 0.01260 | Moderate |
| 97-53-0 | eugenol | 0.700-0.900 | 0.600 | 0.01040 | Moderate |
| 6790-58-5 | Ambronat^{®} | 1.350-1.400 | 1.000 | 0.00930 | Moderate |
| 104-61-0 | γ-NONALACTONE | 0.500-0.600 | 0.390 | 0.00858 | Moderate |
| Natural | Cedar Atlas Oil | 5.400-5.600 | 4.000 | | Moderate |
| Natural | Papyrus Oil | 1.050-1.150 | 0.780 | | Moderate |
| 10339-55-6 | ethyl linalool | 8.000-8.400 | 6.500 | 0.00520 | Moderate |
| 97-54-1 | Isoeugenol | 0.050-0.200 | 0.078 | 0.00519 | Moderate |
| 23696-85-7 | Damascenone | 0.250-0.300 | 0.204 | 0.00503 | Moderate |
| 127-51-5 | Isoraldeine | 0.095-1.120 | 0.800 | 0.00282 | Moderate |
| 33704-61-9 | Cashmeran | 2.500-3.000 | 2.000 | 0.00269 | Moderate |
| 121-33-5 | Vanillin | 15.100-15.200 | 10.000 | 0.00194 | Moderate |
| Natural | Tonka Bean Absolute | 10.000-10.050 | 7.000 | 0.00141 | Moderate |
| 121-32-4 | Ethyl Vanillin | 2.500-3.000 | 2.000 | 0.00088 | Low |
| 4940-11-8 | Ethyl Maltol | 0.400-0.600 | 0.400 | 0.00023 | Low |
| Natural | Guaiacwood Oil | 2.600-2.850 | 2.000 | | Low |
| 63314-79-4 | Delta Muscenone | 2.150-2.250 | 30.000 | 0.00005 | Low |
| Natural | Vetivert Oil | 1.300-1.400 | 1.000 | | Low |
| | Total | 100.00 | | | |

### Example 1 Oil structure:

40.4% high volatile perfume materials;
50.0% moderate volatile perfume materials;
9.6% low volatile perfume materials.

**Table 8 - Fragrance Example 2**

| CAS Number | Perfume Material | Example 2 Parts (Weight %) | Comparative Example 2A (Weight %) | Vapour Pressure (Torr at 25 °C) | Volatility |
|---|---|---|---|---|---|
| Natural | Lavandin Grosso Oil | 0.600-0.700 | 0.467 | | High |
| 39255-32-8 | Manzanate | 0.040-0.050 | 0.031 | 2.90600 | High |
| Natural | Cypress Oil | 0.190-0.250 | 0.156 | | High |
| 3681-71-8 | cis-3-Hexenyl acetate | 0.040-0.050 | 0.031 | 1.21900 | High |
| Natural | Lemon Oil Winter | 3.700-4.000 | 2.802 | | High |
| 928-96-1 | Cis-3-hexenol | 0.110-0.130 | 0.093 | 1.03900 | High |
| Natural | Bergamot oil Reggio Early New Crop | 4.200-4.300 | 3.113 | | High |
| 67633-96-9 | Liffarome^{™} | 0.080-0.090 | 0.062 | 0.72100 | High |
| Natural | Cardamom Oil Guatemala | 0.150-0.190 | 0.125 | | High |
| 68039-49-6 | LIGUSTRAL OR TRIPLAL | 0.040-0.045 | 0.031 | 0.57800 | High |
| Natural | Basil Oil Grand Vert | 0.400-0.500 | 0.311 | | High |
| 18479-58-8 | DIHYDRO MYRCENOL | 17.300-17.900 | 12.954 | 0.16600 | High |
| 88-41-5 | verdox | 7.300-7.600 | 5.448 | 0.10300 | High |
| 78-70-6 | Linalool | 1.450-1.500 | 1.090 | 0.09050 | Moderate |
| 60-12-8 | Phenyl Ethyl Alcohol | 0.200-0.250 | 0.156 | 0.07410 | Moderate |
| Natural | Rose Absolute oil | 0.040-0.050 | 0.031 | | Moderate |
| Natural | Violet Leaves Absolute | 0.040-0.050 | 0.031 | | Moderate |
| Natural | Geranium Bourbon | 0.085 0.075-0.090 | 0.062 | | Moderate |
| 67634-00-8 | Allyl Amyl Glycolate | 0.350-0.400 | 0.280 | 0.04000 | Moderate |
| 134-20-3 | methyl Anthranilate | 0.040-0.050 | 0.031 | 0.01580 | Moderate |
| 150-84-5 | Citronellyl acetate | 0.040-0.050 | 0.031 | 0.01370 | Moderate |
| 68845-00-1 | Boisiris | 3.390-3.410 | 2.491 | 0.01350 | Moderate |
| 106-24-1 | geraniol | 0.800-0.900 | 0.623 | 0.01330 | Moderate |
| 19870-74-7 | CEDRYL METHYL ETHER | 4.500-4.700 | 3.425 | 0.01280 | Moderate |
| 120-57-0 | heliotropin | 0.040-0.050 | 0.031 | 0.01040 | Moderate |
| 3025-30-7 | Ethyl 2 4-Decadienoate | 0.110-0.130 | 0.093 | 0.00954 | Moderate |
| 6790-58-5 | Ambronat^{®} | 0.600-0.700 | 0.467 | 0.00930 | Moderate |
| 2705-87-5 | Allyl Cyclohexane Propionate | 0.200-0.300 | 0.156 | 0.00925 | Moderate |
| Natural | Cedar Atlas Oil | 4.400-4.800 | 3.425 | | Moderate |
| 56973-85-4 | Neobutenone α | 0.110-0.130 | 0.093 | 0.00763 | Moderate |
| 63500-71-0 | florol | 2.100-2.200 | 1.557 | 0.00557 | Moderate |
| 10339-55-6 | Ethyl Linalool | 19.900-20.000 | 14.594 | 0.00520 | Moderate |
| 23696-85-7 | Damascenone | 0.068-0.075 | 0.053 | 0.00503 | Moderate |
| 58567-11-6 | BOISAMBRENE FORTE | 3.380-3.500 | 2.491 | 0.00433 | Moderate |
| 173445-65-3 | HIVERNAL | 0.200-0.300 | 0.187 | 0.00392 | Moderate |
| 93-29-8 | Iso Eugenol Acetate | 0.080-0.090 | 0.062 | 0.00324 | Moderate |
| 476332-65-7 | AMBER XTREME*¹* | 0.080-0.090 | 0.006 | 0.00323 | Moderate |
| 68901-15-5 | Cyclogalbanate | 0.200-0.215 | 0.156 | 0.00323 | Moderate |
| 127-51-5 | Isoraldeine | 0.800-0.900 | 0.623 | 0.00282 | Moderate |
| 1205-17-0 | helional | 1.650-1.750 | 1.245 | 0.00270 | Moderate |
| 33704-61-9 | Cashmeran | 0.800-0.900 | 0.623 | 0.00269 | Moderate |
| 141-13-9 | Adoxal | 0.200-0.220 | 0.156 | 0.00257 | Moderate |
| 121-33-5 | vanillin | 0.400-0.450 | 0.311 | 0.00194 | Moderate |
| Natural | Ginger Oil India | 0.150-0.190 | 0.125 | | Moderate |
| 91-64-5 | Coumarin | 0.095-1.100 | 0.778 | 0.00130 | Moderate |
| 28940-11-6 | Calone | 0.040-0.045 | 0.031 | 0.00083 | Low |
| 24851-98-7 | Hedione^{®} HC | 7.700-7.900 | 5.739 | 0.00071 | Low |
| 70788-30-6 | norlimbanol | 0.600-0.650 | 0.467 | 0.00047 | Low |
| 65405-77-8 | cis-3-Hexenyl salicylate | 2.000-2.200 | 10.874 | 0.00025 | Low |
| 107898-54-4 | Polysantol^{®} | 2.800-3.100 | 10.005 | 0.00012 | Low |
| 63314-79-4 | delta muscenone | 0.800-0.950 | 2.859 | 0.00005 | Low |
| Natural | Vetivert Oil | 1.650-1.750 | 8.700 | | Low |
| 4707-47-5 | LRG 201/Evernyl | 0.280-0.300 | 0.218 | 0.00001 | Low |
| | Total | 100.00 | | | |

| | | | | | |
|---|---|---|---|---|---|
| *¹* added as a 10% solution in DPG; amount given is pure material added | | | | | |

### Example 2 Oil structure:

35.0% high volatile perfume materials;
48.5% moderate volatile perfume materials;
16.5% low volatile perfume materials.

**Table 9 - Fragrance Example 3**

| CAS Number | Perfume Material | Example 3 Parts (Weight %) | Comparative Example 3A (Weight %) | Vapour Pressure (Torr at 25 °C) | Volatility |
|---|---|---|---|---|---|
| 39255-32-8 | Manzanate | 0.450-0.550 | 0.342 | 2.90600 | High |
| Natural | Cypress Oil | 0.200-0.300 | 0.171 | | High |
| 14667-55-1 | Trimethyl Pyrazine-2,3,5 | 0.005-0.015 | 0.007 | 1.72400 | High |
| Natural | Tangerine Oil | 2.000-3.000 | 1.711 | | High |
| 3681-71-8 | cis-3-Hexenyl acetate | 0.010-0.020 | 0.011 | 1.21900 | High |
| 928-96-1 | Cis-3-hexenol | 0.150-0.170 | 0.114 | 1.03900 | High |
| 141-97-9 | ETHYL ACETOACETATE | 0.150-0.170 | 0.114 | 0.89000 | High |
| Natural | Bergamot Oil Reggio Early New Crop | 9.000-9.500 | 6.273 | | High |
| Natural | Coffee Extract CO2 | 6.500-7.000 | 4.587 | | High |
| 67633-96-9 | Liffarome^{™} | 0.050-0.120 | 0.080 | 0.72100 | High |
| Natural | Cardamom Oil Guatemala | 0.600-0.700 | 0.456 | 0.60345 | High |
| 68039-49-6 | LIGUSTRAL OR TRIPLAL | 0.200-0.300 | 0.171 | 0.57800 | High |
| Natural | Pepper Black CO2 Oil | 0.800-0.900 | 0.570 | 0.25326 | High |
| 76-22-2 | Camphor gum | 0.045-0.055 | 0.034 | 0.22500 | High |
| 67674-46-8 | Methyl Pamplemousse | 1.650-1.700 | 1.141 | 0.21400 | High |
| 112-31-2 | DECYL ALDEHYDE*¹* | 0.045-0.055 | 0.034 | 0.20700 | High |
| 93-92-5 | Methyl Phenyl Carbinol Acetate | 0.650-0.700 | 0.456 | 0.20300 | High |
| 18479-58-8 | DIHYDRO MYRCENOL | 6.500-7.000 | 4.562 | 0.16600 | High |
| 88-41-5 | verdox | 6.500-7.000 | 4.562 | 0.10300 | High |
| 104-46-1 | Anethol | 0.075-0.090 | 0.057 | 0.06870 | Moderate |
| 67634-00-8 | Allyl Amyl Glycolate | 0.300-0.350 | 0.228 | 0.04000 | Moderate |
| 464-45-9 | l-Borneol | 0.010-0.020 | 0.011 | 0.03980 | Moderate |
| Natural | Cinnamon Bark Oil | 0.080-0.090 | 0.057 | | Moderate |
| 134-20-3 | METHYL ANTHRANILATE | 0.010-0.020 | 0.011 | 0.01580 | Moderate |
| 19870-74-7 | CEDRYL METHYL ETHER | 6.500-7.000 | 4.562 | 0.01280 | Moderate |
| 27538-10-9 | HOMOFURONOL² | 0.020-0.030 | 0.016 | 0.01210 | Moderate |
| 67634-15-5 | Floralozone | 0.300-0.400 | 0.228 | 0.01110 | Moderate |
| 5462-06-6 | CANTHOXAL | 0.060-0.070 | 0.046 | 0.01020 | Moderate |
| 6790-58-5 | Ambronat^{®} | 6.500-7.000 | 4.562 | 0.00930 | Moderate |
| 104-61-0 | γ-NONALACTONE | 0.160-0.170 | 0.114 | 0.00858 | Moderate |
| Natural | Cedar Atlas Oil | 3.200-3.400 | 2.281 | | Moderate |
| 56973-85-4 | NEOBUTENONE α | 0.010-0.020 | 0.011 | 0.00763 | Moderate |
| 103-60-6 | Phenoxy Ethyl Iso Butyrate | 0.140-0.180 | 0.114 | 0.00562 | Moderate |
| 10339-55-6 | Ethyl Linalool | 2.500-2.700 | 1.768 | 0.00520 | Moderate |
| 23696-85-7 | Damascenone | 0.040-0.060 | 0.034 | 0.00503 | Moderate |
| 58567-11-6 | BOISAMBRENE FORTE | 1.670-1.690 | 1.141 | 0.00433 | Moderate |
| 68901-15-5 | Cyclogalbanate | 0.015-0.018 | 0.011 | 0.00323 | Moderate |
| 127-51-5 | Isoraldeine | 0.750-0.900 | 0.570 | 0.00282 | Moderate |
| 104-67-6 | UNDECALACTONE | 0.030-0.070 | 0.034 | 0.00271 | Moderate |
| 1205-17-0 | helional | 3.200-3.400 | 2.281 | 0.00270 | Moderate |
| 33704-61-9 | cashmeran | 3.200-3.400 | 2.281 | 0.00269 | Moderate |
| 121-33-5 | vanillin | 8.300-8.600 | 5.703 | 0.00194 | Moderate |
| 10094-34-5 | DIMETHYL BENZYL CARBINYL BUTYRATE | 0.830-0.850 | 0.570 | 0.00168 | Moderate |
| Natural | Tonka Bean Absolute | 4.000-4.400 | 2.852 | | Moderate |
| 121-32-4 | Ethyl Vanillin | 1.550-1.700 | 1.141 | 0.00088 | Low |
| 24851-98-7 | Hedione^{®} HC | 2.400-2.600 | 1.711 | 0.00071 | Low |
| 54464-57-2 | Iso E Super | 6.600-6.800 | 23.383 | 0.00054 | Low |
| 70788-30-6 | Norlimbanol | 1.500-1.700 | 4.562 | 0.00047 | Low |
| 28219-61-6 | Laevo Trisandol | 3.600-3.800 | 10.037 | 0.00028 | Low |
| 57082-24-3 | Caryophyllene acetate | 0.150-0.170 | 0.114 | 0.00025 | Low |
| 4940-11-8 | Ethyl Maltol | 0.830-0.850 | 0.570 | 0.00023 | Low |
| Natural | Guaiacwood Oil | 0.830-0.850 | 2.281 | | Low |
| 63314-79-4 | Delta Muscenone | 0.300-0.350 | 0.912 | 0.00005 | Low |
| 4707-47-5 | LRG 201/Evernyl | 0.150-0.175 | 0.114 | 0.00001 | Low |
| Natural | Cocoa Colourless Oil | 0.300-0.400 | 0.228 | | Low |
| Total | | 100.000 | | | |

| | | | | | |
|---|---|---|---|---|---|
| *¹* added as a 10% solution in DPG; amount given is pure material added *²* added as a 20% solution in triethyl citrate; amount given is pure material added | | | | | |

### Example 3 Oil structure:

37.4% high volatile perfume materials;
43.6% moderate volatile perfume materials;
19.0% low volatile perfume materials.

**Table 10 - Fragrance Example 4**

| CAS Number | Perfume Material | Example 4 Parts (Weight %) | Comparative Example 4A (Weight %) | Vapour Pressure (Torr at 25 °C) | Volatility |
|---|---|---|---|---|---|
| 39255-32-8 | Manzanate | 0.010-0.020 | 0.009 | 2.90600 | High |
| Natural | Nutmeg oil | 0.030-0.070 | 0.028 | | High |
| Natural | Tangerine Oil | 3.100-3.400 | 1.856 | | High |
| Natural | Lemon Oil Winter | 1.690-1.730 | 0.954 | | High |
| 928-96-1 | Cis-3-hexenol | 0.100-0.150 | 0.074 | 1.03900 | High |
| Natural | Bergamot Oil Reggio Early New Crop | 4.700-5.100 | 2.784 | | High |
| 67633-96-9 | Liffarome^{™} | 0.130-0.170 | 0.084 | 0.72100 | High |
| 106-72-9 | MELONAL | 0.100-0.150 | 0.074 | 0.62200 | High |
| Natural | Cardamom Oil Guatemala | 0.333-0.999 | 0.371 | | High |
| 68039-49-6 | LIGUSTRAL OR TRIPLAL | 0.100-0.200 | 0.093 | 0.57800 | High |
| 18479-58-8 | DIHYDRO MYRCENOL | 11.400-11.800 | 6.522 | 0.16600 | High |
| 115-95-7 | Linalyl acetate | 4.800-5.000 | 2.784 | 0.11600 | High |
| Natural | Clary Sage Oil French | 0.777-1.100 | 0.557 | | High |
| 88-41-5 | Verdox | 4.800-5.100 | 2.784 | 0.10300 | High |
| 78-70-6 | linalool | 3.000-3.300 | 1.829 | 0.09050 | Moderate |
| 104-46-1 | Anethol | 0.100-0.200 | 0.093 | 0.06870 | Moderate |
| Natural | Cinnamon Oil | 0.025-0.075 | 0.028 | | Moderate |
| 14901-07-6 | IONONE BETA | 0.100-0.400 | 0.186 | 0.01690 | Moderate |
| Natural | Petitgrain Mandarinier Oil | 0.800-1.100 | 0.557 | | Moderate |
| 19870-74-7 | CEDRYL METHYL ETHER | 4.800-5.100 | 2.784 | 0.01280 | Moderate |
| 67634-15-5 | Floralozone | 0.200-0.400 | 0.186 | 0.01110 | Moderate |
| 6790-58-5 | Ambronat^{®} | 0.800-1.100 | 0.557 | 0.00984 | Moderate |
| Natural | Cedar Atlas Oil | 11.500-11.700 | 6.496 | | Moderate |
| 63500-71-0 | Florol | 4.000-4.200 | 2.320 | 0.00789 | Moderate |
| 10339-55-6 | ethyl linalool | 9.800-10.000 | 5.567 | 0.00557 | Moderate |
| 23696-85-7 | Damascenone | 0.025-0.075 | 0.028 | 0.00520 | Moderate |
| 127-51-5 | Isoraldeine | 3.200-3.400 | 1.856 | 0.00503 | Moderate |
| 1205-17-0 | Helional | 2.200-2.600 | 1.392 | 0.00282 | Moderate |
| 33704-61-9 | cashmeran | 1.300-1.800 | 0.928 | 0.00270 | Moderate |
| 36306-87-3 | LRG 182/Kephalis | 4.000-4.200 | 2.320 | 0.00269 | Moderate |
| 121-33-5 | Vanillin | 0.100-0.200 | 0.093 | 0.00269 | Moderate |
| 2050-08-0 | Amyl Salicylate | 0.650-0.750 | 0.398 | 0.00194 | Moderate |
| Natural | Tonka Bean Absolute | 0.010-0.050 | 0.019 | | Moderate |
| 198404-98-7 | Javanol^{®} | 0.010-0.050 | 1.578 | 0.00090 | Low |
| 28940-11-6 | Calone | 0.010-0.050 | 1.578 | 0.00083 | Low |
| 70788-30-6 | norlimbanol | 0.600-1.00 | 1.856 | 0.00047 | Low |
| 67801-20-1 | Ebanol | 0.333-0.999 | 2.227 | 0.00028 | Low |
| 28219-61-6 | Laevo Trisandol | 4.700-5.100 | 16.705 | 0.00028 | Low |
| Natural | Guaiacwood Oil | 1.000-1.400 | 4.295 | | Low |
| 66072-32-0 | Iso Bornyl Cyclohexanol | 7.200-7.600 | 25.058 | 0.00003 | Low |
| 4707-47-5 | LRG 201/Evernyl | 0.100-0.180 | 0.093 | 0.00001 | Low |
| | Total | 100.000 | | | |

### Example 4 Oil structure:

34.1% high volatile perfume materials;
49.7% moderate volatile perfume materials;
16.1% low volatile perfume materials.

**Table 11 - Fragrance Example 5**

| CAS Number | Perfume Material | Example 5 Parts (Weight %) | Comparative Example 5A (Weight %) | Vapour Pressure (Torr at 25 °C) | Volatility |
|---|---|---|---|---|---|
| Natural | Cypress Oil | 1.600-2.000 | 1.337 | | High |
| Natural | Nutmeg Oil | 0.300-0.400 | 0.267 | | High |
| Natural | Lemon Oil WInter | 26.900-27.100 | 20.059 | | High |
| Natural | Black Pepper Oil | 2.600-2.750 | 2.006 | | High |
| Natural | Pink Pepper CO2 Oil | 4.400-4.550 | 3.343 | | High |
| Natural | Clary Sage Oil French | 3.500-3.700 | 2.675 | | High |
| 120-72-9 | Indole*¹* | 0.020-0.030 | 0.017 | 0.02980 | Moderate |
| 105-87-3 | Geranyl Acetate | 0.160-0.200 | 0.134 | 0.02560 | Moderate |
| Natural | Cinnamon Bark Oil | 0.040-0.050 | 0.033 | | Moderate |
| 134-20-3 | Methyl anthranilate | 0.400-0.500 | 0.334 | 0.01580 | Moderate |
| 67634-15-5 | FLORALOZONE | 0.200-0.250 | 0.167 | 0.01110 | Moderate |
| 97-53-0 | eugenol | 0.130-0.140 | 0.100 | 0.01040 | Moderate |
| Natural | Cedar Atlas Oil | 4.490-4.550 | 3.343 | | Moderate |
| 58567-11-6 | BOISAMBRENE FORTE | 4.490-4.550 | 3.343 | 0.00433 | Moderate |
| 93-29-8 | Iso Eugenol Acetate | 0.700-1.00 | 0.669 | 0.00324 | Moderate |
| 127-51-5 | Isoraldeine | 2.500-2.900 | 2.006 | 0.00282 | Moderate |
| 33704-61-9 | CASHMERAN | 13.300-13.700 | 10.030 | 0.00269 | Moderate |
| 121-33-5 | VANILLIN | 0.700-1.00 | 0.669 | 0.00194 | Moderate |
| Natural | Tonka Bean Absolute | 16.000-16.300 | 13.661 | | Moderate |
| 5471-51-2 | Para Hydroxy Phenyl Butanone | 0.100-0.150 | 0.100 | 0.00106 | Moderate |
| 70788-30-6 | Norlimbanol | 1.700-1.900 | 1.337 | 0.00047 | Low |
| 95962-14-4 | nectaryl | 0.430-0.470 | 2.006 | 0.00037 | Low |
| 4940-11-8 | Ethyl Maltol | 0.250-0.290 | 0.201 | 0.00023 | Low |
| Natural | Guaiacwood Oil | 1.700-1.900 | 1.337 | | Low |
| 107898-54-4 | Polysantol^{®} | 2.000-2.500 | 10.030 | 0.00012 | Low |
| Base | BIRCH LEAF GIVCO 166 2015C | 0.850-0.950 | 0.669 | 0.00005 | Low |
| Natural | Ciste Absolute | 0.080-0.100 | 0.067 | | Low |
| 63314-79-4 | Delta Muscenone | 0.350-0.550 | 0.334 | 0.00005 | Low |
| 21145-77-7 | Musk Plus | 4.450-4.550 | 15.045 | 0.00003 | Low |
| 4707-47-5 | LRG 201/Evernyl | 3.100-3.200 | 4.681 | 0.00001 | Low |
| | Total | 100.000 | | | |

| | | | | | |
|---|---|---|---|---|---|
| *¹* added as a 10% solution in DPG; amount given is pure material added | | | | | |

### Example 5 Oil structure:

40.0% high volatile perfume materials;
44.4% moderate volatile perfume materials;
15.7% low volatile perfume materials.

**Table 12 - Fragrance Example 6**

| CAS Number | Perfume Material | Example 6 Parts (Weight %) | Comparative Example 6A (Weight %) | Vapour Pressure (Torr at 25 °C) | Volatility |
|---|---|---|---|---|---|
| Natural | Cypress Oil | 1.350-1.500 | 1.000 | | High |
| Natural | Nutmeg Oil | 0.200-0.300 | 0.200 | | High |
| Natural | Lemon Oil Winter | 21.300-21.500 | 14.000 | | High |
| Natural | Black Pepper Oil | 2.100-2.200 | 1.500 | | High |
| Natural | Pink Pepper CO2 Oil | 3.500-3.600 | 2.500 | | High |
| Natural | Clary Sage Oil French | 2.800-2.900 | 2.000 | | High |
| 120-72-9 | Indole*¹* | 0.020-0.030 | 0.018 | 0.02980 | Moderate |
| 105-87-3 | Geranyl Acetate | 0.200-0.300 | 0.145 | 0.02560 | Moderate |
| Natural | Cinnamon Bark oil | 0.050-0.060 | 0.035 | | Moderate |
| 134-20-3 | Methyl anthranilate | 0.500-0.550 | 0.363 | 0.01580 | Moderate |
| 67634-15-5 | FLORALOZONE | 0.200-0.300 | 0.180 | 0.01110 | Moderate |
| 97-53-0 | eugenol | 0.140-0.160 | 0.110 | 0.01040 | Moderate |
| Natural | Cedar Atlas | 5.000-5.130 | 5.000 | | Moderate |
| 58567-11-6 | BOISAMBRENE FORTE | 5.000-5.130 | 5.000 | 0.00433 | Moderate |
| 93-29-8 | Iso Eugenol Acetate | 0.999-1.100 | 1.000 | 0.00324 | Moderate |
| 127-51-5 | Isoraldeine | 3.000-3.100 | 2.100 | 0.00282 | Moderate |
| 33704-61-9 | CASHMERAN | 15.300-15.400 | 10.000 | 0.00269 | Moderate |
| 121-33-5 | VANILLIN | 0.999-1.100 | 1.000 | 0.00194 | Moderate |
| Natural | Tonka Bean Absolute | 18.000-18.500 | 13.000 | | Moderate |
| 5471-51-2 | Para Hydroxy Phenyl Butanone | 0.140-0.160 | 0.100 | 0.00106 | Moderate |
| 70788-30-6 | Norlimbanol | 1.900-2.200 | 1.800 | 0.00047 | Low |
| 95962-14-4 | nectaryl | 0.500-0.520 | 2.000 | 0.00037 | Low |
| 4940-11-8 | Ethyl Maltol | 0.200-0.400 | 0.220 | 0.00023 | Low |
| Natural | Guaiacwood Oil | 1.900-2.100 | 1.450 | | Low |
| 107898-54-4 | Polysantol^{®} | 2.400-2.600 | 10.819 | 0.00012 | Low |
| Base | BIRCH LEAF GIVCO 166 | 0.999-1.100 | 1.000 | 0.00005 | Low |
| Natural | Ciste Absolute | 0.090-0.150 | 0.100 | 0.00005 | Low |
| 63314-79-4 | Delta Muscenone | 0.490-0.530 | 0.360 | 0.00005 | Low |
| 21145-77-7 | Musk Plus | 4.900-5.130 | 18.000 | 0.00003 | Low |
| 4707-47-5 | LRG 201/Evernyl | 3.300-3.700 | 5.000 | 0.00001 | Low |

| | | | | | |
|---|---|---|---|---|---|
| *¹* added as a 10% solution in DPG; amount given is pure material added | | | | | |

### Example 6 Oil structure:

31.8 % high volatile perfume materials;
50.4 % moderate volatile perfume materials;
17.8 % low volatile perfume materials.

### Example 7 - Compositions Comprising Fragrance Oils and Substantially Non-Odorous

### Fragrance Modulators

Compositions A1, D1, G1, J1, M1 are examples of fragrance compositions not according to the present invention, made with any one of fragrance example oils 1 2, 3, 4, 5, 6 respectively. In parallel, control compositions B1, E1, H1, K1, N1 are prepared by replacing the different substantially non-odorous fragrance modulators by the same amount of deionized water. In addition compositions C1, F1, I1, L1, O1 are examples of fragrance compositions prepared using traditionally constructed oils 1A, 2A, 3A, 4A, 5A and 6A in the absence of a modulator. All of the compositions are prepared by admixture of the components described in Table 13 in the proportions indicated.

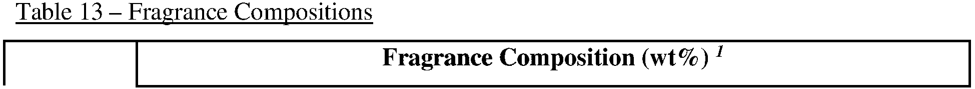

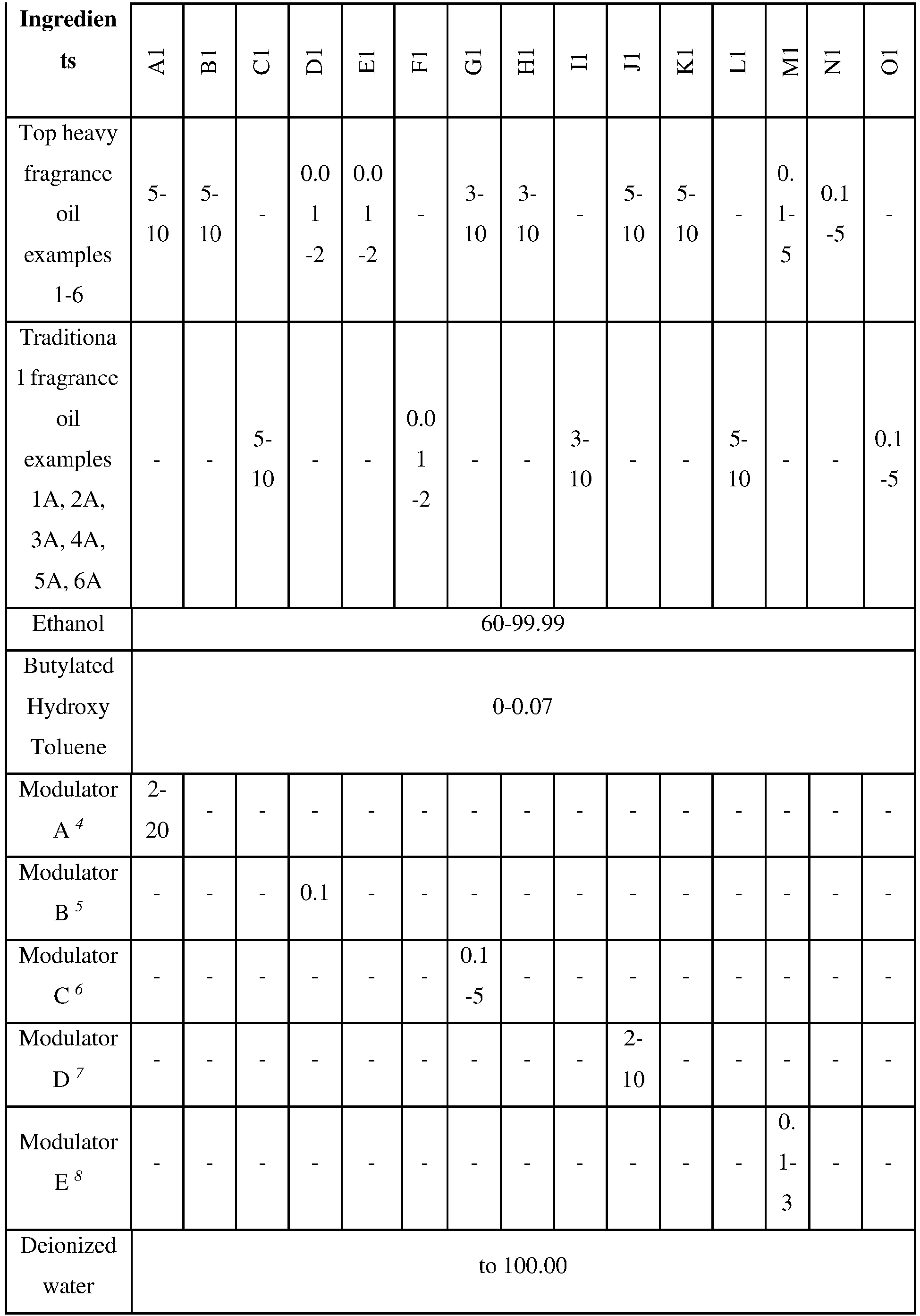

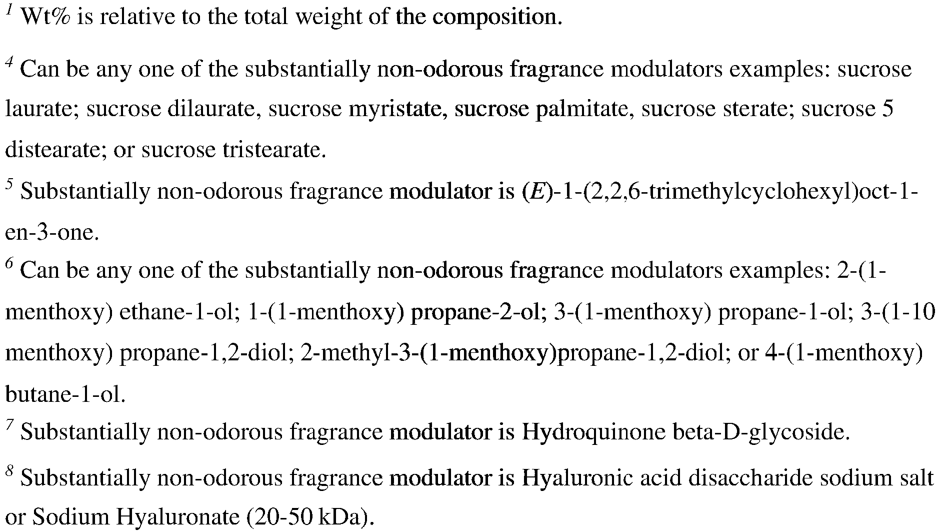

Compositions A2, D2, G2, J2, M2 are examples of fragrance compositions not according to the present invention, made with any one of fragrance examples 1, 2, 3 ,4, 5 and 6 respectively. In parallel, control compositions B2, E2, H2, K2, N2 are prepared by replacing the different substantially non-odorous fragrance modulators by the same amount of deionized water. In addition compositions C2, F2, I2, L2, O2 are examples of fragrance compositions prepared using traditionally constructed oils 1A, 2A, 3A, 4A, 5A and 6A in the absence of a modulator. All of the compositions are prepared by admixture of the components described in Table 14 in the proportions indicated.

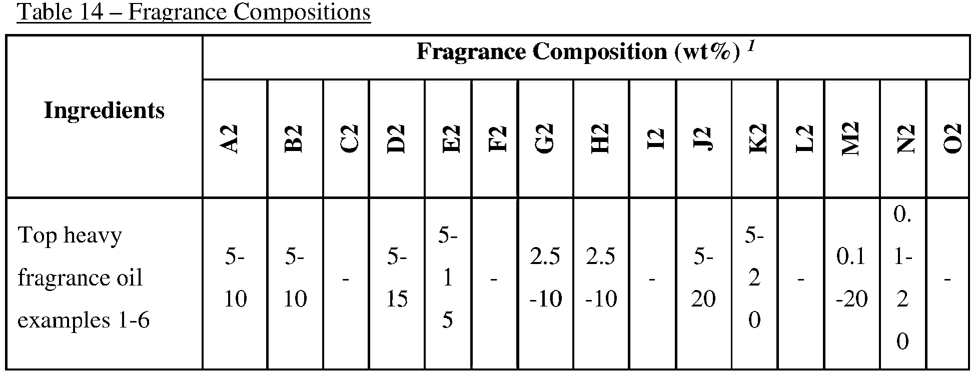

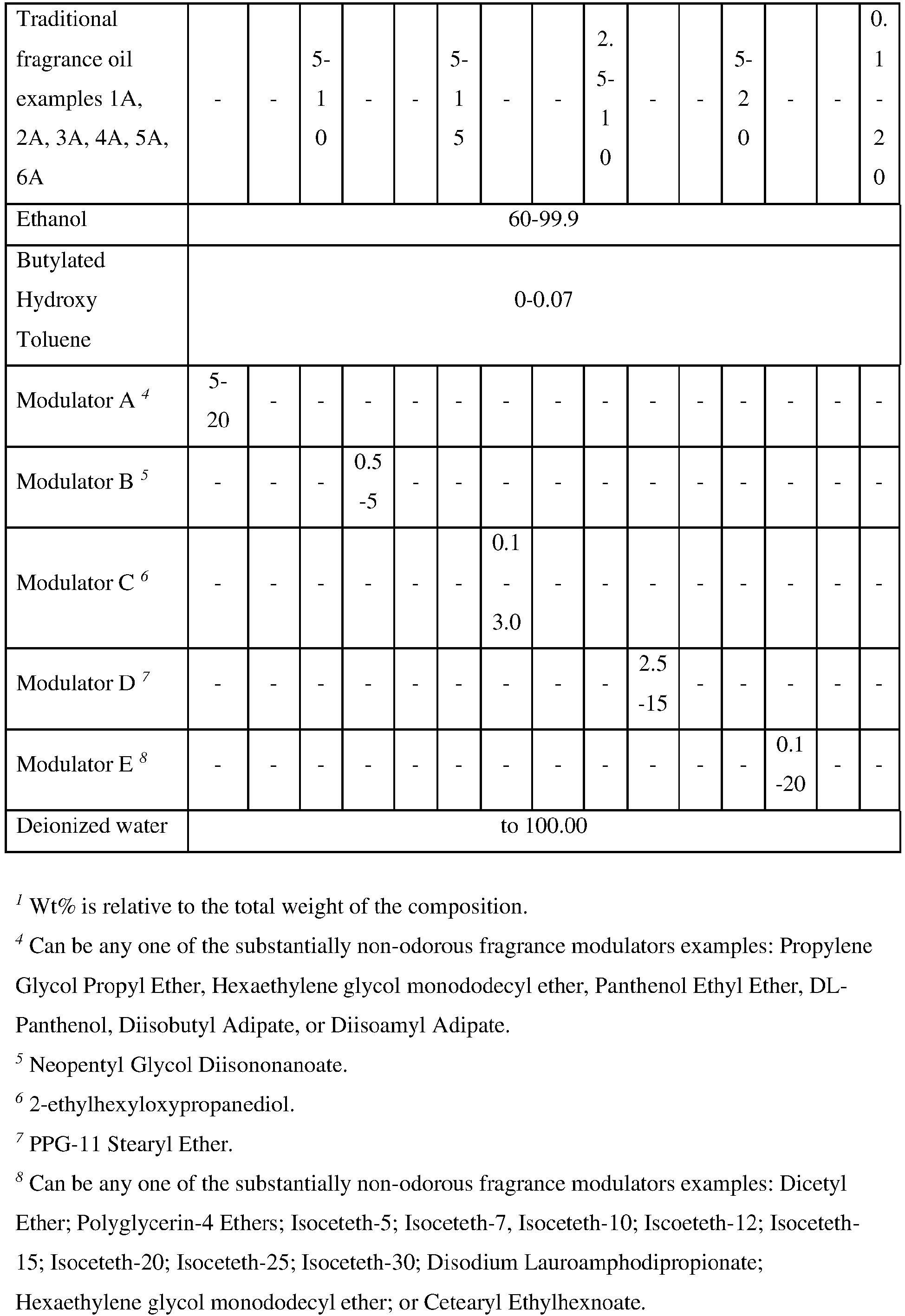

Composition A3 is an example of a fragrance composition according to the present invention, made with any of the fragrance examples 1, 2, 3, 4, 5 and 6, respectively. In parallel, a control composition B3 is prepared by replacing the different substantially non-odorous fragrance fixative by the same amount of deionized water. Composition C3 is an example of a fragrance composition containing traditional or higher levels of low volatile fragrance materials, made with any of the fragrance examples 1A, 2A, 3A, 4A, 5A and 6A, respectively. All of the compositions are prepared by admixture of the components described in Table 15 in the proportions indicated.

**Table15 - Fragrance Composition**

| **Ingredients** | **Fragrance Composition (wt%)*¹*** | | |
|---|---|---|---|
| | **A3** | **B3** | **C3** |
| Top heavy fragrance oil examples 1-6 | 2-15 | 2-15 | - |
| Traditional fragrance oil examples 1A, 2A, 3A, 4A, 5A, 6A | - | - | 2-15 |
| Ethanol | 60-99.99 | | |
| Butylated Hydroxy Toluene | 0-0.07 | | |
| Modulator A *⁴* | 0.1-20 | - | - |
| Deionized water | to 100.00 | | |

| | | | |
|---|---|---|---|
| *¹* Wt% is relative to the total weight of the composition. *⁴* Can be any one of the substantially non-odorous fragrance modulator as disclosed in Tables 4 (a) and 4(b) that is methyl glucoside polyol, ethyt glucoside polyol, propyl glucoside polyol, or mixtures thereof. | | | |

Composition A4 is an example of a fragrance composition according to the present invention and compositions D4, G4, and J4 are not examples of fragrance compositions according to the present invention, made with any one of fragrance oil examples 1, 2, 3 ,4 ,5 and 6, respectively. In parallel, control compositions B4, E4, H4, and K4 are prepared by replacing the different substantially non-odorous fragrance modulators by the same amount of deionized water or ethanol. Compositions C4, F4, I4, and L4 are examples of fragrance compositions containing any one of the following fragrance examples 1A, 2A, 3A, 4A, 5A, 6A, and which are outside the scope of the present invention. All of the compositions are prepared by admixture of the components described in Table 15(a), in the proportions indicated.

**Table 15(a) - Fragrance Compositions**

| **Ingredients** | **Fragrance Composition (wt%) *¹*** | | | | | | | | | | | |
|---|---|---|---|---|---|---|---|---|---|---|---|---|
| | **A4** | **B4** | **C4** | **D4** | **E4** | **F4** | **G4** | **H4** | **I4** | **J4** | **K4** | **L4** |
| Top heavy fragrance oil examples 1-6 | 5-9 | 5-9 | - | 5-9 | 5-9 | - | 5-9 | 5-9 | - | 5-9 | 5-9 | - |
| Traditional fragrance oil examples 1A, 2A, 3A, 4A, 5A, 6A | - | - | 5-9 | - | - | 5-9 | - | - | 5-9 | - | - | 5-9 |
| Ethanol | 73-77 | | | | | | | | | | | |
| Butylated Hydroxy Toluene | 0-0.07 | | | | | | | | | | | |
| PPG-20 Methyl Glucose Ether *⁴* | 13-17 | 0 | 0 | - | - | - | - | - | - | - | - | - |
| Caprylyl/Capryl Glucoside *⁵* | - | - | - | 13-17 | 0 | 0 | - | - | - | - | - | - |
| Undecyl Glucoside *⁶* | - | - | - | - | - | - | 13-17 | 0 | 0 | - | - | - |
| Isocetyl Aclohol *⁷* | - | - | - | - | - | - | - | - | - | 13-17 | 0 | 0 |
| Deionized water | to 100.00 | | | | | | | | | | | |

| | | | | | | | | | | | | |
|---|---|---|---|---|---|---|---|---|---|---|---|---|
| *¹* Wt% is relative to the total weight of the composition. *⁴* Available as GLUCAM^{™} P-20. *⁵* Available as Plantacare^{®} 810 UP. *⁶* Available as Simulsol^{®} SL 11W. *⁷* Available as Ceraphyl^{®} ICA. | | | | | | | | | | | | |

### Example 8 - Exemplary Product Compositions

Compositions I, II, III and IV are examples of body spray compositions according to the present invention. They are prepared by admixture of the components described in Table 16, in the proportions indicated.

**Table 16 - Body Spray Compositions**

| **Ingredients** | **CAS Number** | **Compositions (wt% *¹*)** | | | |
|---|---|---|---|---|---|
| | | **I** | **II** | **III** | **IV** |
| Denatured Ethanol | 64-17-5 | 38.00-40.00 | 58.00-60.00 | 38.00-40.00 | 38.00-40.00 |
| Water | 7732-18-5 | -- | 0.50-0.80 | -- | -- |
| Dipropylene Glycol | 25265-71-8 | 13.00-17.00 | -- | 13.00-17.00 | 13.00-17.00 |
| Isopropyl Myristate | 110-27-0 | 0.50-1.50 | -- | 0.50-1.50 | 0.50-1.50 |
| Zinc Phenosulphonate | 127-82-2 | 0.25-0.75 | -- | 0.25-0.75 | 0.25-0.75 |
| Cavasol^{®} W7 methylated Betacyclodextrin | 128446-36-6 | -- | 0.50-1.50 | -- | -- |
| Fragrance *²* | -- | 1.10-1.30 | 1.10-1.30 | 1.10-1.30 | 11.10-1.3020 |
| Fragrance Modulator *³* | -- | 2.40-2.80 | 2.40-2.80 | 2.40-2.80 | 2.40-2.80 |
| Propane | 74-98-6 | 4.70-4.90 | -- | 4.70-4.90 | 4.70-4.90 |
| Isobutane | 72-28-5 | 26.00-28.00 | -- | 26.00-28.00 | 26.00-28.00 |
| 1,1-Difluoroethane (HFC-152a) | 75-37-6 | 7.00-9.00 | 33.00-37.00 | 7.00-9.00 | 7.00-9.00 |
| Total | | 100.00 | 100.00 | 100.00 | 100.00 |

| | | | | | |
|---|---|---|---|---|---|
| *¹* wt% relative to the total weight of the composition. *²* Can be any one of Fragrances Examples 1, 2, 3, 4, 5 or 6. *³* Can be any one of the substantially non-odorous fragrance modulators disclosed in Tables 4(a) and 4(b) that is methyl glucoside polyol, ethyl glucoside polyol, propyl glucoside polyol, or mixtures thereof. | | | | | |

Composition V, VI and VII are examples of body lotion compositions according to the present invention. They are prepared by admixture of the components as described in Table 17, in the proportions indicated.

**Table 17 - Body Lotion Composition**

| **Ingredients** | **CAS Number** | **Compositions (wt% *¹*)** | | |
|---|---|---|---|---|
| | | **V** | **VI** | **VII** |
| Water | 7732-18-5 | qsp 100 % | qsp 100 % | qsp 100 % |
| Trilon^{®} B | 64-02-8 | 0.02-0.07 | 0.02-0.07 | 0.02-0.07 |
| Carbopol^{®} ETD 2050 | 9003-01-4 | 0.1-0.3 | 0.1-0.3 | 0.1-0.3 |
| Pemulen^{™} TR1 | 9063-87-0 | 0.1-0.3 | 0.1-0.3 | 0.1-0.3 |
| Nexbase^{®} 2008 | 68037-01-4 | 7-9 | 7-9 | 7-9 |
| Silicone V100 | 63148-62-9 | 5-7 | 5-7 | 5-7 |
| Fragrance Modulator *³* | -- | 2-4 | 2-4 | 2-4 |
| Tris Amino^{™} Ultra Pur | 102-71-6 | 0.2-0.6 | 0.2-0.6 | 0.2-0.6 |
| Fragrance *²* | -- | 2-4 | 2-4 | 2-4 |
| Preservatives | -- | qs | qs | qs |
| Total | | 100.00 | 100.00 | 100.00 |

| | | | | |
|---|---|---|---|---|
| *¹* wt% relative to the total weight of the composition. *²* Can be any one of the Fragrances Examples 1, 2, 3, 4, 5 or 6. *³* Can be any one of the substantially non-odorous fragrance modulators disclosed in Tables 4(a) and 4(b) that is methyl glucoside polyol, ethyl glucoside polyol, propyl glucoside polyol, or mixtures thereof. | | | | |

### Example 9: results from Test Method 3

Using the analytical evaporation Test Method 3, it is possible to measure the amount of each component of a perfume mixture that remains as the fragrance mixture evaporates. Test compositions in Tables 13, 14, 15 & 15 (a) are introduced in the aluminum pan at the set temperature for pre-determined periods of time in accordance with the protocol described in Test Method 3. The amount of each individual high volatility fragrance materials remaining is aggregated and the total high volatility fragrance materials remaining is plotted over time.

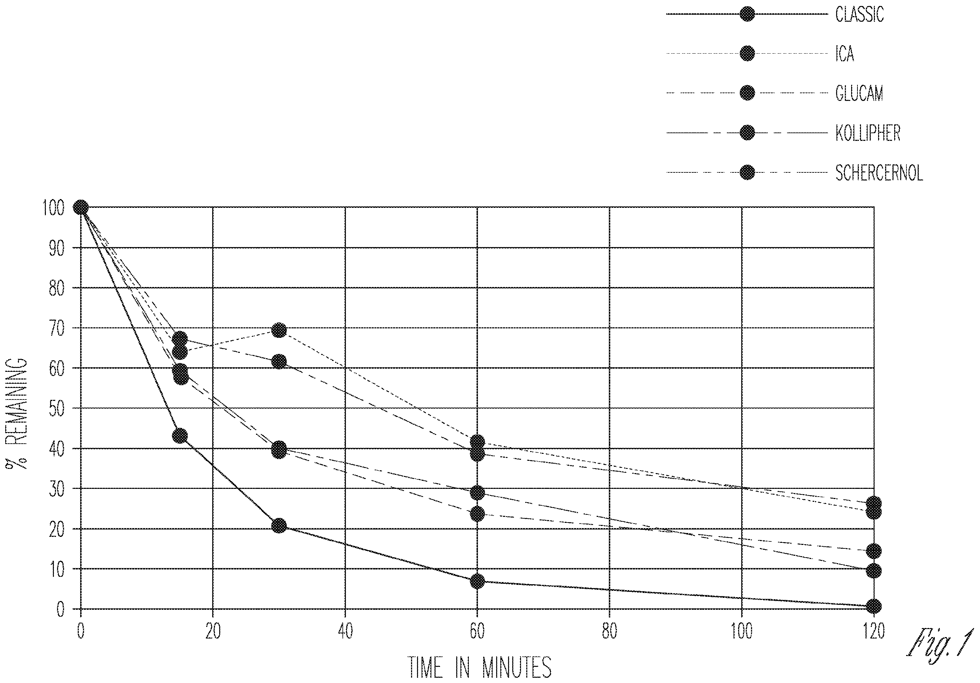

FIGs. 1 to 3, show the total amount of high volatility fragrance materials remaining in an aluminium pan after evaporation for specific lengths of time for Example A3 containing Fragrance oil examples 2 or 3 with a variety of modulators compared to the Example C3 containing Comparative fragrance oil example 2A or 3A respectively with no modulator.

FIG. 1 shows the total amount of high volatility fragrance materials remaining in an aluminium pan after evaporation for 2 hours for Example A3 containing Fragrance oil example 3 with the modulator being either GLUCAM^{™} P-20 ("Glucam"), Ceraphyl^{®} ICA ("ICA"), Schercemol^{™} NGDO ("Schercemol") or Kolliphor^{®} EL ("Kolliphor") compared to the reference product C3 containing Comparative fragrance oil example 3A and no modulator ("Classic").

The percentage of high volatility fragrance materials remaining in a traditionally constructed fragrance oil example 3A in the absence of a modulator decreases very quickly to 21% of starting level in 30 mins, 7% in 60 mins and close to zero in 120 mins. For a fragrance constructed with the high level of high volatility materials in example 3 in combination with one of the modulators, the reduction is much slower demonstrating the delayed evaporation of the high volatility perfume materials. For example for GLUCAM^{™} P-20 it is 40% and for Ceraphyl^{®} ICA 69% after 30 mins and 24% and 39% respectively after 60 mins.. The reduction in evaporation is achieved with a variety of modulators.

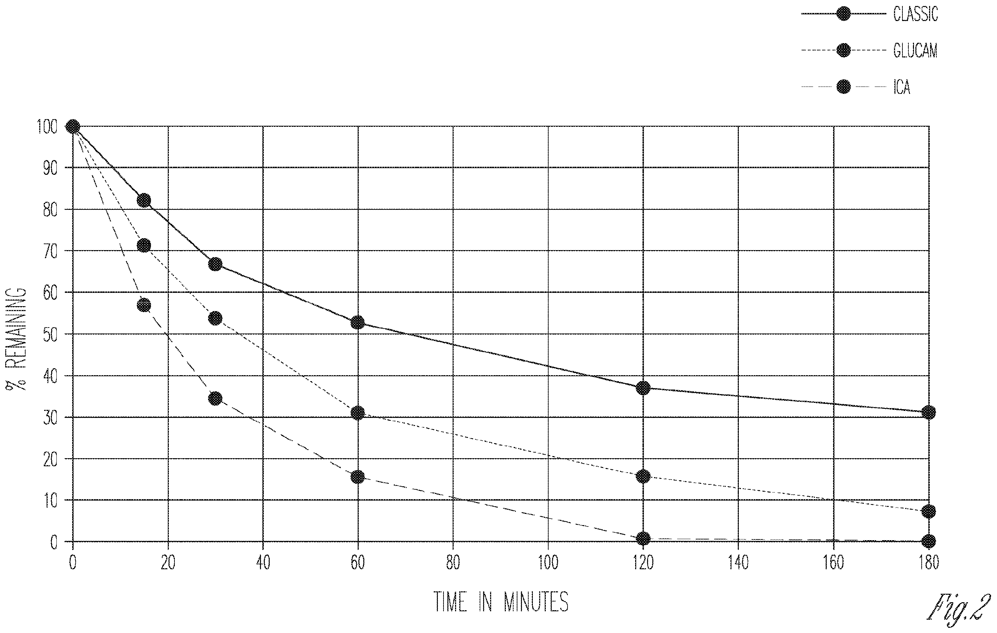

FIG. 2 shows the total amount of high volatility fragrance materials remaining in an aluminium pan after evaporation for 3 hours for Examples A3 containing Fragrance oil example 2 with the modulator being either GLUCAM^{™} P-20 ("Glucam") or Ceraphyl^{®} ICA ("ICA"), compared to the reference product C3 containing Comparative fragrance oil 2A and no modulator ("Classic").

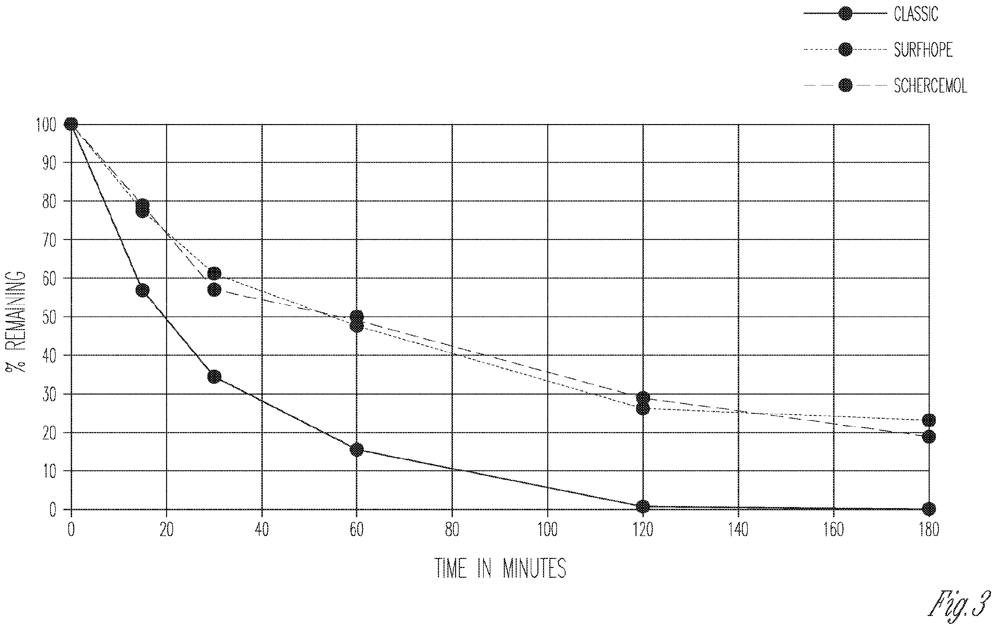

FIG. 3 shows the total amount of high volatility fragrance materials remaining in an aluminium pan after evaporation for 3 hours for Examples A3 containing Fragrance oil example 2 with the modulator being either Schercemol^{™} NGDO ("Schercemol") or Surfhope SE COSME C-1216 ("Surfhope"), compared to the reference product C3 containing Comparative fragrance oil 2A and no modulator ("Classic").

The percentage of high volatility fragrance materials remaining in the traditionally constructed fragrance oil example 2A in the absence of a modulator decreases very quickly to 16% of the starting level in 60 mins and close to zero in 120 mins. For the fragrance constructed with a high level of high volatility materials oil example 2 in combination with one of the modulators, the reduction is much slower demonstrating the delayed evaporation of the high volatility perfume materials. For example, for Ceraphyl^{®} ICA 53% after 60 mins and 37% after 120 mins whilst for Schercemol ^{™} NGDO it is 47% after 60 mins and 27% after 120 mins. The reduction in evaporation is achieved with a variety of modulators.

### Example 10: Results from Olfactory Test 2a

Compositions disclosed in Tables 13, 14, 15 & 15 (a) are applied to glass slides in accordance with the protocol described in the Method Section and panelists evaluate the perceived fragrance profile at initial time 0, then at various time points, typically 30 mins, 1 hour, 2 hours, 3 hours, 4 hours and 6 hours post application. Panelists are asked to score the compositions according to the protocol described in the Methods Section. The results of the panelists are then averaged and discussed below.

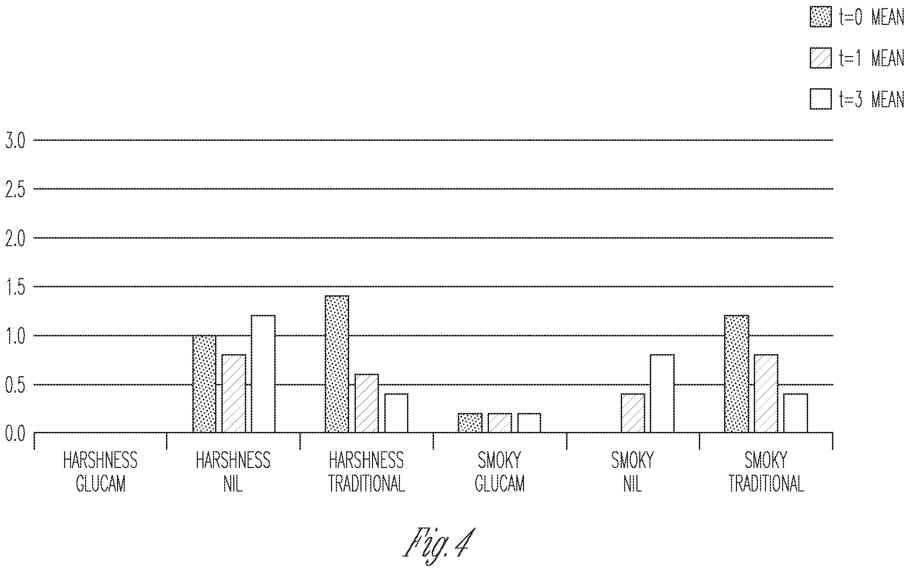

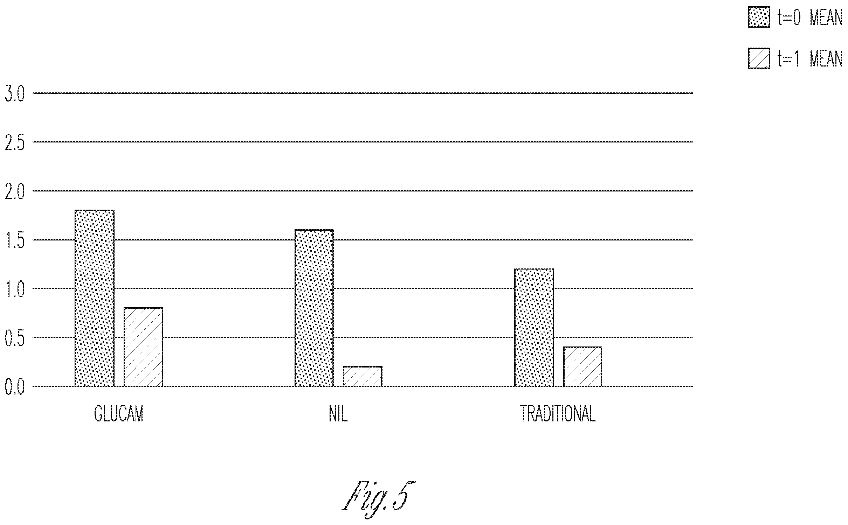

In FIGs. 4 and 5, Fragrance Composition A4 with Oil Example 4 is designated "Glucam", Fragrance Composition B4 with Oil Example 4 is designated "Nil", and Fragrance Composition C4 with Comparative Oil Example 4A is designated "Traditional".

As shown in FIGs. 4-5, the presence of the Glucam P-20 modulator helps to mute the perceived harshness and smoky notes whilst the high volatility citrus fragrance character is maintained for at least 1 hour, as compared to a corresponding traditional fragrance construction and a top heavy fragrance construction that is free of a modulator. As further shown the present of the Glucam p-20 modulator maintains the high volatility citrus fragrance character to a higher degree than the corresponding "nil" construction, which is free of the modulator, but includes more high volatility fragrances. FIG. 4 shows perceived harshness and smoky notes and FIG. 5 shows citrus notes.

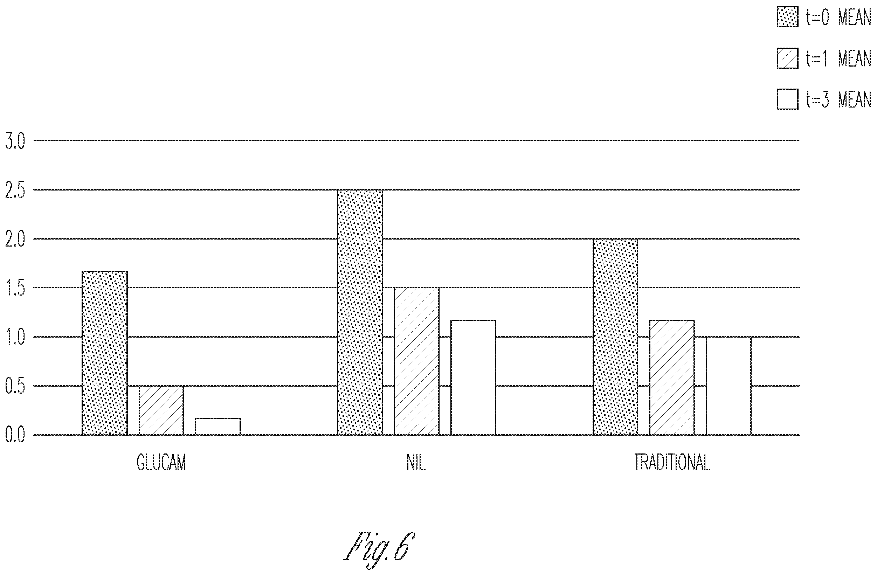

In FIGs. 6-7, Fragrance Composition A4 with Oil Example 6 is designated "Glucam", Fragrance Composition B4 with Oil Example 6- is designated "Nil", and Fragrance Composition C4 with Comparative Oil Example 6A is designated "Traditional".

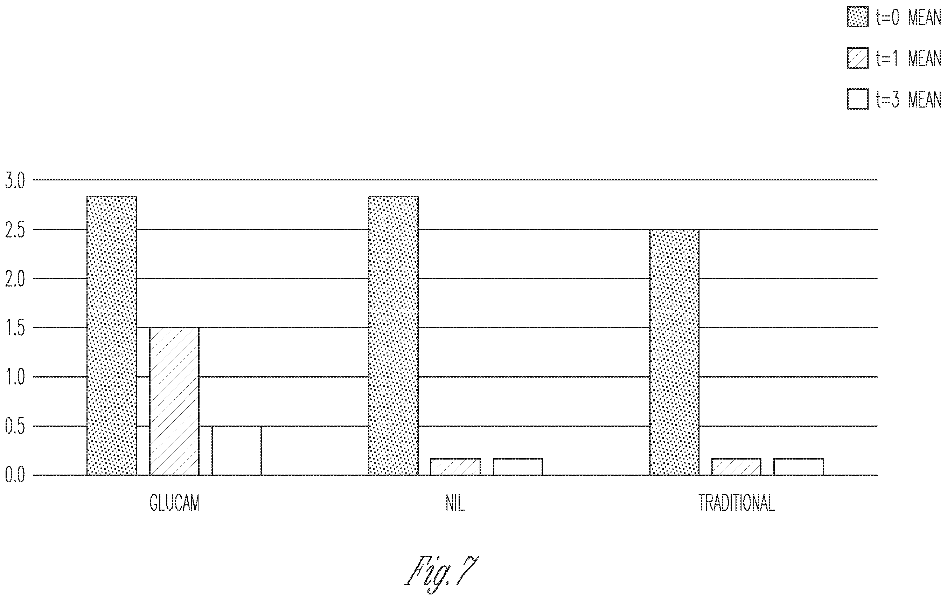

As shown in FIGs. 6-7 the presence of Glucam P-20 helps to maintain the perception of high-volatility characters such as citrus for up to 3 hours, whilst reducing the perceived harshness as compared to a corresponding traditional fragrance construction and a top-heavy fragrance construction that is free of a modulator. As further shown the present of the Glucam p-20 modulator maintains the high volatility citrus fragrance character to a higher degree than the corresponding "nil" construction, which is free of the modulator, but includes more high volatility fragrances. FIG. 6 shows perceived harshness and FIG. 7 shows citrus notes.

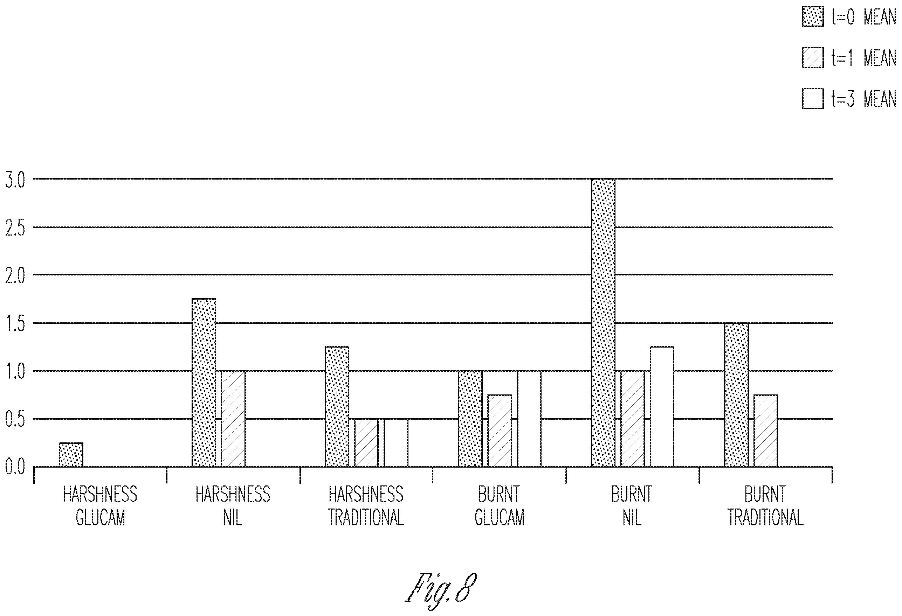

In FIGs. 8-10, Fragrance Composition A4 with Oil Example 3 is designated "Glucam", Fragrance Composition B4 with Oil Example 3 is designated "Nil", and Fragrance Composition C4 with Comparative Oil Example 3A is designated "Traditional".

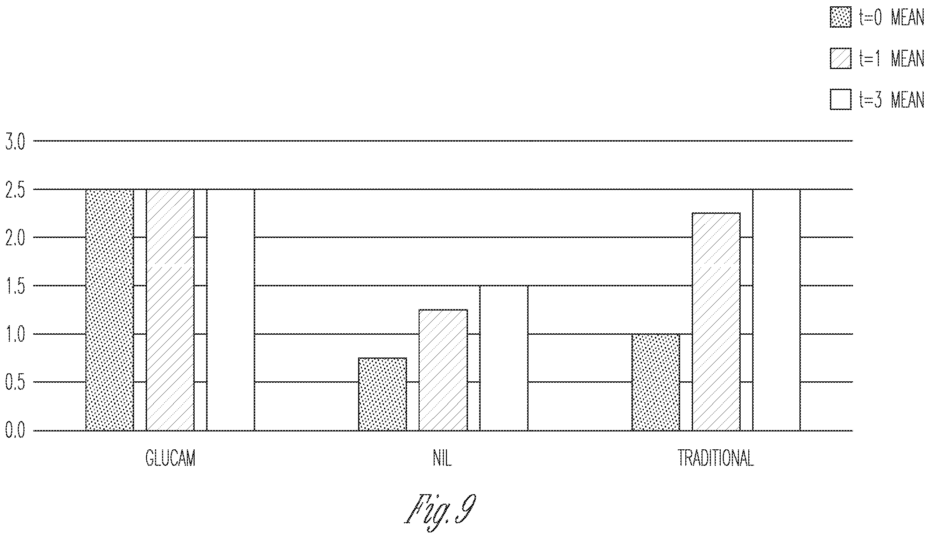

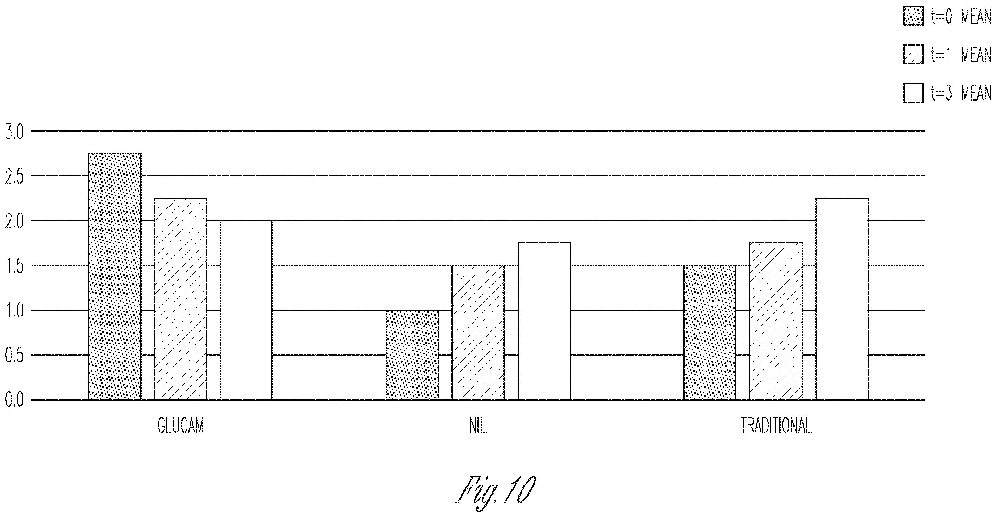

As shown in FIGs. 8-10 the presence of Glucam P-20 results in an initially dominant perception of creamy and vanillic notes which endure for at least an hour, whilst reducing the perceived harshness and burnt notes of fragrances as compared to a corresponding traditional fragrance construction and a top-heavy fragrance construction that is free of a modulator. FIG. 8 shows perceived harshness and burnt notes and FIG. 9 shows creamy notes, and FIG. 10 shows vanillic notes.

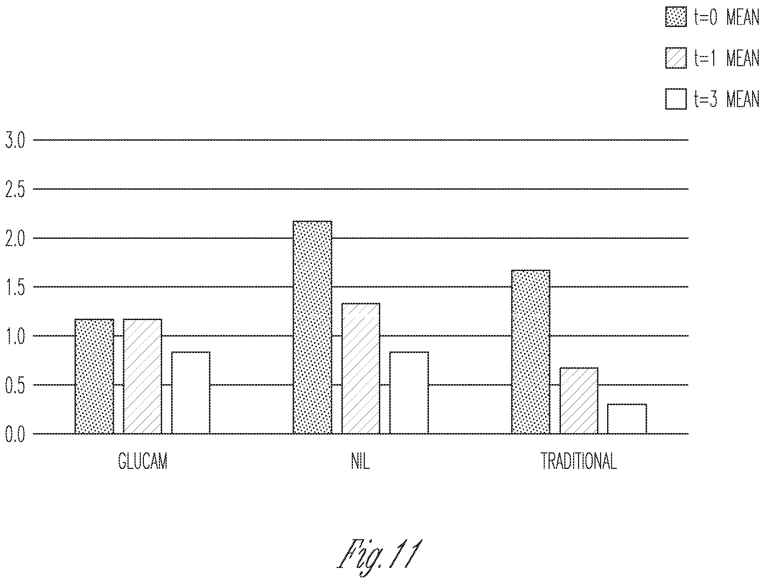

In FIGs. 11-12, Fragrance Composition A4 with Oil Example 1 is designated "Glucam", Fragrance Composition B4 with Oil Example 1- is designated "Nil", and Fragrance Composition C4 with Comparative Oil Example 1A is designated "Traditional".

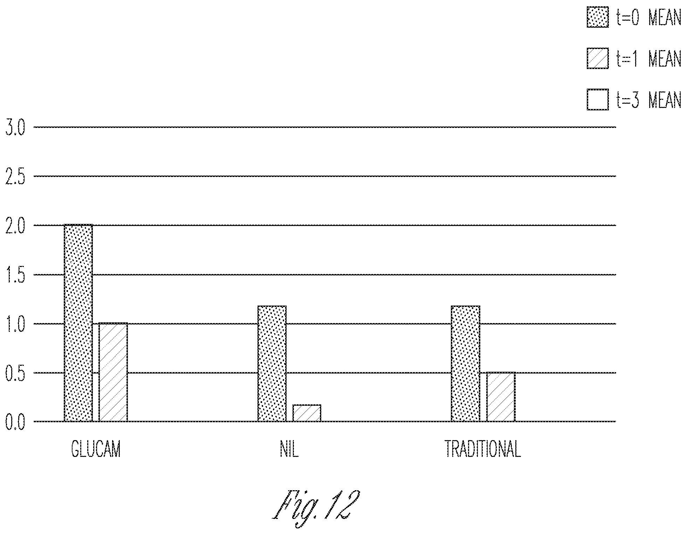

As shown in FIGs. 11 and 12 the presence of Glucam P-20 results in an initially greater perception of high volatility citrus notes that is maintained over time, whilst reducing the perceived harshness as compared to a corresponding traditional fragrance construction and a top-heavy fragrance construction that is free of a modulator. As further shown the present of the Glucam p-20 modulator maintains the high volatility citrus fragrance character to a higher degree than the corresponding "nil" construction, which is free of the modulator, but includes more high volatility fragrances. FIG. 11 shows perceived harshness and FIG. 12 shows citrus notes.

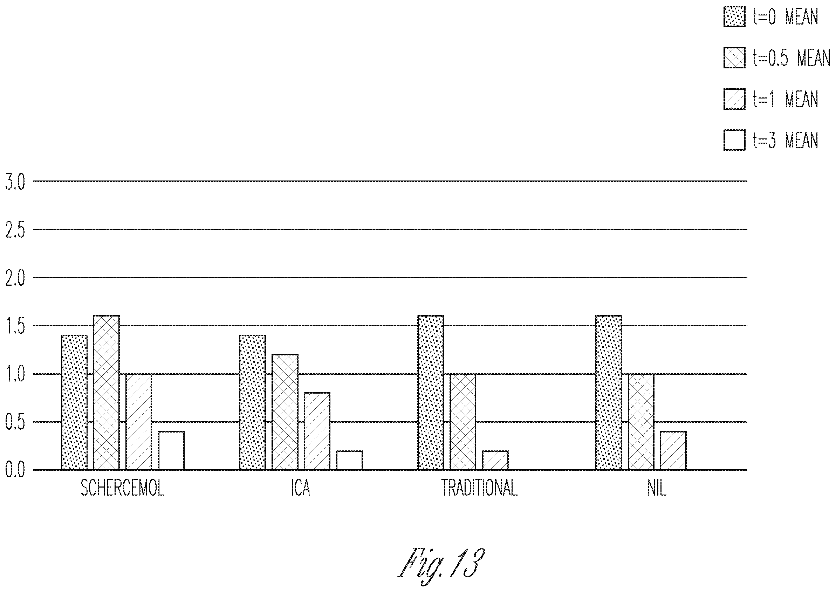

The effect of substituting Glucam P-20 modulator with other modulators was studied. FIG. 13 shows the citrus notes in Fragrance Constructions that include modulators chosen from Schercemol NGDO and iso cetyl alcohol (ICA), in Fragrance Composition Example A3 each including Oil Example 2 compared to Fragrance Composition Example B3 also including Oil Example 2 but no modulator, designated "Nil", and Fragrance Composition Example C3 with Comparative Oil Example 2A, designated "Traditional". As shown the presence of the modulator plays an important role in the preservation of the top-note fragrance characters. This can be seen by comparing the data from the traditional and nil constructions to any Fragrance Construction including a modulator, such as Schercemol NGDO and iso cetyl alcohol (ICA). Both the traditional and nil Constructions show similar behavior that is inferior to that of the Fragrance Construction including a modulator, such as Schercemol NGDO and iso cetyl alcohol (ICA).

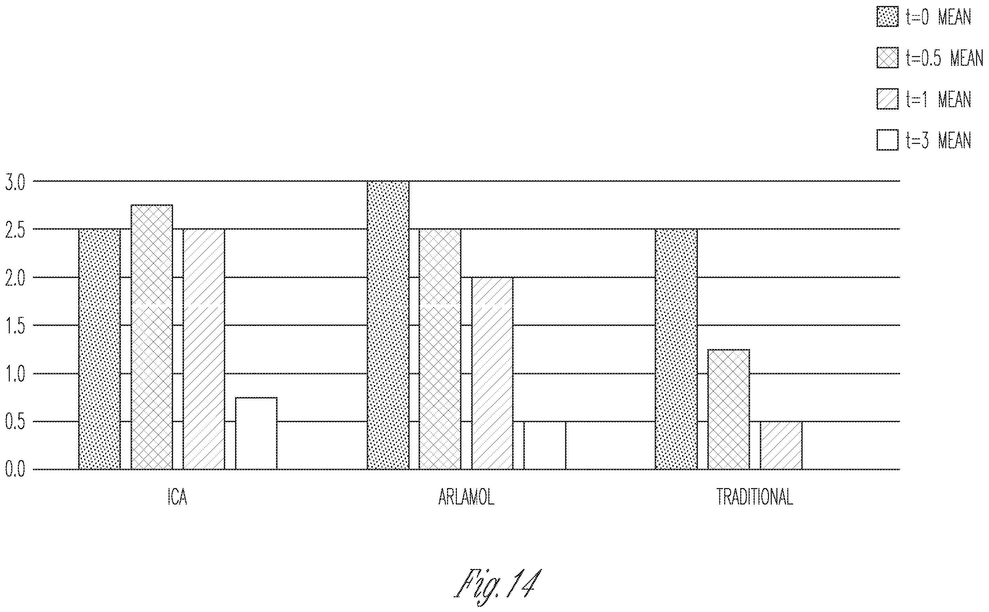

Other constructions are shown in FIG. 14 which shows citrus notes as perceived in Fragrance Composition Example A3 with Oil Example 5 that include modulators chosen from iso cetyl alcohol (ICA) and Arlamol PC-10 compared to Fragrance Composition Example C3 with Comparative Oil Example 5A.

As shown even with different modulators other than Glucam P-20, high volatility citrus notes are extended as compared to fragrances having a traditional construction and that are free of a modulator.

### Example 11: Results from Olfactory Test 2b

Compositions disclosed in Tables 13, 14, 15 & 15 (d) are applied to glass slides in accordance with the protocol described in the Method Section and panelists evaluate the perceived fragrance profile at initial time 0, then at various time points, typically 30 mins, 1 hour, 2 hours, 3 hours, 4 hours and 6 hours post application. Panelists are asked to score the compositions according to the protocol described in the Methods Section. The results of the panelists are then averaged and discussed below.

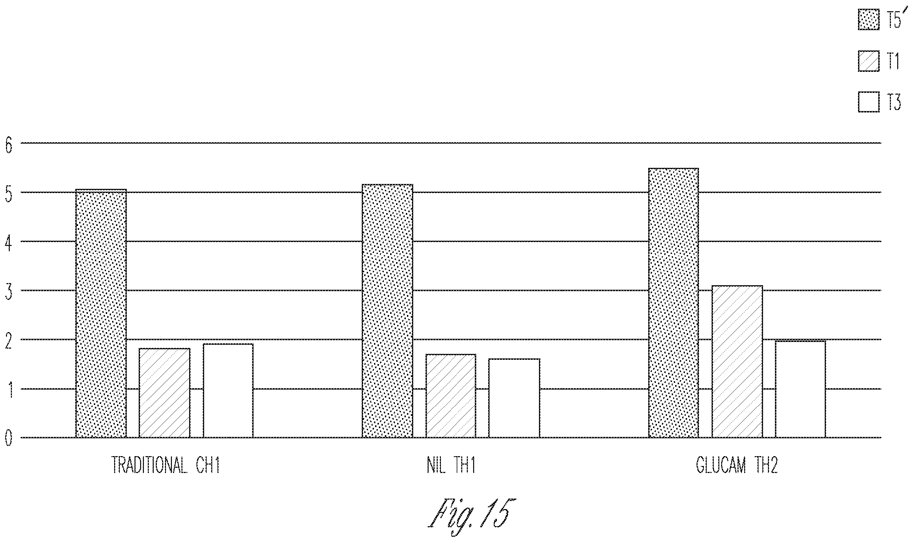

In FIGs. 15-16, Fragrance Composition A4 with Oil Example 2 is designated "Glucam", Fragrance Composition B4 with Oil Example 2 is designated "Nil", and Fragrance Composition C4 with Comparative Oil Example 2A is designated "Traditional".

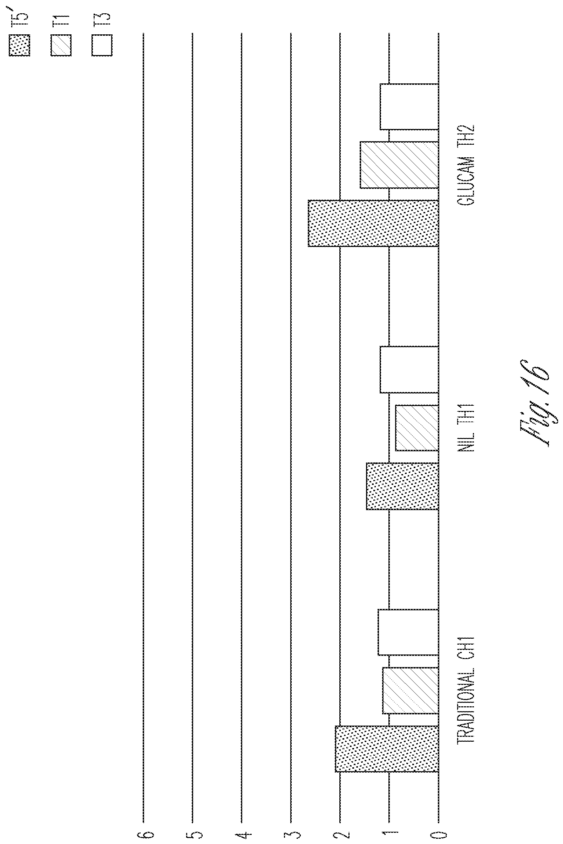

As shown in FIGs. 15-16 the presence of Glucam P-20 results in an initially greater perception of high volatility notes that is maintained over time, such as citrus and mint notes, compared to a corresponding traditional fragrance construction and a top-heavy fragrance construction that is free of a modulator. In FIG. 15 the citrus note in the Glucam P-20 containing formula is statistically significantly stronger at 1 hour at 95% confidence (p-value = 0.027). In FIG. 16 the mint note in the Glucam P-20 containing formula is statistically significantly stronger initially at 95% confidence (p-value = 0.023) than both the other products and significantly stronger at 1 hour at 90% confidence (p-value = 0.09) than the "nil" product.

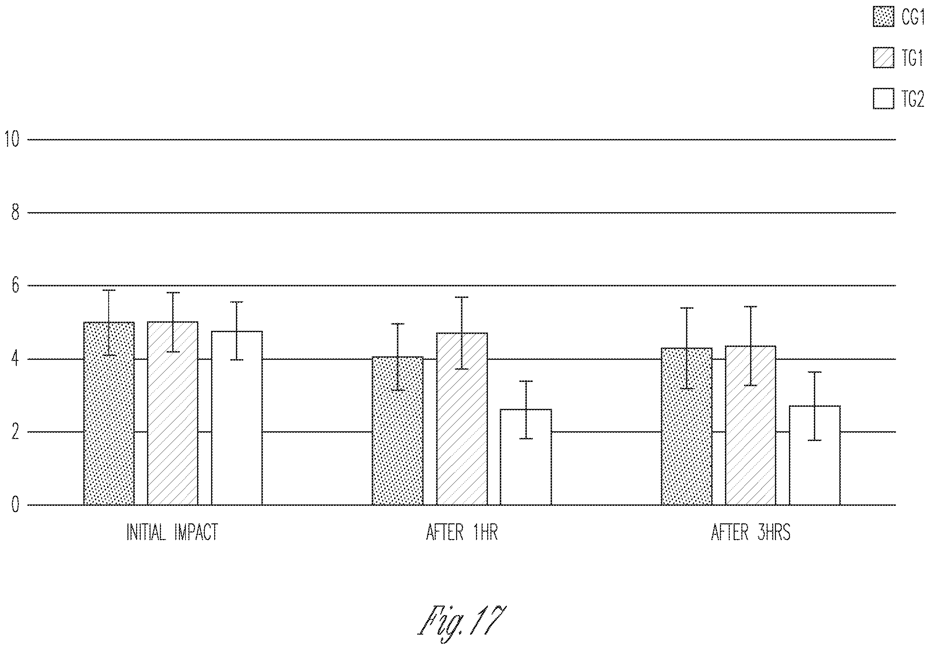

In FIGs. 17-18, Fragrance Composition A4 with Oil Example 5 is designated "Glucam", Fragrance Composition B4 with Oil Example 5 is designated "Nil", and Fragrance Composition C4 with Comparative Oil Example 5A is designated "Traditional".

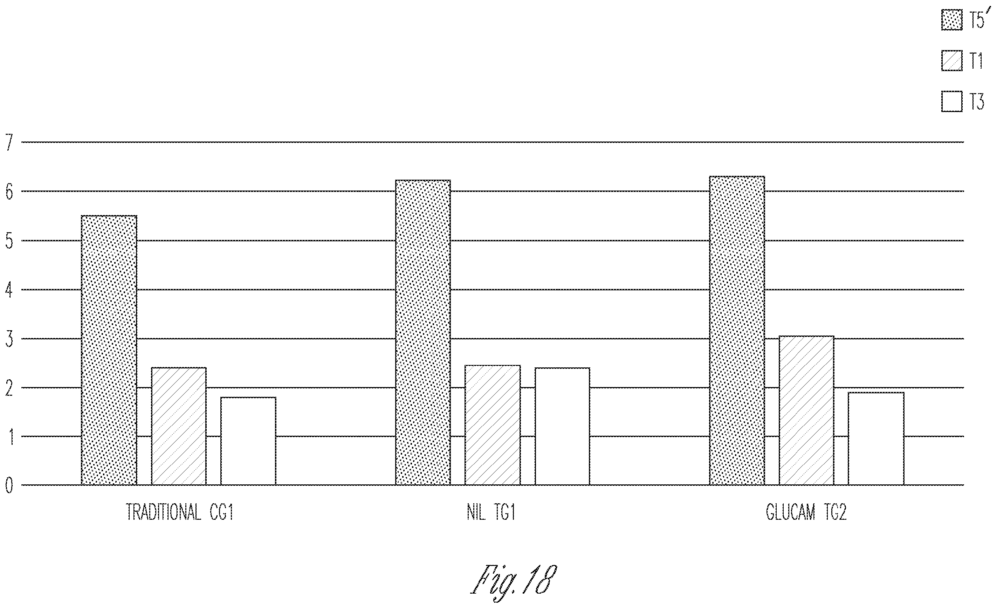

As shown in FIGs. 17-18 the presence of Glucam P-20 results in an initially greater perception of high volatility notes, such as citrus notes, that is maintained over time, without the perceived harshness compared to a corresponding traditional fragrance construction and a top-heavy fragrance construction that is free of a modulator. In FIG. 17 the harshness in the Glucam P-20 containing formula is statistically significantly lower at time 1 hour and 3 hours at 95% confidence (p-value < 0.0001). In FIG. 18 the citrus note in the Glucam P-20 containing formula is statistically significantly stronger initially at 90% confidence (p-value = 0.093) than the traditionally constructed fragrance.

## Claims

1. A composition comprising:
a fragrance component present in an amount of from 0.04 wt% to 30 wt%, relative to the total weight of the composition, and wherein the fragrance component comprises:
at least one low volatile fragrance material having a vapor pressure less than 0.001 Torr (0.000133 kPa) at 25 °C present in an amount of from 1 wt% to 30 wt%, relative to the total weight of the fragrance component;
at least one moderate volatile fragrance material having a vapor pressure in the range of 0.1 Torr to 0.001 Torr (0.0133 kPa to 0.000133 kPa) at 25 °C present in an amount of from 25 wt% to 65 wt%, relative to the total weight of the fragrance component; and
at least one high volatile fragrance material having a vapor pressure greater than 0.1 Torr (0.0133 kPa) at 25 °C present in an amount of greater than 30 wt % relative to the total weight of the fragrance component; and
at least one substantially non-odorous fragrance modulator present in the amount of from 0.1 wt% to 20 wt%, relative to the total weight of the composition;
wherein the at least one substantially non-odorous fragrance modulator is chosen from methyl glucoside polyol, ethyl glucoside polyol, propyl glucoside polyol, or mixtures thereof.

2. The composition of claim 1, wherein the at least one low volatile fragrance material is present in an amount of from 10 wt% to 25 wt% relative to the total weight of the fragrance material.

3. The composition of any one of claims 1 or 2, wherein the at least one moderate volatile fragrance material is present in an amount of from 30 wt% to 55 wt% relative to the total weight of the fragrance material.

4. The composition of any one of claims 1-3, wherein the at least one high volatile fragrance material is present in an amount of from 31 wt% to 60 wt% relative to the total weight of the fragrance material.

5. The composition of any one of claims 1-4, wherein the fragrance material is selected from a citrus-type note, green-type note, watery-type notes, aromatic-type notes, herbal-type notes, mint-type notes, lavender-type notes, rosemary-type notes, spicy-type notes, cinnamontype notes, clove-type notes, pepper-type notes, cumin-type notes, ginger-type notes, fougeretype note, patchouli-type notes, floral-type notes, gourmand-type notes, sweet-type notes, vanilla-type notes, amber-type notes, woody-type notes, cedarwoood-type notes, sandalwood type notes, vetyver-type notes and mixtures thereof.

6. A method for producing a consumer product comprising bringing into contact or mixing into the product an organoleptically active quantity of a composition according to any one of claims 1-5.

7. A method of modifying or enhancing the odor properties of a body surface, comprising contacting or treating the body surface with a composition according to any one of claims 1-5.

8. A composition according to claim 1 wherein the at least one substantially non-odorous fragrance modulator is chosen from polypropylene glycol-10 methyl glucose ether, ethoxylated methyl glucose ether, and polypropylene glycol-20 methyl glucose ether, present in the amount of from 0.1 wt% to 20 wt%, relative to the total weight of the composition.

## Patentansprüche

1. Zusammensetzung, umfassend:
eine Duftstoffkomponente, die in einer Menge von 0,04 Gew.-% bis 30 Gew.-%, bezogen auf das Gesamtgewicht der Zusammensetzung, vorliegt, und wobei die Duftstoffkomponente umfasst:
mindestens ein schwerflüchtiges Duftstoffmaterial mit einem Dampfdruck von weniger als 0,001 Torr (0,000133 kPa) bei 25 °C, das in einer Menge von 1 Gew.-% bis 30 Gew.-%, bezogen auf das Gesamtgewicht der Duftstoffkomponente, vorliegt;
mindestens ein mäßig flüchtiges Duftstoffmaterial mit einem Dampfdruck im Bereich von 0,1 Torr bis 0,001 Torr (0,0133 kPa bis 0,000133 kPa) bei 25 °C, das in einer Menge von 25 Gew.-% bis 65 Gew.-%, bezogen auf das Gesamtgewicht der Duftstoffkomponente, vorliegt; und
mindestens ein hochflüchtiges Duftstoffmaterial mit einem Dampfdruck von mehr als 0,1 Torr (0,0133 kPa) bei 25 °C, das in einer Menge von mehr als 30 Gew.-%, bezogen auf das Gesamtgewicht der Duftstoffkomponente vorliegt; und
mindestens einen im Wesentlichen geruchsfreien Duftstoffmodulator, der in einer Menge von 0,1 Gew.-% bis 20 Gew.-%, bezogen auf das Gesamtgewicht der Zusammensetzung, vorliegt;
wobei der mindestens eine im Wesentlichen geruchsfreie Duftstoffmodulator aus Methylglucosidpolyol, Ethylglucosidpolyol, Propylglucosidpolyol oder Gemischen davon ausgewählt ist.

2. Zusammensetzung nach Anspruch 1, wobei das mindestens eine schwerflüchtige Duftstoffmaterial in einer Menge von 10 Gew.-% bis 25 Gew.-%, bezogen auf das Gesamtgewicht des Duftstoffmaterials, vorliegt.

3. Zusammensetzung nach einem der Ansprüche 1 oder 2, wobei das mindestens eine mäßig flüchtige Duftstoffmaterial in einer Menge von 30 Gew.-% bis 55 Gew.-%, bezogen auf das Gesamtgewicht des Duftstoffmaterials, vorliegt.

4. Zusammensetzung nach einem der Ansprüche 1 bis 3, wobei das mindestens eine hochflüchtige Duftstoffmaterial in einer Menge von 31 Gew.-% bis 60 Gew.-%, bezogen auf das Gesamtgewicht des Duftstoffmaterials, vorliegt.

5. Zusammensetzung nach einem der Ansprüche 1 bis 4, wobei das Duftstoffmaterial aus einer Zitrusnote, einer grünen Note, wässrigen Noten, aromatischen Noten, krautigen Noten, Minznoten, Lavendelnoten, Rosmarinnoten, würzigen Noten, Zimtnoten, Nelkennoten, Pfeffernoten, Kümmelnoten, Ingwernoten, Fougère-Noten, Patchoulinoten, floralen Noten, Gourmand-Noten, süßen Noten, Vanillenoten, Ambernoten, holzigen Noten, Zedernholznoten, Sandelholznoten, Vetivernoten und Mischungen davon ausgewählt ist.

6. Verfahren zur Herstellung eines Verbraucherprodukts, umfassend das Inkontaktbringen oder Mischen einer organoleptisch wirksamen Menge einer Zusammensetzung in das Produkt nach einem der Ansprüche 1 bis 5.

7. Verfahren zum Modifizieren oder Verbessern der Geruchseigenschaften einer Körperoberfläche, umfassend das Inkontaktbringen oder Behandeln der Körperoberfläche mit einer Zusammensetzung nach einem der Ansprüche 1 bis 5.

8. Zusammensetzung nach Anspruch 1, wobei der mindestens eine im Wesentlichen geruchsfreie Duftstoffmodulator aus Polypropylenglykol-10-methylglucoseether, ethoxyliertem Methylglucoseether und Polypropylenglykol-20-methylglucoseether ausgewählt ist, vorhanden in einer Menge von 0,1 Gew.-% bis 20 Gew.-%, bezogen auf das Gesamtgewicht der Zusammensetzung.

## Revendications

1. Composition comprenant :
un composant de parfum présent en une quantité allant de 0,04 % en poids à 30 % en poids, par rapport au poids total de la composition, et dans laquelle le composant de parfum comprend :
au moins un matériau de parfum à volatilité faible ayant une pression de vapeur inférieure à 0,001 Torr (0,000133 kPa) à 25 °C présent en une quantité allant de 1 % en poids à 30 % en poids, par rapport au poids total du composant de parfum ;
au moins un matériau de parfum à volatilité moyenne ayant une pression de vapeur dans la plage de 0,1 torr à 0,001 torr (0,0133 kPa à 0,000133 kPa) à 25 °C présent en une quantité allant de 25 % en poids à 65 % en poids, par rapport au poids total du composant de parfum ; et
au moins un matériau de parfum à volatilité élevée ayant une pression de vapeur supérieure à 0,1 Torr (0,0133 kPa) à 25 °C présent en une quantité supérieure à 30 % en poids par rapport au poids total du composant de parfum ; et
au moins un modulateur de parfum sensiblement non odorant présent dans une quantité allant de 0,1 % en poids à 20 % en poids, par rapport au poids total de la composition ;
dans laquelle au moins un modulateur de parfum sensiblement non odorant est choisi parmi le méthyl glucoside polyol, l'éthyl glucoside polyol, le propyl glucoside polyol, ou des mélanges de ceux-ci.

2. Composition selon la revendication 1, dans laquelle au moins un matériau de parfum faiblement volatil est présent en une quantité allant de 10 % en poids à 25 % en poids par rapport au poids total du matériau de parfum.

3. Composition selon l'une des revendications 1 ou 2, dans laquelle l'au moins un matériau de parfum à volatilité moyenne est présent en une quantité allant de 30 % en poids à 55 % en poids par rapport au poids total du matériau de parfum.

4. Composition selon l'une quelconque des revendications 1 à 3, dans laquelle l'au moins un matériau de parfum à volatilité élevée est présent en une quantité allant de 31 % en poids à 60 % en poids par rapport au poids total du matériau de parfum.

5. Composition selon l'une quelconque des revendications 1 à 4, dans laquelle le composant de parfum est sélectionné parmi une note de type agrumes, une note de type verte, des notes de type acqueux, des notes de type aromatique, des notes de type herbacé, des notes de type menthe, des notes de type lavande, des notes de type romarin, des notes de type épicé, des notes de type cannelle, des notes de type clou de girofle, des notes de type poivre, des notes de type cumin, des notes de type gingembre, des notes de type fougère, des notes de type patchouli, des notes de type floral, des notes de type gourmand, des notes de type sucré, des notes de type vanille, des notes de type ambre, des notes de type boisé, des notes de type cèdre, des notes de type santal, des notes de type vetyver et leurs mélanges.

6. Procédé de production d'un produit de consommation comprenant la mise en contact ou le mélange dans le produit d'une quantité organoleptiquement active d'une composition selon l'une quelconque des revendications 1 à 5.

7. Procédé de modification ou d'amélioration des propriétés odorantes d'une surface corporelle, comprenant la mise en contact ou le traitement de la surface corporelle avec une composition selon l'une quelconque des revendications 1 à 5.

8. Composition selon la revendication 1, dans laquelle au moins un modulateur de parfum essentiellement non odorant est choisi parmi le polypropylène glycol-10 méthyl glucose éther, le méthyl glucose éthoxylé, et le polypropylène glycol-20 méthyl glucose éther, présents en une quantité de 0,1 % en poids à 20 % en poids, par rapport au poids total de la composition.
